# FASCICULE DE BREVET EUROPEEN

(11) **EP 3 207 141 B1**
(45) Date de publication et mention de la délivrance du brevet: **31.07.2019**
(21) Numéro de dépôt: 15787265.6
(22) Date de dépôt: 13.10.2015
(51) Int. Cl.: A61K 38/50, C12N 9/10, C12N 15/62, C12N 9/86, A61K 38/45, C12N 15/55, C12N 15/54, C12N 9/16

(54) **MOLECULE PROTEIQUE HYBRIDE APTE A INHIBER AU MOINS UN ANTIBIOTIQUE ET COMPOSITION PHARMACEUTIQUE LA COMPORTANT**
HYBRIDPROTEIN FÜR DAS INHIBITION VON MINSTENS EINEM ANTIBIOTICA UND PHAMAZEUTISCHE ERHALTENDE ZUSAMMESTELLUNG.,
HYBRID PROTEIN MOLECULE BEING ABLE TO INHIIT AT LEAST ONE ANTIBIOTIC AND PHARMACEUTICAL COMPOSITION CONTAINING IT

(30) Priorité: 16.10.2014 FR 1459935
(43) Date de publication de la demande: 23.08.2017
(60) Demande divisionnaire: 19180903.7
(73) Titulaire: Azurrx SAS, 30980 Langlade (FR)
(72) Inventeur: DUPRET, Daniel, F-30420 Calvisson (FR); SCHUE, Mathieu, F-13280 Raphele-les-arles (FR); JAIS, Philippe, F-92130 Issy-les-Moulineaux (FR)
(74) Mandataire: IP Trust
(86) Numéro de dépôt international: PCT/FR2015/052756
(87) Numéro de publication internationale: WO 2016/059341

(56) Documents cités:
- WO-A1-93/13795
- WO-A1-2008/065247
- WO-A1-2012/140364
- FR-A1- 2 843 302
- RIPOLL AIDA ET AL: "In Vitro Selection of Variants Resistant to beta-Lactams plus beta-Lactamase Inhibitors in CTX-M beta-Lactamases: Predicting the In Vivo Scenario?", ANTIMICROBIAL AGENTS AND CHEMOTHERAPY, vol. 55, no. 10, octobre 2011 (2011-10), pages 4530-4536, XP002741222,
- BONNET R ET AL: "Novel cefotaximase (CTX-M-16) with increased catalytic efficiency due to substitution Asp-240fwdarwGly", ANTIMICROBIAL AGENTS AND CHEMOTHERAPY, vol. 45, no. 8, août 2001 (2001-08), pages 2269-2275, XP002741223, ISSN: 0066-4804
- CLOUTHIER CHRISTOPHER M ET AL: "Chimeric beta-Lactamases: Global Conservation of Parental Function and Fast Time-Scale Dynamics with Increased Slow Motions", PLOS ONE, vol. 7, no. 12, décembre 2012 (2012-12), XP002741233,
- DATABASE WPI Week 200360 Thomson Scientific, London, GB; AN 2003-637146 -& WO 03/068975 A1 (CHEN X) 21 August 2003 (2003-08-21)

## Description

### Domaine de l'invention

Le domaine de l'invention est celui de la prévention des effets indésirables des antibiotiques, secondaires à leur action sur la flore gastro-intestinale et la sélection de souches bactériennes résistantes.

### Généralités

De nombreux antibiotiques administrés par voie parentérale peuvent être excrétés par le foie dans la bile sous forme active non modifiée ou de métabolites actifs. Ces antibiotiques peuvent être réabsorbés au niveau iléal et retournent ainsi au foie par voie portale pour être éventuellement excrétés, ce cycle étant dit entéro-hépatique.

Lorsqu'ils sont excrétés dans le tube digestif sous forme active non-modifiée ou de métabolites actifs, ces antibiotiques peuvent exercer une pression de sélection sur les populations bactériennes constituant la microflore normale de l'intestin et du côlon. Une telle sélection microbienne a deux principales conséquences : la survenue de diarrhées aiguës induites par les antibiotiques, ainsi que la sélection et la dissémination de souches bactériennes résistantes.

L'apparition d'une diarrhée aiguë induite par les antibiotiques est d'observation courante en cas d'antibiothérapie parentérale à large spectre. Sa fréquence et sa gravité sont conditionnées par différents facteurs parmi lesquels la durée de traitement, le spectre d'activité de l'antibiotique utilisé et la dose administrée (Aires, Kohler et al. 1999). Une diarrhée aiguë induite par les antibiotiques est ainsi observée chez environ 5-10% des patients recevant une antibiothérapie par amoxicilline, 10-25% sous association amoxicilline-acide clavulanique, et 2-5% sous céphalosporines de 3^{e} génération, fluoroquinolones, azithromycine, clarithromycine, érythromycine et tétracyclines (Gilbert 1994, Bartlett 1996, Hogenauer, Hammer et al. 1998, Wistrom, Norrby et al. 2001).

Si dans la majorité des cas les diarrhées aiguës induites par les antibiotiques sont bénignes, elles peuvent s'accompagner d'une colite dans 10-20% des cas qui peut être sévère. Dans la majorité des cas, cette colite est secondaire à la sélection de *Clostridium difficile,* une bactérie sporulée Gram-positif anaérobie résistante à la plupart des antibiotiques (Gerding 1989, Anand, Bashey et al. 1994). La pathogénicité de cette bactérie est liée à la production de deux toxines A et B. Ces toxines induisent une réaction inflammatoire intense avec recrutement de polynucléaires au niveau de la *lamina proprio* et dans les formes les plus sévères, une colite pseudomembraneuse, ainsi dénommée en raison de l'aspect que prend la muqueuse colorectale (Kelly, Pothoulakis et al. 1994). La mortalité des colites pseudomembraneuses est élevée, particulièrement chez les jeunes enfants et les sujets âgés. Ces colites auraient été responsables de plus de 7200 décès aux Etats-Unis en 2010 (Sherry, Murphy et al. 2012). D'autres agents pathogènes peuvent être responsables de diarrhées aiguës induites par les antibiotiques de gravité variable, tels que *Klebsiella oxytoca, Clostridium perfringens* de type A, *Staphylocoque aureus* et *Candida albicans* (Sparks, Carman et al. 2001, Gorkiewicz 2009).

La pression de sélection que les antibiotiques exercent sur la microflore intestinale contribue également à la dissémination de bactéries résistantes dans l'environnement. La majorité de ces infections causées par ces micro-organismes en milieu hospitalier, aussi dénommées infections nosocomiales, sont particulièrement fréquentes et graves en soins intensifs et en milieu chirurgical. Il s'agit notamment d'infections de cathéters, d'infections urinaires, de pneumopathies et d'infections post-opératoires. Elles sont principalement causées par des entérobactéries Gram-négatif, des staphylocoques coagulase positif ou négatif, des entérocoques et des *Candida albicans* (Richards, Edwards et al. 2000).

Les infections nosocomiales constituent une cause importante de mortalité et un véritable enjeu de santé publique. L'émergence de bactéries résistantes, secondaire à la généralisation de l'antibiothérapie, complique le traitement de ces infections nosocomiales et représente un coût considérable pour la société. En Europe, les infections nosocomiales sont directement responsables d'environ 25000 décès chaque année et d'un surcoût de dépenses de santé et des pertes de productivité estimé à plus de 1,5 milliard d'euros par an (ECDC/EMEA Joint Working Group 2009). Aux Etats-Unis, environ 1,7 million d'infections nosocomiales sont responsables directement ou indirectement de près de 99000 décès chaque année (Klevens, Edwards et al. 2007).

Les antibiotiques, en particulier les tétracyclines, sont largement utilisés en médecine vétérinaire pour le traitement d'animaux d'élevage et familiers. De plus, l'utilisation de ces antibiotiques est autorisée aux Etats-Unis pour favoriser la croissance et la prise de poids des animaux d'élevage. Cette utilisation intensive participe bien sûr à la dissémination dans le milieu naturel de bactéries résistantes (Institute of Medicine 1998, Committee on Drug Use in Food Animais 1999, Georgetown University Center for Food and Nutrition Policy 1999).

### Bêta-lactamines

Les bêta-lactamines inhibent les transpeptidases et des transglycosylases impliquées dans la formation du peptidoglycane, un constitutif essentiel de la paroi des bactéries Gram positif et négatif (Walsh 2000). Elles possèdent un noyau bêta-lactamine, ainsi que des chaines latérales variables (e.g. céphalosporines de 1^{ère}, 2^{ème}, 3^{ème} et 4^{ème} génération) qui modifient leurs propriétés pharmacocinétiques et leur spectre d'efficacité antimicrobien (Turner 2005). Cette classe regroupe les pénicillines, les céphalosporines, les carbapénemes et les monobactames.

Les antibiotiques de la classe des bêta-lactamines sont les agents antimicrobiens les plus communément prescrits. Ils sont très largement utilisés en clinique pour différents types d'infections (e.g. otites, infections respiratoires et gastro-intestinales). Plusieurs voies d'administration peuvent être utilisées mais c'est la voie parentérale qui est préférée pour les sepsis graves, généralement traités en milieu hospitalier. Ils sont fréquemment associés à d'autres classes d'antibiotiques en cas d'infections graves, comme par exemple l'association amoxicilline/acide clavulanique avec un macrolide en cas de pneumopathie atypique.

L'usage intensif des bêta-lactamines a favorisé l'émergence de résistance bactérienne dont le principal mécanisme est l'acquisition de bêta-lactamases, des enzymes capables d'hydrolyser leur noyau bêta-lactamine. Il existe à ce jour plusieurs centaines voire milliers de bêta-lactamases différentes qui peuvent être regroupées en familles selon leur homologie de séquence, leur structure, ou enfin leur propriétés biochimiques (substrat et inhibiteurs). Ces enzymes sont généralement classifiées en fonction de leur substrat préférentiel (pénicillinase, céphalosporinase, imipénèmase, etc.). De plus, les bêta-lactamases ont évolué pour soit devenir résistantes aux inhibiteurs, soit élargir leur spectre d'action sur les bêta-lactamines les plus récentes (Turner 2005, Drawz and Bonomo 2010). Ces inhibiteurs (acide clavulanique, sulbactam, etc.) sont généralement administrés simultanément à des bêta-lactamines et se fixent de manière irréversible aux bêta-lactamases bactériennes.

La classification d'Ambler illustre la gigantesque diversité de ces enzymes bêta-lactamases, qui sont regroupées en quatre familles A, B, C et D selon leurs séquences nucléotidiques (Ambler 1980). Les groupes A, C et D ont une sérine catalytique alors que le groupe B regroupe les métallo-bêta-lactamases dépendantes du zinc.

Les bêta-lactamases de classe A regroupent plusieurs sous-familles, dont les enzymes de type TEM (Bush and Jacoby 1997), enzymes ancestrales permettant la résistance aux pénicillines et aminopénicillines (e.g. ampicilline, amoxicilline, bacampicilline). TEM-36 est une IR-TEM, c'est-à-dire une bêta-lactamase résistante à l'acide clavulanique ((Zhou, Bordon et al. 1994, Henquell, Chanal et al. 1995, Chaibi, Sirot et al. 1999); identifiant TEM-36 ou IRT-7 sur le site de Lahey Clinic (http://www.lahey.orp,/Studies/temtable.asp). L'acide clavulanique est cliniquement utilisé en association avec l'amoxicilline pour élargir le spectre de l'antibiotique aux germes ayant acquis une bêta-lactamase classique (TEM-1). Sous la pression de sélection, les TEM ont acquis progressivement la résistance à l'inhibiteur en mutant quelques acides aminés aujourd'hui bien caractérisés (Chaibi, Sirot et al. 1999).

Parmi les bêta-lactamases de classe A, nous retrouvons également les cefotaximases, enzymes ayant évolué à partir des TEM pour élargir leur spectre d'action aux céphalosporines de 3^{ème} génération (Salverda, De Visser et al. 2010). Par exemple, l'enzyme CTX-M16 (identifiant Genbank de la protéine: AAK32961) hydrolyse préférentiellement la cefotaxime par rapport aux différentes pénicillines(Bonnet, Dutour et al. 2001).

L'enzyme PC1 (identifiant UniProtKB/Swiss-Prot de la protéine: P00807) codée par le gène *blaZ* est une bêta-lactamase de classe A à serine catalytique (Ambler 1975, Knott-Hunziker, Waley et al. 1979, Kemal and Knowles 1981, Herzberg and Moult 1987). Cette enzyme produite par *Staphylococcus aureus* coagulase positif et *Bacillus subtilis* methi-R hydrolyse le noyau bêta-lactamine de la méthicilline (Novick 1963).

### Aminoglycosides

Les aminoglycosides, aussi dénommés aminosides, sont des sucres aminés liés par un pont glycosidique à un noyau central aminocyclitol. La structure de ce noyau central permet de distinguer trois groupes : les streptomycines (Streptomycine), les désoxystreptamines (Kanamycine, Amikacine, Gentamicine) et les fortimicines (Dactamicine).

Le spectre antibiotique théorique des aminoglycosides est très large incluant des bactéries Gram-négatif aérobies (bacilles, cocci et coccobacilles), les staphylocoques coagulase positif ou négatif, ainsi que les bacilles Gram-positif. En outre, la streptomycine est active sur *Mycobacterium tuberculosis* et l'amikacine sur les mycobactéries atypiques (*Mycobacterium avium intracellulare,* etc.), ainsi que *Nocardia asteroides.* Les aminoglycosides ont une activité synergique avec les bêta-lactamines et la vancomycine.

Les aminoglycosides sont généralement administrés par voie parentérale, car peu ou pas absorbés au niveau digestif. Ils sont éliminés sous forme inchangée principalement dans les urines par filtration glomérulaire (85 à 90 % de la dose administrée est éliminée en 24 heures), ainsi qu'à moindre degré dans la bile sans cycle entéro-hépatique (de l'ordre de 0,5 à 2 % de la dose administrée selon l'antibiotique) (Leroy, Humbert et al. 1978). Cette excrétion digestive des aminoglycosides a pour conséquence la sélection de bactéries résistantes, ce qui expose à un risque faiblement accru de colite pseudomembraneuse par *Clostridium difficile* (Anand, Bashey et al. 1994).

L'effet bactéricide des aminoglycosides est rapide sur de nombreux agents pathogènes. Les aminoglycosides se fixent sur la sous-unité ribosomale 30S procaryote et altèrent la fidélité de la traduction bactérienne (Poehlsgaard and Douthwaite 2005). La plupart des aminoglycosides se fixe à l'ARN ribosomique 16S, le constituant ARN de la sous-unité ribosomale 30S, au niveau du site de décodage (site A).

Les bactéries peuvent acquérir une résistance aux aminoglycosides par trois mécanismes principaux qui peuvent coexister dans une même cellule (Ramirez and Tolmasky 2010). Un premier mécanisme d'origine chromosomique aboutit à une réduction d'affinité de l'aminoglycoside pour sa cible ribosomale, l'ARN ribosomal 16S, par le biais de sa méthylation (Doi and Arakawa 2007). Un second mécanisme repose sur un défaut de perméabilité cellulaire par modification de la perméabilité membranaire (Hancock 1981) ou un efflux de l'aminoglycoside hors de la cellule par un mécanisme actif (Aires, Kohler et al. 1999). Enfin, l'inactivation enzymatique est le mécanisme le plus fréquemment observé. Les gènes codant pour ces enzymes sont portés par des plasmides et/ou des transposons. Il s'agit donc d'une résistance transférable et donc souvent épidémique en milieu hospitalier. Ces enzymes catalysent la liaison irréversible de l'aminoglycoside à différents groupements chimiques et sont regroupées en trois grandes classes (Shaw, Rather et al. 1993, Ramirez and Tolmasky 2010). Les phosphotransférases (APH) qui catalysent une réaction de transfert d'un radical phosphate de l'ATP ou du GTP, les nucléotidyltransférases (ANT) permettent le transfert d'un radical adényl et utilisent l'ATP comme substrat et les acétyltransférases (AAC) transfèrent un radical acétyle utilisant l'acétyl-CoA comme substrat. Chaque classe enzymatique comporte différentes sous-classes suivant le substituant de l'aminoglycoside. Une même enzyme peut inactiver plusieurs aminoglycosides présentant des sites moléculaires identiques.

AAC(6')-lb-cr (identifiant Genbank de la protéine : ABC17627.1) est une enzyme appartenant à la famille des N-acétyltransférases naturellement produite par des entérobactéries. Elle catalyse l'acétylation d'un groupement -NH₂ en position 6', ce qui confère à cette enzyme un profil de résistance identique à celui d'autres enzymes de la sous-classe AAC(6') (Robicsek, Strahilevitz et al. 2006). Tout comme les autres enzymes de la famille AAC(6'), AAC(6')-lb-cr est active sur une large gamme d'aminoglycosides parmi lesquels l'amikacine et les énantiomères Cla et C2 de la gentamicine, mais a une activité très faible sur l'énantiomère C1 de la gentamicine (Robicsek, Strahilevitz et al. 2006). AAC(6')-lb-cr, qui résulte probablement de l'évolution de l'enzyme AAC(6')-lb par mutation des résidus Trp102Arg et Asp179Tyr. Ceci a pour conséquence l'acquisition d'une activité enzymatique vis-à-vis de la ciprofloxacine, une fluoroquinolone, activité qui est nettement plus faible pour la norfloxacine et la péfloxacine.

### Fluoroquinolones

Les fluoroquinolones (e.g. norfloxacine, ofloxacine, ciprofloxacine et péfloxacine) constituent une large classe d'antibiotiques bactéricides de synthèse qui dérivent des quinolones par modifications chimiques, notamment addition d'un atome de fluor.

Les fluoroquinolones sont des antibiotiques à large spectre qui diffère d'un antibiotique à l'autre. Le spectre des fluoroquinolones inclut notamment des bacilles Gram-négatif (*Salmonella* spp., *Escherichia* spp., *Shigella* spp., *Proteus* spp., *Enterobacter* spp*., Helicobacter pylori*)*,* des cocci gram positif (staphylocoque coagulase positif et négatif, streptocoque, entérocoque), des cocci gram négatif (gonocoque, méningocoque) et des bacilles Gram positif.

La distribution tissulaire des fluoroquinolones est excellente y compris dans le liquide céphalo-rachidien, la prostate, les os, et la bile. Les fluoroquinolones sont donc largement utilisées en cas d'infections tissulaires (méningite, pneumopathie, infection ostéoarticulaire, infection urinaire haute ou prostatique,etc.). Par contre, le taux sérique de fluoroquinolones est souvent bas et peut même être inférieur à la CMI (Concentration Minimale Inhibitrice) de certains germes, favorisant l'émergence de résistances bactériennes.

La voie d'élimination des fluoroquinolones dépend du produit utilisé. Elle est principalement hépatique pour la péfloxacine, alors qu'elle est principalement rénale pour l'ofloxacine et les autres fluoroquinolones. Leur élimination biliaire et digestive explique le risque de colite pseudomembraneuse par *Clostridium difficile,* notamment par la souche Bl/NAP1/027 qui est particulièrement virulente (Vardakas, Konstantelias et al. 2012, Deshpande, Pasupuleti et al. 2013, Slimings and Riley 2013).

Les fluoroquinolones ont pour cible l'ADN gyrase et les topoisomérases II et IV bactériennes. Elles forment un complexe irréversible entre ces enzymes et l'ADN bactérien, ce qui empêche la réplication de l'ADN et entraine la mort de la bactérie.

Quatre mécanismes de résistance aux quinolones ont été caractérisés (Robicsek, Jacoby et al. 2006) : (*i*) une augmentation d'activité de pompes à efflux, ce qui entraine une diminution de la concentration intracellulaire de l'antibiotique (Morita, Kodama et al. 1998), (*ii*) la production de protéines se liant à l'ADN gyrase ou la topoisomérase IV, les protégeant ainsi de la fixation des fluoroquinolones (Robicsek, Jacoby et al. 2006), (*iii*) une modification de l'ADN gyrase ou de la topoisomérase IV responsables des résistances à haut niveau par réduction d'affinité des fluoroquinolones pour leurs cibles (Robicsek, Jacoby et al. 2006), (*iv*) enfin, la production d'une aminoglycoside N-acétyltransférase, AAC(6')-lb-cr (identifiant Genbank de la protéine : ABC17627.1), susceptible de cataboliser la modification de la ciprofloxacine, ainsi qu'à moindre degré de la norfloxacine et la péfloxacine (Robicsek, Strahilevitz et al. 2006).

Ces mécanismes de résistance, dont la fréquence augmente rapidement, ont été identifiés dans de nombreuses espèces bactériennes, parmi lesquelles des *Staphylocoques aureus* coagulase positif, des streptocoques bêta-hémolytiques et des entérocoques (Robicsek, Jacoby et al. 2006).

### Macrolides

Les macrolides constituent une famille homogène d'antibiotiques naturels et semi-synthétiques dont le nombre de représentants est relativement limité (e.g. érythromycine, spiramycine, josamycine, clarithromycine, roxithromycine, azithromycine et télithromycine).

Le spectre d'activité des macrolides est large et inclut des cocci Gram positif aérobies (streptocoque, pneumocoque, staphylocoque et entérocoque) et anaérobies, des cocci gram négatif, certains bacilles gram négatif comme *Helicobacter pylori,* et différentes bactéries à réplication intracellulaire stricte comme *Legionella pneumophilia, Mycoplasma pneumoniae* et *Mycoplasma urealyticum, Clamydiae pneumoniae* et *Clamydiae trachomatis.* Par contre, les entérobactéries sont intrinsèquement résistantes aux macrolides, leur membrane cellulaire externe empêchant le passage de molécules hydrophobes comme les macrolides.

Les macrolides se distribuent de façon large dans l'organisme, à l'exception du liquide céphalorachidien, du cerveau et des urines. Leur élimination est essentiellement biliaire, après métabolisation par le cytochrome P450. L'administration de macrolides est associée à un risque accru de colite pseudomembraneuse secondaire à la sélection de *Clostridium difficile* (Gilbert 1994, Bartlett 1996, Hogenauer, Hammer et al. 1998, Wistrom, Norrby et al. 2001).

Les macrolides sont des inhibiteurs qui se lient de façon réversible à la sous-unité 50S des ribosomes procaryotes, au niveau du site P. Ils empêchent ainsi le transfert et la dissociation du complexe peptidyl-ARNt (ARN de transfert) depuis le site P vers le site A, et ainsi inhibent l'élongationde la chaîne peptidique (Tenson, Lovmar et al. 2003).

Trois mécanismes de résistance acquise aux macrolides sont connus (Roberts, Sutcliffe et al. 1999, Roberts 2008): (*i*) une modification de la cible par méthylation ou mutation de l'ARN ribosomal bactérien 23S, qui compose l'ARN ribosomal 50S. Ce mécanisme de résistance, qui est celui le plus fréquemment rencontré, est macrolides, lincosamides et les streptogramines B (Weisblum 1995), (*ii*) production par cellule de pompes qui effluent l'antibiotique hors de la cellule, ce qui entraine une diminution de sa concentration intracellulaire, (*iii*) une inactivation par des enzymes (érythromycine estérases et érythromycine phosphotransférases) modifiant les macrolides au point de réduire fortement leur affinité pour le ribosome. Ce type de résistance est également transmis par des éléments génétiques mobiles.

Les enzymes érythromycine estérases inactivent les macrolides par hydrolyse du noyau macrolactone. La plupart de ces enzymes appartient à deux sous-classes : ereA (Ounissi and Courvalin 1985) et ereB (Arthur, Autissier et al. 1986). L'enzyme ereB (identifiant UniProtKB/Swiss-Prot de la protéine : P05789.1) a un spectre très large (érythromycine, clarithromycine, roxithromycine et azithromycine), la télithromycine étant par contre résistante à l'hydrolyse (Morar, Pengelly et al. 2012).

### Tétracyclines

Les cyclines ou tétracyclines constituent une famille d'antibiotiques bactéricides dérivés de la tétracycline (e.g. chlorotétracycline, doxycycline, minocycline). Ces molécules ont pour caractéristique de posséder quatre cycles accolés, d'où leur nom.

Le spectre d'activité des tétracyclines s'étend à de nombreuses bactéries gram positif et négatif, aérobies et anaérobies. Les tétracyclines sont également actifs sur des agents pathogènes non conventionnels tels que *Mycoplasma* spp., *Chlamydia* spp., rickettsies tréponèmes, ainsi que certains protozoaires (Babesia divergens, Babesia microti, Theileria parva). Les mycobactéries, et les entérobactéries des genres *Proteus* et *Pseudomonas* sont naturellement résistants.

La plupart des tétracyclines est éliminée par filtration glomérulaire sous forme active à l'exception de la chlorotétracycline. Les tétracyclines sont également éliminées par voie biliaire avec cycle entéro-hépatique. La prise de tétracyclines est également associée à un risque accru de colite pseudomembraneuse secondaire à la sélection de *Clostridium difficile* (Gilbert 1994, Bartlett 1996, Hogenauer, Hammer et al. 1998, Wistrom, Norrby et al. 2001).

Les antibiotiques de la famille des cyclines inhibent la fixation de l'aminoacyl-ARNt du site A de la sous-unité ribosomale30S et inhibent ainsi la traduction (Chopra and Roberts 2001). Au moins trois mécanismes de résistance ont été identifiés : (*i*) l'expression de protéines d'efflux, ce qui entraine une diminution de la concentration intracellulaire de tétracyclines, (*ii*) une modification enzymatique de la cible moléculaire, ce qui empêche la liaison des tétracyclines, (*iii*) une inactivation par TetX (identifiant GenBank de la protéine : AAA27471.1), une tétracycline oxydoréductase NADPH-dépendante (Speer, Bedzyk et al. 1991). Cette enzyme induit une résistance bactérienne vis-à-vis de toutes les tétracyclines.

### Lincosamides

Les lincosamides (lincomycine et son dérivé semi-synthétique clindamycine) sont des antibiotiques bactériostatiques qui, bien que de structure chimique distincte de celle des macrolides, possèdent un mode d'action similaire et sont regroupés avec les spectogramines B dans une même famille dite MLS (macrolides, lincosamides et streptogramines).

Le spectre d'action des lincosamides recouvre à la majorité des bactéries gram positif (e.g. *Bacillus cereus, Corynebacterium diphtheriae, Enterococcus faecium, Staphylococcus* méti-S et méti-R, *Streptococcus* B, *Streptococcus* non groupable, *Streptococcus pneumoniae, Streptococcus pyogenes)* à quelques exceptions près telles *Streptococcus faecalis,* ainsi que de nombreuses bactéries anaérobies à l'exception notable de *Clostridium difficile* (e.g. *actinomyces, bacteroides, fusobacterium, Propionibacterium acnes*)*.* La plupart des bactéries gram négatifs sont résistantes en dehors de quelques rares exceptions (i.e. *Bordetella pertussis, Campylobacter, Chlamydia, Helicobacter,* et *Legionella*).

Les lincosamides sont principalement éliminées par voie urinaire, ainsi qu'à moindre degré par voie biliaire avec cycle entéro-hépatique. Leur élimination par voie digestive et leur spectre d'activité antibactérien explique la fréquence élevée de colites pseudomembraneuses par sélection de *Clostridium difficile.*

Comme les macrolides, les lincosamides inhibent la traduction bactérienne en se fixant de façon réversible à la sous-unité 50S des ribosomes, au niveau des sites A et P (Tu, Blaha et al. 2005). Trois mécanismes de résistance acquise aux lincosamides sont connus (Roberts, Sutcliffe et al. 1999) : (*i*) une modification de la cible par méthylation ou mutation de l'ARN ribosomal bactérien 23S, qui compose l'ARN ribosomal 50S. Ce mécanisme de résistance qui est celui le plus fréquemment rencontré, est commun aux macrolides, lincosamides et les streptogramines B (Weisblum 1995), (*ii*) expression par la bactérie de pompes qui effluent l'antibiotique hors de la cellule, ce qui entraine une diminution de sa concentration intracellulaire, (*iii*) une inactivation enzymatique des lincosamides qui réduit leur affinité pour leur cible moléculaire.

Les enzymes susceptibles d'inactiver les lincosamides appartiennent à la famille des lincomycine nucleotidyltransferases lnu(A) ou lin(A) chez staphylocoques, lnu(B) ou lin(B) chez *Enterococcus faecium,* et linB-like chez des anaérobies (Leclercq, Brisson-Noel et al. 1987, Bozdogan, Berrezouga et al. 1999). Par exemple, l'enzyme lnu(B) (identifiant UniProtKB/TrEMBLde la protéine : Q9WVY4) catalyse le transfert d'un résidu adenyl sur le groupement hydroxyle en position 3 de la lincomycine et la clindamycine (Bozdogan, Berrezouga et al. 1999).

### Contrer les effets secondaires de ces antibiotiques

Il est connu de l'art antérieur des inhibiteurs d'antibiotiques visant à diminuer les effets secondaires précédemment présentés au niveau intestinal. Ces inhibiteurs sont essentiellement ciblés contre les bêta-lactamines et consistent principalement en des bêta-lactamases administrées par voie orale pour leur diffusion au niveau du tractus intestinal.

Le brevet EP0671942 se rapporte à une application médicale, à un procédé médical et à une préparation pharmaceutique. Cette invention permet de cibler l'action des bêta-lactamines administrés par voie parentérale et de réduire leurs effets secondaires grâce à l'inactivation d'une partie de l'antibiotique dans le tractus digestif, en administrant, séparément de l'antibiotique ou simultanément à lui, une enzyme, telle que la β-lactamase, par voie orale qui entraîne la dégradation de l'antibiotique.

La demande de brevet EP2086570 utilise d'une manière similaire une bêta-lactamase de classe A, plus spécifiquement l'enzyme P1A, pour réduire les effets secondaires intestinaux associés à une antibiothérapie combinant une bêta-lactamine et un inhibiteur de bêta-lactamase.

L'efficacité de la bêta-lactamase P1A a été évaluée pré-cliniquement et cliniquement. Cette enzyme hydrolyse les aminopénicillines (e.g. amoxicilline) et les ureidopénicillines (e.g. pipéracilline), mais est sensible aux inhibiteurs des bêta-lactamines. Son administration orale chez le chien, simultanément à une administration parentérale d'ampicilline, réduisait de manière dose dépendante la quantité d'ampicilline détectée dans la lumière du jéjunum de ces animaux. De plus, la présence de la bêta-lactamase P1A n'était pas détectée au niveau circulant et les concentrations sériques d'ampicilline n'étaient pas significativement modifiées (Harmoinen, Vaali et al. 2003, Harmoinen, Mentula et al. 2004). L'administration de cette enzyme par voie orale à des souris recevant une administration parentérale de pipéracilline a permis de réduire de manière très significative le nombre de microorganismes résistants à cet antibiotique dans les selles (VRE*-Enterococcus faecium, Klebsiella pneumoniae* et *Candida glabrata*) (Stiefel, Pultz et al. 2003, Mentula, Harmoinen et al. 2004).

Enfin, une étude clinique a montré que l'administration orale de la bêta-lactamase P1A permettait de diminuer le nombre d'isolats d'entérobactéries résistantes à l'ampicilline chez les volontaires sains après administrations intraveineuses d'ampicilline, ainsi que le nombre d'isolats d'entérobactéries résistantes à d'autres classes d'antibiotiques, en particulier résistants aux tétracyclines (Tarkkanen, Heinonen et al. 2009).

Des enzymes bêta-lactamases recombinantes appartenant à d'autres classes de bêta-lactamases ont également été développées. Le brevet EP2038411 décrit une métallo bêta-lactamase mutante et son procédé de synthèse pour réduire les effets secondaires intestinaux chez des patients recevant des carbapénemes.

Le brevet WO2003/068975 décrit une molécule hybride protéique comportant deux enzymes capables d'inhiber l'activité d'au moins un antibiotique: une béta-lactamase et une aminoglycoside acetyl transférase. Cette molécule est proposée pour lutter contre les maladies nocosomiales.

On connaît également de l'art antérieur des formes galéniques pour cibler la délivrance de ces enzymes au niveau de l'intestin. Le brevet FR2843302 décrit des formes galéniques multi-particulaires administrables par voie orale pour la délivrance limitée au colon d'enzymes capables d'inhiber des macrolides, par exemple l'érythromycine estérase.

### Inconvénient des solutions de l'art antérieur

Les solutions de l'art antérieur utilisent des enzymes ne ciblant qu'un seul type d'antibiotique, les bêta-lactamases dégradant les bêta-lactamines ou l'érythromycine estérase inhibant les macrolides.

Différents antibiotiques sont généralement utilisés chez les malades en milieu hospitalier, voire même combinés chez un même malade. L'inhibition d'une seule classe d'antibiotique n'a donc qu'un effet attendu modeste ou minime sur l'émergence de souches bactériennes résistantes.

A titre d'exemple, les infections sévères à bacilles gram négatifs sont fréquemment traitées par association d'une céphalosporine de 3^{e} génération et d'un aminoglycoside, alors que les pneumopathies atypiques sont volontiers traitées par association amoxicilline-acide clavulanique et macrolide ou tétracycline.

Les inhibiteurs d'antibiotiques décrits dans l'art antérieur étant dirigés contre une unique classe d'antibiotiques, ils ne permettent pas de prévenir efficacement l'émergence de souches bactériennes résistantes dans un environnement où sont utilisées simultanément différentes classes d'antibiotiques, notamment en milieu hospitalier.

### Solution apportée par l'invention

La présente invention se propose de remédier aux inconvénients de l'art antérieur en proposant une molécule protéique hybride comportant deux enzymes bêta-lactamases aptes à inhiber l'activité d'au moins un antibiotique, chaque protéine ayant des caractéristiques biochimiques différentes, lesdites enzymes étant liées entre elles. Dans un mode de réalisation, lesdites enzymes aptes à inhiber l'activité d'au moins un antibiotique sont liées entre elles de manière covalente.

La molécule protéique hybride conformément à l'invention inhibe l'activité d'au moins un antibiotique pour diminuer les effets secondaires intestinaux des antibiotiques, tels que les diarrhées aiguës induites par les antibiotiques et les infections nosocomiales secondaires à une antibiothérapie parentérale.

L'on comprend que la présente invention présente donc un spectre d'action élargi par rapport aux solutions de l'art antérieur.

Préférentiellement, la molécule protéique hybride de la présente invention est administrée oralement. La molécule protéique hybride conforme à l'invention comporte deux protéines aptes à inhiber deux béta-lactamines différentes, et notamment les associations les plus couramment utilisées en pratique clinique. Ainsi, l'administration de la seule molécule protéique hybride permet de cibler plusieurs antibiotiques, ce qui réduit le nombre de produits prescrits.

On entend par « molécule protéique hybride », une protéine hybride constituée par l'assemblage artificiel de deux ou plusieurs chaines polypeptidiques.

On entend par « inhibition de l'activité antibiotique », tout processus aboutissant à une réduction ou une suppression de l'activité biologique de l'antibiotique considéré, ce qui inclus, sans être limité, à une fixation de l'antibiotique à une autre molécule de manière spécifique (e.g. grâce à un anticorps monoclonal ou un fragment d'anticorps monoclonal) ou aspécifique (e.g. par adsorption), une modification de l'antibiotique par addition enzymatique ou non-enzymatique d'un groupement chimique, ou l'hydrolyse de l'antibiotique par un mécanisme antibiotique ou non-antibiotique.

L'on comprend que chaque enzyme dégrade préférentiellement deux antibiotiques différents.

La présente invention démontre, de manière inattendue, que les molécules protéiques hybrides comme définies précédemment combinent les activités fonctionnelles respectives des protéines les constituant, ce qui aboutit à l'élargissement de leur spectre d'action et la potentialisation de leur efficacité dans un environnement hospitalier soumis à forte pression de sélection.

Plusieurs types de protéines capables d'inactiver un ou plusieurs antibiotiques peuvent être utilisés pour constituer cette molécule protéique hybride, incluant des fragments d'anticorps monoclonaux (par exemple ScFv monovalent ou bivalent), des enzymes de type hydrolase ou catalysant d'autres types de modifications.

Avantageusement, la séquence de l'une au moins des protéines constitutives de la protéine hybride présente une homologie de séquence protéique d'au moins 40% avec les SEQ ID1 à SEQ ID3. Cette homologie de séquence est déterminée au moyen du logiciel CLUSTALW2 ou CLUSTALOMEGA avec des paramètres standards de calculs (Thompson, Higgins et al. 1994, Larkin, Blackshields et al. 2007). Préférentiellement, cette homologie de séquence est d'au moins 50%, encore plus préférentiellement d'au moins 60% avec les séquences SEQ ID1 à SEQ ID3.

Les protéines ou enzymes constitutives de la molécule protéique hybride peuvent comporter aucune, une ou plusieurs glycosylations.

Selon un mode de réalisation, les protéines ou enzymes sont fusionnées en une protéine unique monocaténaire.

Les inventeurs ont de façon inattendue et surprenante constaté que la molécule protéique hybride présente une efficacité à l'encontre de ses antibiotiques cibles équivalente aux enzymes la constituant prises isolément.

Ces protéines hybrides peuvent être obtenues artificiellement sous la forme d'une seule chaine protéique résultant de la traduction d'un unique cadre de lecture obtenu soit par fusion directe des cadres de lecture respectifs des protéines la constituant, soit par l'intermédiaire d'une séquence nucléotidique codant pour un adaptateur constitué d'un ou plusieurs acides-aminés. Cet adaptateur, réputé inerte et sans action biologique particulière, peut être souple, semi-rigide ou rigide (Chen, Zaro et al. 2013).

Selon un autre mode de réalisation, les protéines ou enzymes sont liées entre elles de manière covalente par cross-linking.

Ces protéines hybrides peuvent également être obtenues artificiellement par cross-linking au moyen d'agents chimiques (Chen, Nielsen et al. 2013), d'une catalyse enzymatique (Paguirigan and Beebe 2007), d'une exposition aux ultra-violets (Fancy and Kodadek 1999), ou tout autre procédé aboutissant à la formation d'une liaison covalente. Alternativement, ces protéines hybrides peuvent être obtenues par assemblage entre elles de plusieurs sous-unités protéiques de manière non covalente au moyen de ligands de forte affinité, tels que par exemple le complexe (strept)avidin/biotine (Schultz, Lin et al. 2000, Lin, Pagel et al. 2006, Pagel, Lin et al. 2006).

La présente invention selon un autre aspect porte sur une composition pharmaceutique à usage humain ou vétérinaire comportant la molécule protéique hybride conformément à l'invention pour une utilisation dans la prévention de modifications de la flore intestinale.

Avantageusement, l'invention porte sur une composition pharmaceutique pour une utilisation dans la prévention des infections nosocomiales.

Avantageusement, l'invention porte sur une composition pharmaceutique pour une utilisation dans la prévention des diarrhées associées à la prise d'antibiotiques.

Selon un mode de réalisation préférentiel, la composition pharmaceutique comportant la molécule protéique hybride conforme à l'invention est sous une forme galénique pour une administration par voie orale.

L'on comprend que l'invention porte sur une composition pharmaceutique comportant une molécule protéique hybride pour la prévention de modifications de la flore intestinale normale dans le but de prévenir la dissémination de bactéries résistantes dans l'environnement, réduire le risque et/ou la gravité des infections nosocomiales causées par des microorganismes multi-résistants, ainsi que de diminuer la fréquence et la gravité de diarrhées induites par les antibiotiques.

Avantageusement, la composition pharmaceutique est destinée à une administration chez l'Homme, y compris en pédiatrie. L'on comprend que la composition pharmaceutique selon la présente invention peut être administrée, préférentiellement par voie orale, avant, simultanément ou après à une administration d'un ou plusieurs antibiotiques.

Selon un autre mode de réalisation, la composition pharmaceutique est à usage vétérinaire pour une utilisation dans la prévention de la dissémination de résistance bactérienne aux antibiotiques par les animaux domestiques de compagnie et chez les animaux d'élevage recevant des antibiotiques.

La composition pharmaceutique selon la présente invention comporte également toute substance pharmaceutiquement acceptable telle que des adjuvants, des excipients, des stabilisants, des sels, des texturants, des liants, des lubrifiants, des agents d'enrobage, des colorants, des arômes, ou tout autre agent classiquement utilisé et connu de l'Homme du métier.

La composition pharmaceutique selon l'invention peut être sous forme solide ou liquide, non limitativement sous forme de gel, de sirop, de gélules, de comprimés, de suspension ou d'émulsion.

Selon une variante, la composition pharmaceutique comporte au moins un agent gastroprotecteur.

L'on entend par « agent gastroprotecteur », un agent résistant aux sucs gastriques pour une libération différée de la molécule protéique hybride au niveau de l'intestin, préférentiellement au niveau du duodénum et du jéjunum. L'agent gastroprotecteur peut également être un agent d'enrobage tel que non limitativement despolyméthacrylates (par exemple Eudragit®), des polymères à base de cellulose (éthers de cellulose : par exemple Duodcell®, esters de cellulose), des copolymères d'acétate de polyvinyl (par exemple Opadry®) ou tout autre agent d'enrobage ou d'encapsulation ou procédé connus de l'Homme du métier.

Selon un autre aspect, l'invention porte sur un procédé de production de la molécule protéique hybride dans un organisme recombinant vivant non humain.

L'on entend par « organisme recombinant vivant », toute cellule apte à être génétiquement modifiée pour la production de la molécule protéique hybride.

De façon non limitative, on entend par « cellule apte à être génétiquement modifiée », toute cellule procaryote (e.g. *Escherichia coli*), levure (e.g. *Saccharomyces cerevisiae, Pichia pastoris, Kluyveromyces lactis, Yarrowia lipolytica*), cellules d'insecte infectées ou non par un baculovirus, cellules de mammifères (e.g. CHO, CHO-K1, HEK293, HEK293T).

### Description

La présente invention sera mieux comprise à la lumière de la description des exemples non limitatifs suivants.

### Description des figures

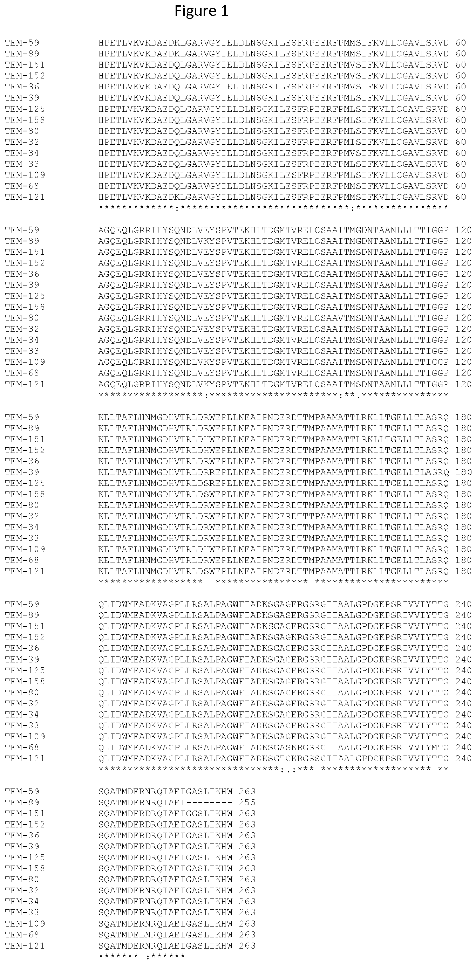

La figure 1 décrit un alignement de séquences protéiques de différentes bêta-lactamases appartenant à la famille des IR-TEM (Amber Classe A). L'alignement a été effectué avec le logiciel CLUSTALW2 sur le site https://www.ebi.ac.uk/Tools/msa/clustalw2/ en utilisant les paramètres par défaut. La figure 1 montre par un alignement de plusieurs séquences, que les enzymes IR-TEM présentent >90% d'identité protéique entre elles. Ces enzymes sont relativement bien conservées partageant entre 80 et 99% d'identité de séquence protéique entre elles, et également bien caractérisées (Bonnet 2004).

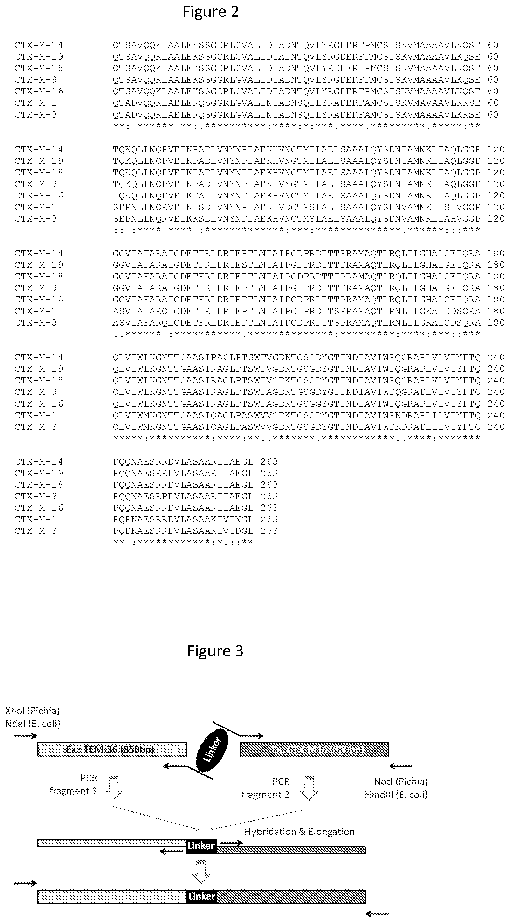

La figure 2 décrit un alignement de séquences protéiques de différentes bêta-lactamases appartenant à la famille des CTX-M cefotaximases (Ambler Classe A). L'alignement a été effectué sur le site https://www.ebi.ac.uk/Tools/msa/clustalw2/ en utilisant les paramètres par défaut du logiciel.
La figure 3 représente une illustration de la technique de PCR utilisée pour générer des protéines hybrides. Deux protéines sont tout d'abord amplifiées séparément avec une séquence commune (aux extrémités 3' et 5' respectivement) constitutive d'un linker. Les deux fragments sont alors hybridés au niveau de la séquence d'ADN complémentaire (celle du linker) et le tout est réamplifié à l'aide d'amorces externes.

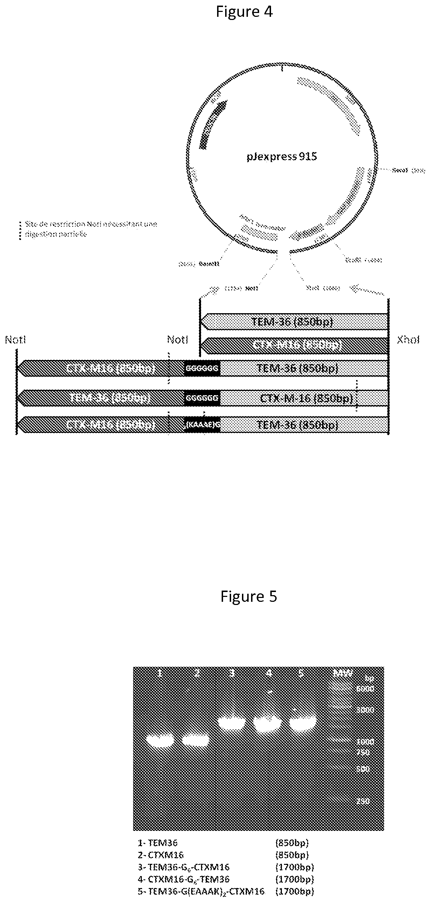

La figure 4 décrit l'ensemble des constructions utilisées pour exprimer les protéines TEM-36 (SEQ ID1), CTXM-16 (SEQ ID2), TEM36-GGGGGG-CTXM16 (SEQ ID8), CTXM16-GGGGGG-TEM36 (SEQ ID9) et TEM36-G(EAAAK)2-CTXM16 (SEQ ID 10) chez *Pichia pastoris.* Le clonage dans le vecteur pJexpress915 a été effectué en Xhol/Notl en utilisant lorsque nécessaire des digestions modérées pour préserver les sites Notl internes à la séquence ADN codante de CTXM 16.
La figure 5 montre les fragments d'ADN clonés en Xhol / Notl codant pour TEM-36 (SEQ ID1), CTXM-16 (SEQ ID2), TEM36-GGGGGG-CTXM16 (SEQ ID8), CTXM16-GGGGGG-TEM36 (SEQ ID9) et TEM36-G(EAAAK)2-CTXM16 (SEQ ID 10) et qui ont été obtenus par PCR en utilisant les amorces pJexpress915-SEQ-F et pJexpress915-SEQ-R et le programme suivant (95°C 30 sec - 25 cycles [95°C 30sec - 53°C 45sec -72°C 1min] -72°C 5 min - 4°C).

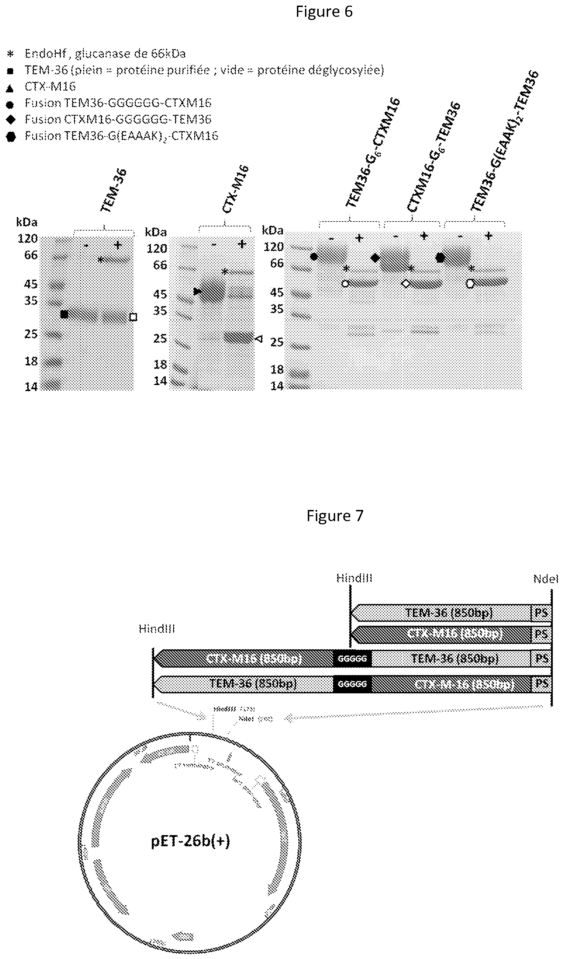

La figure 6 montre les protéines hybrides et leurs enzymes constitutives TEM-36 (SEQ ID1) et CTXM-16 (SEQ ID 2) sur un gel SDS-PAGE 12% telles que obtenues par expression et purification dans *Pichia Pastoris* ainsi qu'après avoir subi un traitement de déglycosylation par EndoHf.
La figure 7 décrit l'ensemble des constructions utilisées pour exprimer les protéines TEM-36 (SEQ ID1), CTXM-16 (SEQ ID2) et les protéines hybrides TEM36-GGGGG-CTXM16, CTXM16-GGGGG-TEM36 chez *Escherichia coli.* Le clonage dans le vecteur pET-26b(+) a été effectué en Ndel/Hindlll.

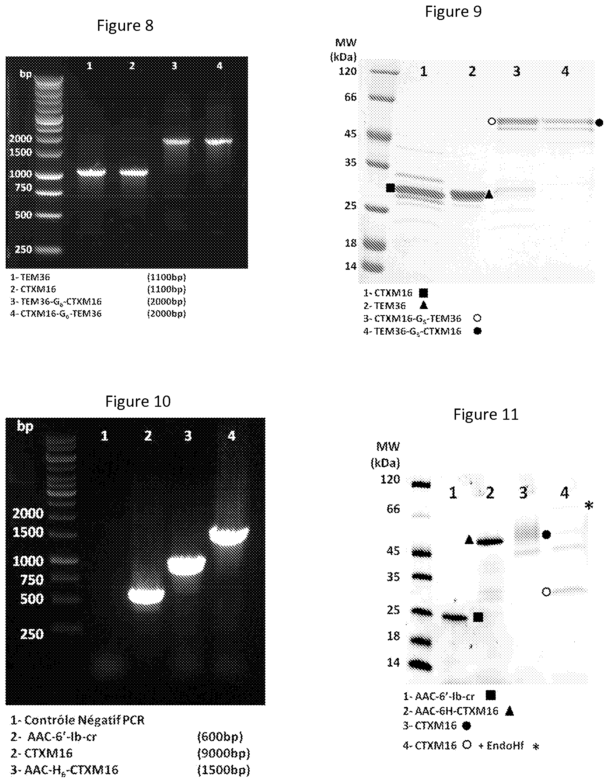

La figure 8 montre les fragments d'ADN clonés en Ndel / Hindlll dans le pET-26b(+) codant pour TEM-36 (SEQ ID1), CTXM-16 (SEQ ID2), TEM36-GGGGG-CTXM16, CTXM16-GGGGG-TEM36 et qui ont été obtenus par PCR en utilisant les amorces T7-F et T7-R et le programme suivant (95°C 30 sec - 25 cycles [95°C 30sec - 55°C 45sec -72°C 1min] -72°C 5 min - 4°C).
La figure 9 montre les protéines hybrides (TEM36-G₅-CTXM16 et CTXM16-Gs-TEM36) et leurs enzymes constitutives TEM-36 (SEQ ID1) et CTXM-16 (SEQ ID 2) sur un gel SDS-PAGE 12% telles que obtenues par expression dans *E. coli* et purification à partir de la fraction périplasmique.
La figure 10 (hors du cadre de l'invention) montre les fragments d'ADN codant pour AAC-6'-Ib-cr (SEQ ID4), CTXM-16 (SEQ ID2), et AAC-H6-CTXM16 qui ont été obtenus dans la construction de la fusion AAC-H6-CTXM16 (SEQ ID 18).
La figure 11 (hors du cadre de l'invention) montre la protéine hybride AAC-H₆-CTXM16 et ses enzymes constitutives AAC-6'-Ib-cr (SEQ ID4) et CTXM-16 (SEQ ID 2) sur un gel SDS-PAGE 12% telles que obtenues après expression et purification dans *E. coli* pour AAC-6'-Ib-cr et la protéine hybride et *P. pastoris* pour CTXM16.

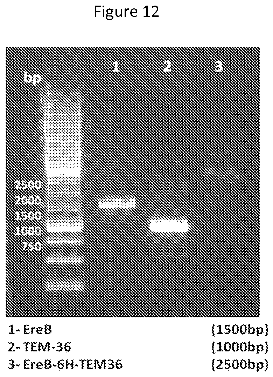

La figure 12 (hors du cadre de l'invention) montre les fragments d'ADN codant pour EreB (SEQ ID5), TEM36 (SEQ ID1), et EreB-H6-TEM36 qui ont été obtenus dans la construction de la fusion EreB-H6-CTXM16 (SEQ ID 20).

### Description du listage de séquences

La table 1 ci-après récapitule les séquences du listage de séquence et fait correspondre à chaque identifiant SEQ-ID (N° ID dans la table) le type de séquence, son nom, son origine, son organisme d'expression, sa taille.

**Table 1. Identification des séquences 1 à 22 du listage de séquences**

| Nature | N° ID | Nom commun | Identifiant_{(GeneBank/Swiss-Prot/UniProtKB)} | Hôte d'origine | Micro-organisme d'expression | Longueur | Description |
|---|---|---|---|---|---|---|---|
| Protéique | 1 | TEM36 | Non disponible | *Escherichia coli* | *E. coli*/*P. pastoris* | 263 aa | β-lactamase (aminopenicillinase) |
| Protéique | 2 | CTXM16 | AAK32961 | *Escherichia coli* | *E. coli*/*P. pastoris* | 263 aa | β-lactamase (cefotaximase) |
| Protéique | 3 | PC1 | P00807 | *staphylococcus aureus* | *E. coli*/*P. pastoris* | 257 aa | β-lactamase (methicillinase) |
| Protéique | 4 | AAC-6'-lb-cr | ABC17627.1 | *Escherichia coli* | *E. coli*/*P. pastoris* | 199 aa | fluroquinolone acetylating aminoglycoside acetyltransferase |
| Protéique | 5 | EreB | P05789.1 | *Escherichia coli* | *E. coli*/*P. pastoris* | 419 aa | Erythromycine estérase |
| Protéique | 6 | TetX | AAA27471.1 | *Bacteroides fragilis* | *E. coli*/*P. pastoris* | 388 aa | Tétracycline oxydoreductase NADPH-dépendante |
| Protéique | 7 | LnuB (ou linB) | Q9WVY4 | *Enterococcus faecium* | *E. coli*/*P. pastoris* | 267 aa | Lincomycine nucleotidyltransferase |
| Protéique | 8 | TEM36-G₆-CTXM16 | Non applicable | Artificielle | *Pichia Pastoris* | 532 aa | Fusion TEM36 et CTXM16 avec linker flexible 6 Glycines |
| Protéique | 9 | CTXM16-G₆-TEM36 | Non applicable | Artificielle | *Pichia Pastoris* | 532 aa | Fusion CTXM16 et TEM36 avec linker flexible 6 Glycines |
| Protéique | 10 | TEM36-G(EAAAK)₂-CTXM16 | Non applicable | Artificielle | *Pichia Pastoris* | 537 aa | Fusion TEM36 et CTXM16 avec linker rigide G(EAAAK)₂ |
| Nucléotidique | 11 | TEM36 | Non disponible | *Escherichia coli* | *E. coli*/*P. pastoris* | 4158 bp | Séquence totale du vecteur pJexpress915 avec l'insert TEM36 cloné en XhoI/NotI |
| Nucléotidique | 12 | CTXM16 | AAK32961 | *Escherichia coli* | *E. coli*/*P. pastoris* | 900 bp | Séquence de l'insert CTXM16 cloné en XhoI/NotI |
| Nucléotidique | 13 | TEM36-G₆-CTXM16 | Non applicable | Artificielle | *Pichia Pastoris* | 1680 bp | Séquence de l'insert TEM 36-G₆-CTXM16 cloné en XhoI/NotI |
| Nucléotidique | 14 | CTXM16-G₆-TEM36 | Non applicable | Artificielle | *Pichia Pastoris* | 1634 bp | Séquence de l'insert CTXM16-G₆-TEM36 cloné en XhoI/NotI |
| Nucléotidique | 15 | TEM36-G(EAAAK)₂-CTXM16 | Non applicable | Artificielle | *Pichia Pastoris* | 1648 bp | Séquence de l'insert TEM36-G(EAAAK)₂-CTXM16 cloné en XhoI/NotI |
| Nucléotidique | 16 | CTXM16 | AAK32961 | *Escherichia coli* | *E. coli* | 870 | Insert Ndel/HindIII codant pour CTX-M16 dans le vecteur pET26b+ |
| Nucléotidique | 17 | AAC-6'-lb-cr | ABC17627.1 | *Escherichia coli* | *E. coli* | 627 | Insert Ndel/HindIII codant pour AAC dans le vecteur pJ404. |
| Nucléotidique | 18 | AAC-H6-CTXM16 | Non applicable | Artificielle | *E. coli* | 1416 | Fusion AAC-6'-Ib-cr avec CTX-M16 avec un linker de type Tag polyhistidine |
| Nucléotidique | 19 | EreB | P05789.1 | *Escherichia coli* | *E. coli* | 1287 | Insert Ndel/HindIII codant pour AAC dans le vecteur pET26b+. |
| Nucléotidique | 20 | EreB-H6-TEM36 | Non applicable | Artificielle | *E. coli* | 2076 | Fusion EreB avec TEM36 avec un linker de type Tag polyhistidine |
| Protéique | 21 | AAC-H6-CTXM16 | Non applicable | Artificielle | *E. coli* | 468 | Fusion AAC-6'-lb-cr avec CTX-M16 avec un linker de type Tag polyhistidine |
| Protéique | 22 | EreB-H6-TEM36 | Non applicable | Artificielle | *E. coli* | 688 | Fusion EreB avec TEM36 avec un linker de type Tag polyhistidine |

### Exemple 1 : molécule protéique hybride constituée de la fusion de TEM-36 et de CTX-M16 par un linker flexible

Le premier mode de réalisation de la présente invention décrit la fusion d'une IR-TEM (SEQ ID1; (Chaibi, Sirot et al. 1999)) avec une cefotaximase (SEQ ID2; (Bonnet, Dutour et al. 2001)) par l'intermédiaire d'un linker flexible polyglycine (6 résidus glycine dans cet exemple) et donnant naissance à une protéine hybride (SEQ ID8) capable d'hydrolyser l'amoxicilline même en présence d'acide clavulanique ainsi que la ceftriaxone.

Les séquences nucléotidiques codant pour les protéines TEM-36 (SEQ ID1) et CTX-M16 (SEQ ID2) ont été obtenues commercialement par synthèse de gène dans un vecteur d'expression pour *Pichia pastoris* (*httpsllwww.dna20.comlserviceslgene-svnthesis?gclid=CPnQlp3c9r0CFaoewwodnREAIg*). La séquence nucléotidique de TEM-36 insérée dans le vecteur d'expression correspond à la SEQ ID 11 et la séquence nucléotidique de CTX-M16 insérée dans le vecteur d'expression correspond à la SEQ ID 12. Ces séquences ont alors été amplifiées par PCR (95°C 30 sec - 25 cycles [95°C 30 sec - TM°C45sec - 72°C 1min] - 72°C 5 min - 4°C) en utilisant une ADN polymerase de haute-fidélité (Pfu, Promega) et des amorces partiellement complémentaires et constitutives du linker protéique (GGGGGG dans cet exemple) comme indiqué dans la Table 2. TEM-36 a été amplifiée en utilisant le couple d'amorce TEM36-F et TEM36-6G-R avec un TM de 57°C. CTXM16 a été amplifiée en utilisant le couple d'amorce 6G-CTXM16-F et CTXM16-R avec un TM de 57°C. Les séquences constitutives du linker GGGGGG sont indiquées en gras souligné dans la table 2.

**Table 2. Liste des amorces utilisées pour construire et séquencer la protéine hybride TEM-36/CTX-M16 à linker rigide.**

| Nom amorce | SEQ ID n° | Séquence5'-3' | Hôte |
|---|---|---|---|
| TEM36-F | 23 | GAGGGTGTCTCTCTCGAG | *Pichia Pastoris* |
| TEM36-6G-R | 24 | **TCCACCTCCACCTCCTCC**CCAATGTTTGATTAGGGA | *Pichia Pastoris* |
| 6G-CTXM16-F | 25 | **GGAGGTGGAGGTGGA**CAGACGTCAGCCGTGCAGCAAAAG | *Pichia Pastoris* |
| CTXM16-R | 26 | GCTAGGCGGCCGCTTTTACAAACCTTCAGC | *Pichia Pastoris* |

Les deux fragments PCR ainsi obtenus ont été hybridés à un TM de 55°C et complémentés pendant 5 cycles de PCR sans amorces (95°C 30sec - 55°C 45 sec - 72°C 2min15) en utilisant une ADN polymérase de haute-fidélité (Pfu, Promega). La séquence totale codant pour la protéine hybride a été réamplifiée après rajout des amorces externes (TEM36-F + CTXM16-R). Ce mode de réalisation des fusions par PCR est schématiquement représenté dans la Figure 3.

La fusion TEM36-GGGGGG-CTXM16 ainsi réalisée a été clonée avec les enzymes de restrictions Xhol et Notl dans le vecteur pJexpress915 (expression *Pichia pastoris*) (Figure 4). La séquence de la construction hybride a été vérifiée dans les 2 sens et correspond à la fusion décrite. La figure 5 récapitule les fragments PCR obtenus avec les amorces pJexpress915-SEQ-F &pJexpress915-SEQ-R.

Les vecteurs d'expressions pJexpress915 exprimant TEM-36, CTX-M16 et la fusion TEM36-GGGGGG-CTXM16 ont été amplifiés dans *Escherichia coli* DH10B en maintenant une pression de sélection Zéocine (Invitrogen) à 25 µg/ml sur milieu LB appauvri en sel (Extrait de Levure 5 g/l ; Tryptone 10 g/l ; NaCl 5 g/l pH=7.5). Pour transformer *Pichia pastoris,* 2 µg de vecteur linéarisé avec l'enzyme Swa I ont été électroporés dans 60 µl de cellules compétentes par un choc à 1500 V. Les cellules transformées sont reprises dans 1 ml de Sorbitol 1 M froid et cultivées 2 heures à 30°C avant étalement (par 200 µl) sur milieu YPD-agar (Extrait de levure 10 g/l ; Peptone 20 g/l ; Dextrose 20 g/L; agar 15g/l - Phosphate 10mM pH=6.8) contenant 100 à 400 µg/ml de Zéocine et de la nitrocéfine (20 µg/ml).

L'expression dans *Pichia pastoris* à partir du vecteur pJexpress915 aboutit à une sécrétion des protéines d'intérêt qui se retrouvent dans le milieu de culture. Sur boîtes YPD-agar contenant de la nitrocéfine, les bêta-lactamases sécrétées induisent un halo d'hydrolyse rouge (λ = 486 nm) autour des colonies qui est représentatif du niveau d'expression. Après 48 heures d'incubation, les transformants présentant la meilleure expression pour chaque construction sont inoculés en tube sterilin contenant 5 ml de YPD et 100 µg/ml de Zéocine et cultivés pendant 48 heures à 30°C et 200 rpm pour l'agitation.

Les pré-cultures sont alors inoculées dans 400 ml (200 ml par erlen baflé) de milieu YPD frais sans pression de sélection antibiotique avec une densité optique initiale de 0.2 à 600 nm. La culture est réalisée 48 heures à 30°C sous une agitation de 100 rpm. Après centrifugation à 10,000 xg pendant 15 minutes à 4°C, le milieu de culture (surnageant) est conservé à 4°C en présence de benzamidine (1 mM final). Les protéines du milieu de culture sont concentrées 10 fois par filtration tangentielle sur une membrane de 10 kDa (Module Vivaflow 10, Sartorius) jusqu'à un volume de 40 ml, puis jusqu'à 10 ml par ultrafiltration sur membrane de 10 kDa (Centricon, Millipore). Les 10 ml sont injectés sur une colonne de chromatographie d'exclusion (Superdex G75 pour les protéines TEM-36 et CTX-M16, Superdex G200 pour la fusion ; colonnes 26^{∗}60 GE Healthcare), et éluées en tampon phosphate de sodium 10 mM pH = 7.0 par fraction de 1 ml et avec un débit de 1 ml/min.

Les fractions présentant la plus forte activité sur nitrocéfine (VWR) et la meilleure pureté (SDS-PAGE) sont combinées et concentrées à environ 0.1-0.5 mg/ml. La teneur protéique des échantillons est mesurée par BCA (Pierce), absorbance à 280 nm et vérification sur gel SDS-PAGE. La figure 6 présente les résultats obtenus.

Pour CTX-M16 et la fusion, qui présentent des sites de N-glycosylation, la glycosylation est vérifiée par coupure des sucres avec l'enzyme EndoHf (New EnglandBiolabs) selon les recommandations du fabricant (sur la nuit à 37°C en conditions non-dénaturantes). Les protéines produites et purifiées ont été confirmées par digestion trypsique et analyse spectrométrie de masse MALDI-TOF.

Comme indiqué par la figure 6, la protéine TEM-36 n'est pas glycosylée et présente une taille compatible avec la taille attendue de 28 kDa, contrairement aux protéines CTX-M16 et les différentes fusions réalisées. Ces dernières ont un poids moléculaire apparent supérieur sur gel SDS-PAGE (45 kDa contre 28 kDa et >66 kDa contre 56 kDa pour CTX-M16 et les fusions respectivement). La coupure avec EndoHf permet d'obtenir des protéines de poids moléculaire attendu (28 kDa et 56 kDa pour CTX-M16 et les fusions respectivement) ce qui confirme que ces protéines sont glycosylées.

Les protéines purifiées ont été testées sur différentes bêta-lactamines (amoxicilline (Apollo Scientific), Augmentin® (GlaxoSmithKline) et Ceftriaxone (Rocéphine®, Roche)). Les dosages sont effectués au pH-STAT (Titrino 2.5, Metrohm) dans un volume réactionnel de 25 ml à 37°C et pH = 7.0. Les substrats sont préparés à 4 g/l dans un tampon Tris 0.3 mM, NaCl 150 mM. L'hydrolyse enzymatique des noyaux bêta-lactame libère un acide et entraine une baisse du pH. Le principe du dosage consiste à compenser cette acidification par ajout de soude 0,1 N de manière à rester à pH = 7.0. Dans ces conditions, une unité correspond à 1 µmole de soude ajoutée par minute, c'est-à-dire une µmole de bêta-lactame hydrolysée par minute. La table 3 ci-dessous récapitule les activités spécifiques mesurées pour chaque protéine sur les trois substrats (moyenne de 6 réplicats).

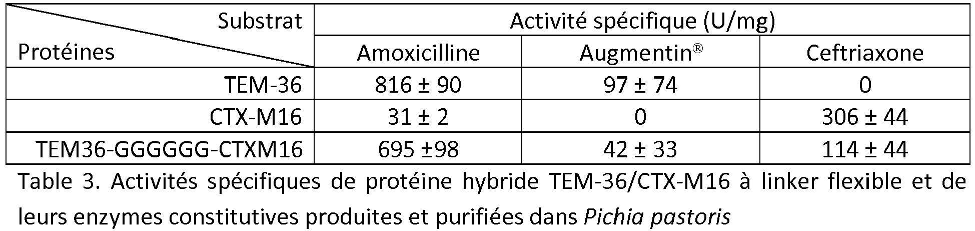

Ces résultats montrent que la fusion entre TEM-36 et CTX-M16 génère une protéine hybride ayant une activité à la fois sur une aminopénicilline même en présence d' inhibiteur de bêta-lactamase et une céphalosporine de troisième génération. Ces deux activités étant décrites comme antagonistes dans la littérature (Ripoll, Baquero et al. 2011), il n'existe aucune enzyme naturelle connue ayant des constantes catalytiques aussi élevées sur ces deux substrats.

### Exemple 2 : molécule protéique hybride constituée de la fusion de CTX-M16 et de TEM-36 par un linker flexible

Le deuxième mode de réalisation de la présente invention décrit la fusion d'une céfotaximase (SEQ ID2; (Bonnet, Dutour et al. 2001)) avec une IR-TEM (SEQ ID1; (Chaibi, Sirot et al. 1999)) par l'intermédiaire d'un linker flexible polyglycine (6 résidus glycine dans cet exemple) et donnant naissance à une protéine hybride (SEQ ID9) capable d'hydrolyser l'amoxicilline même en présence d'acide clavulanique ainsi que la céftriaxone.

Ce mode de réalisation est identique à celui décrit dans l'exemple 1 à l'exception des amorces de PCR utilisées pour l'amplification des séquences CTXM16 et TEM-36.

CTXM-16 a été amplifiée en utilisant le couple d'amorce CTXM16-F et CTXM16-6G-R avec un TM de 58°C. TEM-36 a été amplifiée en utilisant le couple d'amorce 6G-TEM36-F et TEM36-R avec un TM de 58°C. Les séquences constitutives du linker GGGGGG sont indiquées en gras souligné dans la table 4.

**Table 4. Liste des amorces utilisées pour construire et séquencer la protéine hybride CTX-M16/TEM-36 à linker flexible.**

| Nom amorce | SEQ ID N° | Séquence5'-3' | Hôte |
|---|---|---|---|
| CTXM16-F | 27 | GAGGGTGTCTCTCTCGAG | *Pichia Pastoris* |
| CTXM16-6G-R | 28 | **TCCTCCTCCTCCTCCTCC**CAAACCTTCAGCTATGATCCG | *Pichia Pastoris* |
| TEM36-6G-F | 29 | **GGAGGAGGAGGAGGAGGA**CACCCTGAGACACTTGTCAAG | *Pichia Pastoris* |
| TEM36-R | 30 | TTGAGCGGCCGCCCCTCA | *Pichia Pastoris* |

Les deux fragments PCR ainsi obtenus ont été hybridés à un TM de 55°C et complémentés pendant 5 cycles de PCR sans amorces (95°C 30 sec - 55°C 45 sec - 72°C 2min15) en utilisant une ADN polymérase de haute-fidélité (Pfu, Promega). La séquence totale codant pour la protéine hybride a été réamplifiée après rajout des amorces externes (CTXM16-F + TEM-36-R).

Les conditions utilisées pour l'expression et la purification de la protéine hybride CTXM16-GGGGGG-TEM36 ont été strictement identiques à celles décrites dans l'exemple 1. La protéine hybride ainsi obtenue est également glycosylée comme le montre la figure 6 et combine une activité pénicillinase persistante en présence d'acide clavulanique avec une activité céfotaximase.

Comme dans le cas de l'exemple 1, aucune protéine naturelle ne présente les activités spécifiques décrites dans la table 5 sur les deux substrats amoxicilline/acide clavulanique et ceftriaxone.

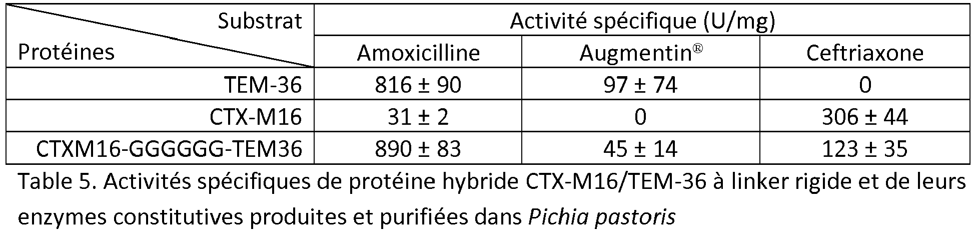

### Exemple 3 : molécule protéique hybride constituée de la fusion de TEM-36 et de CTX-M16 par un linker rigide

Le troisième mode de réalisation de la présente invention décrit la fusion d'une IR-TEM (SEQ ID1; (Chaibi, Sirot et al. 1999)) avec une cefotaximase (SEQ ID2; (Bonnet, Dutour et al. 2001)) par l'intermédiaire d'un linker protéique rigide (motif G(EAAAK)₂ dans cet exemple) et donnant naissance à une protéine hybride (SEQ ID10) capable d'hydrolyser l'amoxicilline même en présence d'acide clavulanique ainsi que la ceftriaxone.

Ce mode de réalisation est identique à celui décrit dans l'exemple 1 à l'exception des amorces de PCR utilisées pour l'amplification des séquences CTXM16 et TEM-36. TEM-36 a été amplifiée en utilisant le couple d'amorce TEM36-F et TEM36-G(EAAAK)₂-R avec un TM de 58°C. CTXM16 a été amplifiée en utilisant le couple d'amorce CTXM16-G(EAAAK)₂-F et CTXM16-R avec un TM de 58°C. Les séquences constitutives du linker G(EAAAK)₂ sont indiquées en gras souligné dans la table 6.

**Table 6. Liste des amorces utilisées pour construire et séquencer la protéine hybride CTX-M16 et TEM-36 à linker rigide.**

| Nom amorce | SEQ ID N° | Séquence5'-3' | Hôte |
|---|---|---|---|
| TEM36-F | 23 | GAGGGTGTCTCTCTCGAG | *Pichia Pastoris* |
| CTXM16-R | 26 | GCTAGGCGGCCGCTTTTACAAACCTTCAGC | *Pichia Pastoris* |
| TEM36-G(EAAAK)₂-R | 31 | **TGCTGCCTCTTTAGCGGC**CGCCTCTCCCCAATGTTTGATTAGGGA | *Pichia Pastoris* |
| CTXM16-G(EAAAK)₂-F | 32 | **GCCGCTAAAGAGGCAGCA**GCAAAACAGACGTCAGCCGTG | *Pichia Pastoris* |

Les 2 fragments PCR ainsi obtenus ont été hybridés à un TM de 55°C et complémentés pendant 5 cycles de PCR sans amorces (95°C 30 sec-55°C 45 sec-72°C 2min15) en utilisant une ADN polymérase de haute-fidélité (Pfu, Promega). La séquence totale codant pour la protéine hybride a été réamplifiée après rajout des amorces externes (TEM36-F + CTXM16-R).

Les conditions utilisées pour l'expression et la purification de la protéine hybride TEM36-G(EAAAK)₂-TEM36 ont été strictement identiques à celles décrites dans l'exemple 1. La protéine hybride ainsi obtenue est également glycosylée (Figure 6) et combine une activité pénicillinase persistante en présence d'acide clavulanique avec une activité céfotaximase.

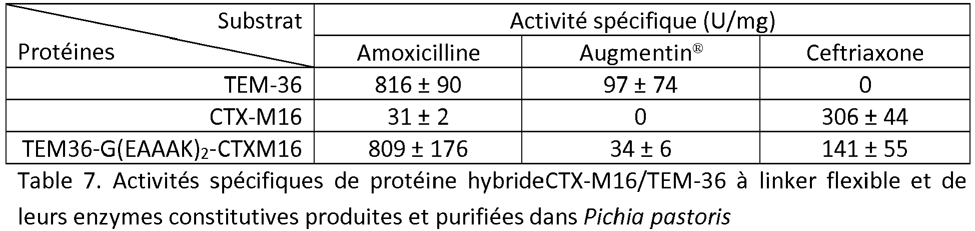

Comme dans les exemples 1 et 2, aucune protéine naturelle ne présente les activités spécifiques décrites dans la table 7 sur les deux substrats amoxicilline/acide clavulanique et ceftriaxone.

### Exemple 4 : molécules protéiques hybrides non-glycosylées constituées de la fusion de TEM-36 et de CTX-M16 (et inversement) par un linker flexible

Les séquences codantes pour TEM-36 (SEQ ID1) et CTXM16 (SEQ ID2) ont été obtenues commercialement par synthèse génique (*https:*//*www.dna20.com*/*services*/*gene-synthesis?gclid=CPnQIp3c9r0CFaoewwodnREAIg*) dans un vecteur d'expression périplasmique pour *E. coli.* Les séquences nucléotidiques ont été obtenues en phase avec le cadre de lecture codant pour un peptide d'adressage périplasmique (MSIQHFRVALIPFFAAFCLPVFA) avec un site de restriction Ndel au niveau de la méthionine initiatrice et un site de restriction Hindlll après le codon stop. Les fragments gèniques Ndel/Hindlll ont ensuite été sous-clonés dans le vecteur pET-26b(+) (Invitrogen).

Comme indiqué dans la figure 3, nous avons réalisé deux molécules protéiques hybrides par PCR de chevauchement . La séquence nucléotidique de TEM-36 et de CTX-M16 ont été amplifiées par PCR (95°C 30 sec - 25 cycles [95°C 30 sec - TM°C45sec - 72°C 1min] - 72°C 5 min -4°C) en utilisant une ADN polymerase de haute-fidélité (Pfu, Promega) et des amorces partiellement complémentaires et constitutives du linker protéique (GGGGGG dans cet exemple). TEM-36 a été amplifiée avec un TM de 55°C en utilisant soit le couple d'amorce T7-F et TEM36-G6-R-co soit le couple d'amorces TEM36-G6-F-co et T7-R. CTXM16 a été amplifiée avec un TM de 55°C en utilisant le couple d'amorce CTXM16-G6-F-co et T7-R soit le couple d'amorce CTXM16-G6-R-co et T7-F. Les séquences constitutives du linker GGGGGG sont indiquées en gras souligné dans la table 8.

**Table 8. Liste des amorces utilisées pour construire et séquencer la protéine hybride TEM-36/CTX-M16 à linker flexible.**

| Nom amorce | SEQ ID N° | Séquence5'-3' | Hôte |
|---|---|---|---|
| T7-F | 33 | TAATACGACTCACTATAGGGGAAT | *E. coli* |
| TEM36_ G6_R_co | 34 | **TCCTCCTCCTCCTCCTCC**CCAATGTTTAATCAGGCT | *E. coli* |
| CTXM16_G6_F_co | 35 | **GGAGGAGGAGGAGGA**CAGACGTCAGCCGTGCAGCAAAAG | *E. coli* |
| CTXM16_G6_ R_ co | 36 | **TCCTCCTCCTCCTCCTCC**CAAACCTTCAGCTATGATCCG | *E. coli* |
| TEM36-6G-F | 37 | **GGAGGAGGAGGAGGAGGA**CACCCTGAGACACTTGTCAAG | *E. coli* |
| T7-R | 38 | CTAGTTATTGCTCAGCGGTGG | *E. coli* |

Les fragments PCR ainsi obtenus (TEM36-G6 + G6-CTXM16 et CTXM16-G6 + G6-TEM36) ont été hybridés à un TM de 53°C et complémentés pendant 5 cycles de PCR sans amorces (95°C 30 sec - 53°C 45 sec - 72°C 2min15) en utilisant une ADN polymérase de haute-fidélité (Pfu, Promega). La séquence totale codant pour la protéine hybride a été réamplifiée après rajout des amorces externes (T7-F + T7-R) fonctionnant à un TM de 55°C.

Les fusions TEM36-GGGGGG-CTXM16 et CTX-M16-GGGGGG-TEM36 ainsi réalisées ont été clonées avec les enzymes de restrictions Ndel et Hindlll dans le vecteur pET26b+ (expression dans *E. coli*) (Figure 7).

Les séquences des constructions hybrides ont été vérifiées dans les 2 sens et ont révélé que le linker des 2 molécules protéiques hybrides était en fait constitué de seulement 5 glycines. Un réarrangement nucléique plus favorable a probablement eu lieu au moment des PCR d'hybridations. La figure 8 récapitule les fragments PCR obtenus avec les amorces T7-F & T7-R.

Les vecteurs d'expressions pET-26b(+) exprimant TEM-36 (SEQ ID1), CTX-M16 (SEQ ID2) et les fusions TEM36-G₅-CTXM16 et CTXM16-G₅-TEM36 ont été amplifiés dans *Escherichia coli* DH10B en maintenant une pression de sélection Kanamycine (Euromedex) à 50 µg/ml sur milieu LB (Extrait de Levure 5 g/l ; Tryptone 10 g/l ; NaCl10 g/l pH=7). La souche d'expression BL21(DE3) pLysS a été transformée avec les vecteurs d'expression par choc thermique. Les cellules transformées sont étalées sur milieu LB-agar (Extrait de levure 5 g/l; Tryptone10 g/l; NaCl10 g/L; agar 15g/lpH=7) contenant 50 µg/ml de Kanamycine.

L'expression dans *E. coli* à partir du vecteur pET-26b(+) aboutit à un adressage des protéines d'intérêt dans le compartiment périplasmique des bactéries où elles sont fonctionnelles. Les fusions créées telles que décrites dans l'exemple 4 ont été évaluées de 2 façons complémentaires, i) en caractérisant biochimiquement les protéines partiellement purifiées et ii) en comparant les concentrations minimales inhibitrices (CMI) de différents b-lactams (amoxicilline, Augmentin et Rocéphine) sur les souches d'expressions.

### Purification des molécules protéiques hybrides et de leur constituant dans E. coli :

Les bactéries transformées sont mises en culture dans 100 ml de LB + 50 µg/ml de Kanamycine à 37°C et sous 200 rpm d'agitation jusqu'à saturation. Les pré-cultures sont alors inoculées au 1/40^{ème} dans 1 L de milieu LB + Kanamycine 50 µg/ml. Lorsque la DO600 nm a atteint une valeur de 0.6 (environ 2 heures), la production de protéine est induite par ajout d'IPTG 0.5 mM final et se poursuit pendant 16 heures à 20°C sous une agitation de 200 rpm. Les cellules sont centrifugées à 5,000 xg pendant 15 minutes à 4°C et le culot immédiatement repris dans un tampon d'osmolyse pour casser la membrane externe et récupérer le périplasme. Les cellules sont reprises avec 1 ml de tampon (Phosphate 100 mM, Sucrose 500 mM, EDTA 1 mM pH = 7.0) pour 120 Unités de DO600 nm et incubées quelques minutes avec homogénéisation au vortex (protocole adapté de (Schlegel, Rujas et al. 2013)). Après centrifugation à 12,000 xg pendant 20 minutes à 4°C, le surnageant contenant les protéines périplasmiques est concentré sur Amicon Ultra (15 ml, 10 kDa, Millipore) jusqu'à un volume inférieur à 10 ml. La totalité des protéines est injectée sur une colonne de chromatographie d'exclusion (Superdex G75, GE Healthcare) et les protéines sont éluées en tampon phosphate (Phosphate 10 mM, NaCl 100 mM pH = 7.0) par fraction de 1 ml et avec un débit de 1 ml/min. Les fractions présentant la plus forte activité sur nitrocéfine (VWR) et la meilleure pureté (SDS-PAGE) sont combinées et concentrées à environ 0.1-0.5 mg/ml. La teneur protéique des échantillons est mesurée par absorbance à 280 nm et vérification sur gel SDS-PAGE. La figure 9 présente les protéines partiellement purifiées telles qu'obtenues avant caractérisation biochimiques.

Les activités enzymatiques des protéines purifiées ont été mesurées sur différentes bêta-lactamines (amoxicilline (Apollo Scientific), Augmentin® (GlaxoSmithKline) et Ceftriaxone (Rocéphine®, Roche)) tel que décrit dans les exemples précédents et les résultats sont compilés dans la table 9 ci-dessous.

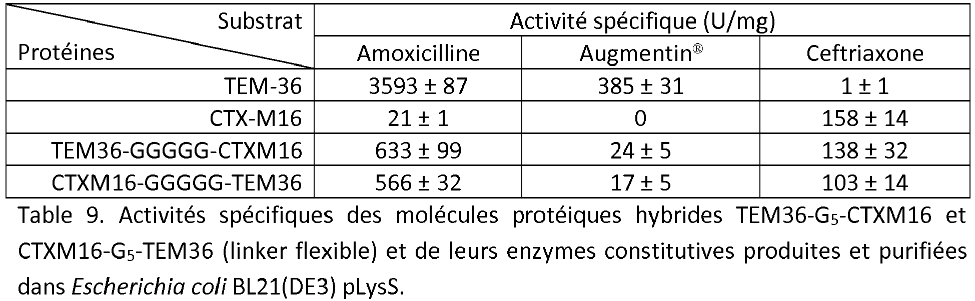

Comme dans les exemples 1 à 3, aucune protéine naturelle ne présente les activités spécifiques décrites dans le tableau 9 sur les deux substrats amoxicilline et ceftriaxone.

### Résistance des souches d'E. coli exprimant les molécules protéiques hybrides aux divers β-lactames:

Les bactéries transformées (exprimant TEM36, CTXM16 ou les fusions TEM36-G5-CTXM16 et CTXM16-G5-TEM36) ou la souche vide (BL21(DE3)pLysS) sont mises en culture dans 5 ml de LB + 50 µg/ml de Kanamycine lorsque nécessaire à 37°C et sous 200 rpm d'agitation jusqu'à saturation. Les pré-cultures sont alors inoculées au 1/40^{ème} dans 5 ml de milieu LB + Kanamycine 50 µg/ml et lorsque la DO600 nm a atteint une valeur de 0.6 (environ 2 heures), la production de protéine est induite par ajout d'IPTG 1 mM final et se poursuit pendant 1.5 heure à 37°C sous une agitation de 200 rpm.

Les cellules sont normalisées à 10⁸ cfu/ml et diluées en cascade à 10⁷, 10⁶, 10⁵, 10⁴ cfu/ml et 5 µl de chaque suspension sont déposés sur une boite LB-Agar contenant des concentrations croissantes d'antibiotiques (Amoxicilline 0, 2, 4, 8, 16, 32, 64, 128, 256, 512, 1024 µg/ml ; Augmentin 0, 1, 2, 4, 8, 16, 32, 64, 128, 256, 512 µg/ml et Rocéphine 0, 0.5, 1, 2, 4, 8, 16, 32, 64, 128, 256, 512 µg/ml).

Les boites sont incubées à 37°C sur la nuit et les résultats sont enregistrés le lendemain. Les CMls correspondent à la concentration d'antibiotique la plus faible à laquelle le plus fort inoculum ne pousse plus.

Les données sont répertoriées dans la table 10 ci-dessous.

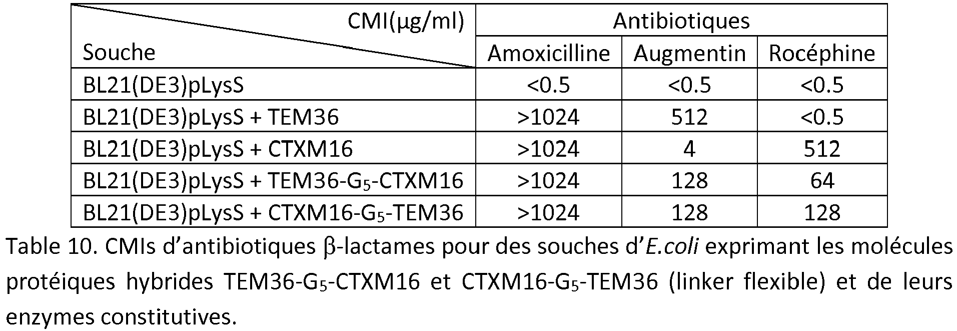

Les cellules d*'E.coli* exprimant les molécules protéiques hybrides telles que décrites dans l'exemple 4 présente un phénotype de résistance multiple aux aminopénicillines (avec ou sans inhibiteurs comme l'acide clavulanique) et aux céphalosporines de 3^{ème} génération comme la ceftriaxone. Aucune souche bactérienne naturelle n'a pour l'instant été décrite dans la littérature avec un tel phénotype.

### Exemple 5 : molécule protéique hybride constituée de la fusion de CTX-M16 et de AAC-6'-Ib-cr par un linker de type poly-histidine

NB : Cet exemple ne fait pas partie de la présente invention.

Les séquences nucléotidiques codant pour les protéines CTX-M16 (SEQ ID2) et AAC-6'-Ib-cr (ci-après abrégée AAC) (SEQ ID4) ont été obtenues commercialement par synthèse de gène dans un vecteur d'expression pour *E. coli, respectivement pJexpress411 (KanR) et pJ404(AmpR).* La séquence nucléotidique de CTX-M16 insérée dans le vecteur pJexpress411 correspond à la SEQ ID 16 et la séquence nucléotidique de AAC insérée dans le vecteur pJ404 correspond à la SEQ ID 12. Ces séquences ont alors été amplifiées par PCR (95°C 30 sec - 25 cycles [95°C 30 sec - TM°C45sec - 72°C 1min] - 72°C 5 min - 4°C) en utilisant une ADN polymérase de haute-fidélité (Pfu, Promega) et des amorces partiellement complémentaires et constitutives du linker protéique (HHHHHH dans cet exemple) comme indiqué dans la Table 11. CTX-M16 a été amplifiée en utilisant le couple d'amorce 6H-CTX-F et T7R avec un TM de 62°C. ACC a été amplifiée en utilisant le couple d'amorce AAC-F_coli et AAC-6H-R avec un TM de 50°C. Les séquences constitutives du linker HHHHHH sont indiquées en gras souligné dans la table 11.

**Table 11. Liste des amorces utilisées pour construire et séquencer la protéine hybride AAC-H6-CTXM16 à linker polyhistidine.**

| Nom amorce | SEQ ID N° | Séquence5'-3' | Hôte |
|---|---|---|---|
| AAC-F_coli | 39 | GAAGGAGATATACATATGAGCAACGCT | *E. coli* |
| AAC-6H-R | 40 | GTGGTGATGATGGTGGTGCGC | *E. coli* |
| H6-CTX-F | 41 | CACCATCATCACCACCAGACGTCAGCCGTGCAGCAAAAG | *E. coli* |
| T7-R | 38 | CTAGTTATTGCTCAGCGGTGG | *E. coli* |

Les deux fragments PCR ainsi obtenus ont été hybridés à un TM de 62°C et complémentés pendant 5 cycles de PCR sans amorces (95°C 30sec - 62°C 45 sec- 72°C 2min15) en utilisant une ADN polymérase de haute-fidélité (Pfu, Promega). La séquence totale codant pour la protéine hybride a été réamplifiée après rajout des amorces externes (AAC-F_coli + T7R). Ce mode de réalisation des fusions par PCR est schématiquement représenté dans la Figure 3. Les inserts codant pour AAC, CTXM16 et la fusion AAC-6H-CTXM16 ont été chargés sur gel d'agarose 1% pour illustrer leur taille respective (Figure 10).

La fusion AAC-6H-CTXM16 ainsi réalisée a été clonée avec les enzymes de restrictions Ndel et HinDlll dans le vecteur pET26b+ (*expression E. coli*) selon le principe illustré par la Figure 7.

La séquence de la construction hybride a été vérifiée dans les 2 sens et correspond à la fusion décrite.

Les vecteurs d'expressions pJ404-AAC exprimant AAC et pET-AAC-6H-CTXM16 exprimant la fusion AAC-H6-CTXM16 ont été transformés dans *Escherichia coli* BL21(DE3)pLysS en maintenant respectivement une pression de sélection Ampicilline (Euromedex) à 100 µg/ml et Kanamycine (Euromedex) à 50 µg/ml sur milieu LB (Extrait de Levure 5 g/l ; Tryptone 10 g/l; NaCl 10 g/l pH=7.5). L'expression de AAC ainsi que la fusion AAC-6H-CTX dans *E. coli* à partir du vecteur pJ404 aboutit à des corps d'inclusions pour les protéines d'intérêt.

Pour chaque protéine, 1L de LB est ensemencé au 1/40^{ème} à partir d'une pré-culture saturée puis cultivée à 37°C 200 rpm d'agitation jusqu'à une DO(600nm) d'environ 0,4-0,6. Les cultures sont induites 4 heures à 37°C et 200 rpm par l'ajout d'IPTG 0.5 mM final. En fin de production, les cellules sont centrifugées et le culot est repris à 40 ml/L de culture dans du tampon de lyse (10 mM Tris-HCl, 150 mM NaCl, 1 mM EDTA, 0.1% Triton X100 pH=8.0 et 0.25 mg/ml lysozyme) puis congelé à -80°C. Les cellules sont décongelées et lysées pendant 45 minutes à température ambiante en présence de MgSO₄ (20 mM) et DNAse (10 µg/ml). Le lysat est centrifugé (30 min à 12,000 xg à 4°C) et le culot contenant les corps d'inclusion des protéines d'intérêt (AAC et AAC-6H-CTXM16) est repris dans un tampon A (10 mM Phosphate, 150 mM NaCl, 10 mM Imidazole, 8 M Urée pH=8.0). Les protéines sont purifiées par chromatographie d'affinité Nickel et éluées avec un gradient de tampon B (10 mM Phosphate, 150 mM NaCl, 500 mM imidazole, 8 M Urée pH=8.0).

Les fractions contenant les protéines d'intérêt sont mélangées, incubées 1 heure à 4°C en présence de 1mM DTT, puis renaturées par 3 dialyses successives en tampon 10 mM Phosphate, 150 mM NaCl pH=8. Les protéines sont clarifiées par centrifugation puis concentrées jusqu'à un volume inférieur à 10 ml par ultrafiltration sur membrane de 10 kDa (Centricon, Millipore). Les protéines sont injectées sur une colonne de chromatographie d'exclusion (Superdex G200 ; colonnes 26^{∗}60 GE Healthcare), et éluées en tampon 10 mM phosphate, 150 mM NaCl pH = 8.0 par fraction de 1 ml et avec un débit de 1 ml/min.

Les fractions présentant la plus forte activité sur nitrocéfine (VWR) et la meilleure pureté (SDS-PAGE) sont combinées et concentrées à environ 0.5 mg/ml. La teneur protéique des échantillons est mesurée par BCA (Pierce), absorbance à 280 nm et vérification sur gel SDS-PAGE. La figure 11 présente les résultats protéines purifiées obtenues.

Les protéines purifiées ont été testées sur différents antibiotiques : Ceftriaxone (Rocéphine®, Roche) pour les beta-lactamines et kanamycine pour les Aminoglycosides. Les dosages sur ceftriaxone sont effectués au pH-STAT (Titrino 2.5, Metrohm) dans un volume réactionnel de 25 ml à 37°C et pH = 7.0. Les substrats sont préparés à 4 g/l dans un tampon Tris 0.3 mM, NaCl 150 mM. L'hydrolyse enzymatique des noyaux bêta-lactames libère un acide et entraine une baisse du pH. Le principe du dosage consiste à compenser cette acidification par ajout de soude 0,1 N de manière à rester à pH = 7.0. Dans ces conditions, une unité correspond à 1 µmole de soude ajoutée par minute, c'est-à-dire une µmole de bêta-lactame hydrolysée par minute.

La mesure d'activité acétyl-transferase se fait par un dosage colorimétrique indirect avec de l'acetyl-CoA (Sigma-Aldrich) comme donneur de groupement acetyl, de la Kanamycine comme accepteur et du réactif d'Elleman (DTNB, Sigma-aldrich) pour titrer les molécules de CoEnzymeA réduites (-SH) libérées au cours de la réaction enzymatique [Ref]. La réaction se fait en plaque de microtitration avec des quantités croissantes d'enzymes purifiées, dans un volume final de 200 µl et avec les concentrations de 500 µM Kanamycine, 500 µM AcetylCoA et 250 µM DTNB. Dans ces conditions expérimentales, l'ion TNB²⁻ absorbe à 412 nm avec un ε_{(λ=412nm)} apparent de 19 000 M⁻¹.puits⁻¹ (puits d'une plaque 96 puits rempli avec 200 µl) et 1 unité correspond à une nanomole de TNB²⁻ libérée par minute à 37°C.

La table 12 ci-dessous récapitule les activités spécifiques mesurées pour chaque protéine sur les trois substrats (moyenne de 6 expériences soit n=6).

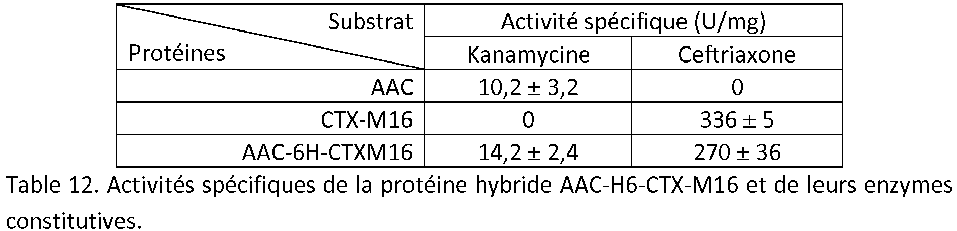

Ces résultats montrent que la fusion entre AAC-6'-Ib-cr et CTX-M16 génère une protéine hybride capable d'hydrolyser une céphalosporine de troisième génération et d'inactiver un aminoglycoside par acétylation. Il n'existe aucune enzyme naturelle connue susceptible d'inactiver un antibiotique de ces deux classes.

### Exemple 6 : molécules protéiques hybrides non-glycosylées constituées de la fusion de EreB et de TEM36 par un linker de type tag polyhistidine.

NB : Cet exemple ne fait pas partie de la présente invention.

Les séquences codantes pour TEM-36 (SEQ ID1) et EreB (SEQ ID5) ont été obtenues commercialement par synthèse génique dans un vecteur d'expression pour *E. coli.* Les fragments géniques Ndel/Hindlll ont ensuite été sous-clonés dans le vecteur pET-26b(+) (Invitrogen).

Selon le principe décrit dans la figure 3, nous avons réalisé une molécule protéique hybride EreB-H6-TEM36 par PCR de chevauchement. La séquence nucléotidique de TEM-36 et de EreB ont été amplifiées par PCR (95°C 30 sec - 25 cycles [95°C 30 sec - TM°C45sec - 72°C 1min] - 72°C 5 min - 4°C) en utilisant une ADN polymérase de haute-fidélité (Pfu, Promega) et des amorces partiellement complémentaires et constitutives du linker protéique (tag 6 histidine dans cet exemple). TEM-36 a été amplifiée avec un TM de 65°C en utilisant le couple d'amorces EreB6HTEM36_coli_F et T7_R. EreB a été amplifiée avec un TM de 65°C en utilisant le couple d'amorces EreB_coli_F et EreB6HTEM36_R. Les séquences constitutives du linker HHHHHH sont indiquées en gras souligné dans la table 13.

**Table 13. Liste des amorces utilisées pour construire et séquencer la protéine hybride EreB-H6-TEM-36.**

| Nom amorce | SEQ ID N° | Séquence 5'-3' | Hôte |
|---|---|---|---|
| EreB_coli_F | 42 | GATATA*CATATG*CGTTTTGAAGAGTGG | *E. coli* |
| EreB6HTEM36_R | 43 | **GTGATGGTGATGGTGGTG**CTCATAAAC | *E. coli* |
| EreB6HTEM36_coli_F | 44 | **CACCACCATCACCATCAC**CACCCGGAAACCCTGGTGAAAGTT | *E. coli* |
| T7-R | 38 | CTAGTTATTGCTCAGCGGTGG | *E. coli* |

Les fragments PCR ainsi obtenus ont été hybridés à un TM de 55°C et complémentés pendant 5 cycles de PCR sans amorces (95°C 30 sec-55°C 45 sec-72°C 2min15) en utilisant une ADN polymérase de haute-fidélité (Pfu, Promega). La séquence totale codant pour la protéine hybride a été réamplifiée après rajout des amorces externes (EreB_coli_F + T7-R) fonctionnant à un TM de 55°C.

La fusion EreB-H6-TEM36 ainsi réalisée a été clonée avec les enzymes de restrictions Ndel et Hindlll dans le vecteur pET26b+ (expression dans *E. coli*) (Figure 7).

La séquence de la construction hybride a été vérifiée dans les 2 sens. La figure 12 récapitule les fragments PCR obtenus avec les amorces T7-F & T7-R sur la série pET-TEM36, EreB et EreB-H6-TEM36.

Les vecteurs d'expressions pET-26b(+) exprimant TEM-36 (SEQ ID1), EreB (SEQ ID2) et la fusion EreB-H6-TEM36 ont été amplifiés dans *Escherichia coli* DH10B en maintenant une pression de sélection Kanamycine (Euromedex) à 50 µg/ml sur milieu LB (Extrait de Levure 5 g/l ; Tryptone 10 g/l ; NaCl 10 g/l pH=7). La souche d'expression BL21(DE3) pLysS a été transformée avec les vecteurs d'expression par choc thermique. Les cellules transformées sont étalées sur milieu LB-agar (Extrait de levure 5 g/l ; Tryptonel10 g/l ; NaCl10 g/L; agar 15g/lpH=7) contenant 50 µg/ml de Kanamycine.

La fonctionnalité des protéines d'intérêt (TEM36, EreB et la fusion EreB-H6-TEM36) dans *E*. *coli* a été évaluée en mesurant la résistance des souches d'expression à différents antibiotiques de type b-lactams (amoxicilline et augmentin) et macrolides (erythromycine).

Les bactéries transformées (exprimant TEM36, EreB ou EreB-H6-TEM36) ou la souche vide (BL21(DE3)pLysS) sont mises en culture dans 5 ml de LB + 50 µg/ml de Kanamycine lorsque nécessaire à 37°C et sous 200 rpm d'agitation jusqu'à saturation. Les pré-cultures sont alors inoculées au 1/40^{ème} dans 5 ml de milieu LB + Kanamycine 50 µg/ml et lorsque la DO600 nm a atteint une valeur de 0.6 (environ 2 heures), la production de protéine est induite par ajout d'IPTG 1 mM final et se poursuit pendant 1.5 heure à 37°C sous une agitation de 200 rpm.

Les cellules sont normalisées à 10⁸ cfu/ml et diluées en cascade à 10⁷, 10⁶, 10⁵, 10⁴ cfu/ml et 5 µl de chaque suspension sont déposés sur une boite LB-Agar contenant des concentrations croissantes d'antibiotiques (Amoxicilline 0, 2, 4, 8, 16, 32, 64, 128, 256, 512, 1024, 2048 µg/ml ; Augmentin 0, 2, 4, 8, 16, 32, 64, 128, 256, 512, 1024 µg/ml et Erythromycine 0, 2, 4, 8, 16, 32, 64, 128, 256, 512, 1024 µg/ml).

Les boites sont incubées à 37°C sur la nuit et les résultats sont enregistrés le lendemain. Les CMls correspondent à la concentration d'antibiotique la plus faible à laquelle le plus fort inoculum ne pousse plus.

Les données sont répertoriées dans la table 14 ci-dessous.

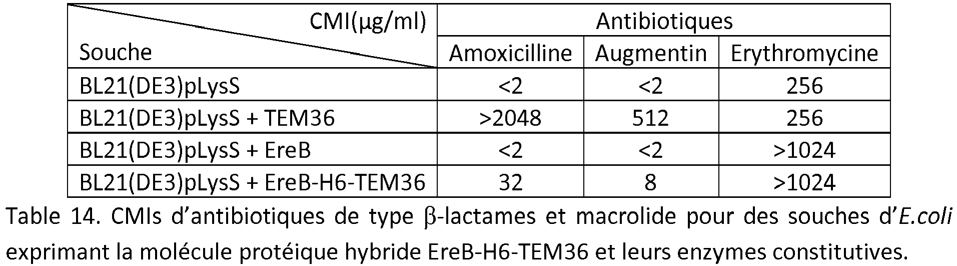

Les cellules d'*E.coli* exprimant la molécule protéique hybride telles que décrite dans l'exemple 6 présente un phénotype de résistance multiple aux aminopénicillines (avec ou sans inhibiteurs comme l'acide clavulanique) et aux macrolides comme l'erythromycine. Aucune souche bactérienne naturelle n'a pour l'instant été décrite dans la littérature avec un tel phénotype résultant de l'expression d'une seule et même protéine.

### Références

Aires, J. R., T. Kohler, H. Nikaido and P. Plesiat (1999). "Involvement of an active efflux system in the natural résistance of Pseudomonas aeruginosa to aminoglycosides." Antimicrob Agents Chemother43(11): 2624-2628.
Ambler, R. P. (1975). "The amino acid sequence of Staphylococcus aureus penicillinase." Biochem J151(2): 197-218.
Ambler, R. P. (1980). "The structure of beta-lactamases." Philos Trans R Soc Lond B Biol Sci289(1036): 321-331.
Anand, A., B. Bashey, T. Mir and A. E. Glatt (1994). "Epidemiology, clinical manifestations, and outcome of Clostridium difficile-associated diarrhea." Am J Gastroenterol89(4): 519-523. Arthur, M., D. Autissier and P. Courvalin (1986). "Analysis of the nucleotide sequence of the ereB gene encoding the erythromycin esterase type II." Nucleic Acids Res14(12): 4987-4999. Bartlett, J. G. (1996). "Management of Clostridium difficile infection and other antibiotic-associated diarrhoeas." Eur J Gastroenterol Hepatol8(11): 1054-1061.
Bonnet, R. (2004). "Growing group of extended-spectrum beta-lactamases: the CTX-M enzymes." Antimicrob Agents Chemother48(1): 1-14.
Bonnet, R., C. Dutour, J. L. Sampaio, C. Chanal, D. Sirot, R. Labia, C. De Champs and J. Sirot (2001). "Novel cefotaximase (CTX-M-16) with increased catalytic efficiency due to substitution Asp-240-->Gly." Antimicrob Agents Chemother45(8): 2269-2275.
Bozdogan, B., L. Berrezouga, M. S. Kuo, D. A. Yurek, K. A. Farley, B. J. Stockman and R. Leclercq (1999). "A new résistance gene, linB, conferring résistance to lincosamides by nucleotidylation in Enterococcus faecium HM1025." Antimicrob Agents Chemother43(4): 925-929.
Bush, K. and G. Jacoby (1997). "Nomenclature of TEM beta-lactamases." J Antimicrob Chemother39(1): 1-3.
Chaibi, E. B., D. Sirot, G. Paul and R. Labia (1999). "Inhibitor-resistant TEM beta-lactamases: phenotypic, genetic and biochemical characteristics." J Antimicrob Chemother43(4): 447-458.
Chen, F., S. Nielsen and R. Zenobi (2013). "Understanding chemical reactivity for homo- and heterobifunctional protein cross-linking agents." J Mass Spectrom48(7): 807-812.
Chen, X., J. L. Zaro and W. C. Shen (2013). "Fusion protein linkers: property, design and functionality." Adv Drug Deliv Rev65(10): 1357-1369.
Chopra, I. and M. Roberts (2001). "Tetracycline antibiotics: mode of action, applications, molecular biology, and epidemiology of bacterial resistance." Microbiol Mol Biol Rev65(2): 232-260 ; second page, table of contents.
Committee on Drug Use in Food Animais (1999). The use of drugs in food animals, benefits and risks. Washington, D.C., National Academy Press.
Deshpande, A., V. Pasupuleti, P. Thota, C. Pant, D. D. Rolston, T. J. Sferra, A. V. Hernandez and C. J. Donskey (2013). "Community-associated Clostridium difficile infection and antibiotics: a meta-analysis." J Antimicrob Chemother68(9): 1951-1961.
Doi, Y. and Y. Arakawa (2007). "16S ribosomal RNA methylation: emerging résistance mechanism against aminoglycosides." Clin Infect Dis45(1): 88-94.
Drawz, S. M. and R. A. Bonomo (2010). "Three decades of beta-lactamase inhibitors." Clin Microbiol Rev23(1): 160-201.
ECDC/EMEA Joint Working Group (2009). The bacterial challenge: time to react". Estimates based on bacteria most frequently isolated from blood cultures in Europe. E. E. J. T. Report.
Fancy, D. A. and T. Kodadek (1999). "Chemistry for the analysis of protein-protein interactions: rapid and efficient cross-linking triggered by long wavelength light." Proc Natl Acad Sci U S A96(11): 6020-6024.
Georgetown University Center for Food and Nutrition Policy (1999). Antibiotic use in animais: food safety and risk assessment, Washington, D.C.
Gerding, D. N. (1989). "Disease associated with Clostridium difficile infection." Ann Intern Med110(4): 255-257.
Gilbert, D. N. (1994). "Aspects of the Safety Profile of Oral Antibacterial Agents." Infectious Diseases in Clinical Practice3(4): 236-245.
Gorkiewicz, G. (2009). "Nosocomial and antibiotic-associated diarrhoea caused by organisms other than Clostridium difficile." Int J Antimicrob Agents33 Suppl 1: S37-41.
Hancock, R. E. (1981). "Aminoglycoside uptake and mode of action--with special reference to streptomycin and gentamicin. I. Antagonists and mutants." J Antimicrob Chemother8(4): 249-276.
Harmoinen, J., S. Mentula, M. Heikkila, M. van der Rest, P. J. Rajala-Schultz, C. J. Donskey, R. Frias, P. Koski, N. Wickstrand, H. Jousimies-Somer, E. Westermarck and K. Lindevall (2004). "Orally administered targeted recombinant Beta-lactamase prevents ampicillin-induced selective pressure on the gut microbiota: a novel approach to reducing antimicrobial resistance." Antimicrob Agents Chemother48(1): 75-79.
Harmoinen, J., K. Vaali, P. Koski, K. Syrjanen, O. Laitinen, K. Lindevall and E. Westermarck (2003). "Enzymatic degradation of a beta-lactam antibiotic, ampicillin, in the gut: a novel treatment modality." J Antimicrob Chemother51(2): 361-365.
Henquell, C., C. Chanal, D. Sirot, R. Labia and J. Sirot (1995). "Molecular characterization of nine différent types of mutants among 107 inhibitor-resistant TEM beta-lactamases from clinical isolates of Escherichia coli." Antimicrob Agents Chemother39(2): 427-430.
Herzberg, O. and J. Moult (1987). "Bacterial résistance to beta-lactam antibiotics: crystal structure of beta-lactamase from Staphylococcus aureus PC1 at 2.5 A resolution." Science236(4802): 694-701.
Hogenauer, C., H. F. Hammer, G. J. Krejs and E. C. Reisinger (1998). "Mechanisms and management of antibiotic-associated diarrhea." Clin Infect Dis27(4): 702-710.
Institute of Medicine, D. o. H. P. a. D. P. (1998). Report of a study. Human health risks with the subtherapeutic use of penicillin or tetracyclines in animal feed. Washington, D.C., National Academy Press.
Kelly, C. P., C. Pothoulakis and J. T. LaMont (1994). "Clostridium difficile colitis." N Engl J Med330(4): 257-262.
Kemal, C. and J. R. Knowles (1981). "Penicillanic acid sulfone: interaction with RTEM beta-lactamase from Escherichia coli at différent pH values." Biochemistry20(13): 3688-3695.
Klevens, R. M., J. R. Edwards, C. L. Richards, Jr., T. C. Horan, R. P. Gaynes, D. A. Pollock and D. M. Cardo (2007). "Estimating health care-associated infections and deaths in U.S. hospitals, 2002." Public Health Rep122(2): 160-166.
Knott-Hunziker, V., S. G. Waley, B. S. Orlek and P. G. Sammes (1979). "Penicillinase active sites: labelling of serine-44 in beta-lactamase I by 6beta-bromopenicillanic acid." FEBS Lett99(1): 59-61.
Larkin, M. A., G. Blackshields, N. P. Brown, R. Chenna, P. A. McGettigan, H. McWilliam, F. Valentin, I. M. Wallace, A. Wilm, R. Lopez, J. D. Thompson, T. J. Gibson and D. G. Higgins (2007). "Clustal W and Clustal X version 2.0." Bioinformatics23(21): 2947-2948.
Leclercq, R., A. Brisson-Noel, J. Duval and P. Courvalin (1987). "Phenotypic expression and genetic heterogeneity of lincosamide inactivation in Staphylococcus spp." Antimicrob Agents Chemother31(12): 1887-1891.
Leroy, A., G. Humbert, G. Oksenhendler and J. P. Fillastre (1978). "Pharmacokinetics of aminoglycosides in subjects with normal and impaired renal function." Antibiot Chemother (1971)25: 163-180.
Lin, Y., J. M. Pagel, D. Axworthy, A. Pantelias, N. Hedin and O. W. Press (2006). "A genetically engineered anti-CD45 single-chain antibody-streptavidin fusion protein for pretargeted radioimmunotherapy of hematologic malignancies." Cancer Res66(7): 3884-3892.
Mentula, S., J. Harmoinen, P. Koski, E. Westermarck, M. Rautio, P. Huovinen and E. Kononen (2004). "Inhibition of ampicillin-induced emergence of résistance in intestinal coliforms by targeted recombinant beta-lactamase." Int J Antimicrob Agents24(6): 555-561.
Morar, M., K. Pengelly, K. Koteva and G. D. Wright (2012). "Mechanism and diversity of the erythromycin esterase family of enzymes." Biochemistry51(8): 1740-1751.
Morita, Y., K. Kodama, S. Shiota, T. Mine, A. Kataoka, T. Mizushima and T. Tsuchiya (1998). "NorM, a putative multidrug efflux protein, of Vibrio parahaemolyticus and its homolog in Escherichia coli." Antimicrob Agents Chemother42(7): 1778-1782.
Novick, R. P. (1963). "Analysis by Transduction of Mutations Affecting Penicillinase Formation in Staphylococcus Aureus." J Gen Microbiol33: 121-136.
Ounissi, H. and P. Courvalin (1985). "Nucleotide sequence of the gene ereA encoding the erythromycin esterase in Escherichia coli." Gene35(3): 271-278.
Pagel, J. M., Y. Lin, N. Hedin, A. Pantelias, D. Axworthy, D. Stone, D. K. Hamlin, D. S. Wilbur and O. W. Press (2006). "Comparison of a tetravalent single-chain antibody-streptavidin fusion protein and an antibody-streptavidin chemical conjugate for pretargeted anti-CD20 radioimmunotherapy of B-cell lymphomas." Blood108(1): 328-336.
Paguirigan, A. L. and D. J. Beebe (2007). "Protocol for the fabrication of enzymatically crosslinked gelatin microchannels for microfluidic cell culture." Nat Protoc2(7): 1782-1788. Poehlsgaard, J. and S. Douthwaite (2005). "The bacterial ribosome as a target for antibiotics." Nat Rev Microbiol3(11): 870-881.
Ramirez, M. S. and M. E. Tolmasky (2010). "Aminoglycoside modifying enzymes." Drug Resist Updat13(6): 151-171.
Richards, M. J., J. R. Edwards, D. H. Culver and R. P. Gaynes (2000). "Nosocomial infections in combined medical-surgical intensive care units in the United States." Infect Control Hosp Epidemiol21(8): 510-515.
Ripoll, A., F. Baquero, A. Novais, M. J. Rodriguez-Dominguez, M. C. Turrientes, R. Canton and J. C. Galan (2011). "In vitro selection of variants resistant to beta-lactams plus beta-lactamase inhibitors in CTX-M beta-lactamases: predicting the in vivo scénario?" Antimicrob Agents Chemother55(10): 4530-4536.
Roberts, M. C. (2008). "Update on macrolide-lincosamide-streptogramin, ketolide, and oxazolidinone résistance genes." FEMS Microbiol Lett282(2): 147-159.
Roberts, M. C., J. Sutcliffe, P. Courvalin, L. B. Jensen, J. Rood and H. Seppala (1999). "Nomenclature for macrolide and macrolide-lincosamide-streptogramin B résistance determinants." Antimicrob Agents Chemother43(12): 2823-2830.
Robicsek, A., G. A. Jacoby and D. C. Hooper (2006). "The worldwide emergence of plasmid-mediated quinolone resistance." Lancet Infect Dis6(10): 629-640.
Robicsek, A., J. Strahilevitz, G. A. Jacoby, M. Macielag, D. Abbanat, C. H. Park, K. Bush and D. C. Hooper (2006). "Fluoroquinolone-modifying enzyme: a new adaptation of a common aminoglycoside acetyltransferase." Nat Med12(1): 83-88.
Salverda, M. L., J. A. De Visser and M. Barlow (2010). "Natural evolution of TEM-1 beta-lactamase: experimental reconstruction and clinical relevance." FEMS Microbiol Rev34(6): 1015-1036.
Schlegel, S., E. Rujas, A. J. Ytterberg, R. A. Zubarev, J. Luirink and J. W. de Gier (2013). "Optimizing heterologous protein production in the periplasm of E. coli by regulating gene expression levels." Microb Cell Fact12: 24.
Schultz, J., Y. Lin, J. Sanderson, Y. Zuo, D. Stone, R. Mallett, S. Wilbert and D. Axworthy (2000). "A tetravalent single-chain antibody-streptavidin fusion protein for pretargeted lymphoma therapy." Cancer Res60(23): 6663-6669.
Shaw, K. J., P. N. Rather, R. S. Hare and G. H. Miller (1993). "Molecular genetics of aminoglycoside résistance genes and familial relationships of the aminoglycoside-modifying enzymes." Microbiol Rev57(1): 138-163.
Sherry, L., B. Murphy, J. Xu. and K. D. Kochanek (2012). Deaths: Preliminary Data for 2010. National Vital Statistics Reports. 60: 1-69.
Slimings, C. and T. V. Riley (2013). "Antibiotics and hospital-acquired Clostridium difficile infection: update of systematic review and meta-analysis." J Antimicrob Chemother.
Sparks, S. G., R. J. Carman, M. R. Sarker and B. A. McClane (2001). "Genotyping of enterotoxigenic Clostridium perfringens fecal isolates associated with antibiotic-associated diarrhea and food poisoning in North America." J Clin Microbiol39(3): 883-888.
Speer, B. S., L. Bedzyk and A. A. Salyers (1991). "Evidence that a novel tetracycline résistance gene found on two Bacteroides transposons encodes an NADP-requiring oxidoreductase." J Bacteriol173(1): 176-183.
Stiefel, U., N. J. Pultz, J. Harmoinen, P. Koski, K. Lindevall, M. S. Helfand and C. J. Donskey (2003). "Oral administration of beta-lactamase preserves colonization résistance of piperacillin-treated mice." J Infect Dis188(10): 1605-1609.
Tarkkanen, A. M., T. Heinonen, R. Jogi, S. Mentula, M. E. van der Rest, C. J. Donskey, T. Kemppainen, K. Gurbanov and C. E. Nord (2009). "P1A recombinant beta-lactamase prevents emergence of antimicrobial résistance in gut microflora of healthy subjects during intravenous administration of ampicillin." Antimicrob Agents Chemother53(6): 2455-2462.
Tenson, T., M. Lovmar and M. Ehrenberg (2003). "The mechanism of action of macrolides, lincosamides and streptogramin B reveals the nascent peptide exit path in the ribosome." J Mol Biol330(5): 1005-1014.
Thompson, J. D., D. G. Higgins and T. J. Gibson (1994). "CLUSTAL W: improving the sensitivity of progressive multiple sequence alignment through sequence weighting, position-specific gap penalties and weight matrix choice." Nucleic Acids Res22(22): 4673-4680.
Tu, D., G. Blaha, P. B. Moore and T. A. Steitz (2005). "Structures of MLSBK antibiotics bound to mutated large ribosomal subunits provide a structural explanation for resistance." Cell121(2): 257-270.
Turner, P. J. (2005). "Extended-Spectrum β-Lactamases." Clinical Infectious Diseases41(Supplement 4): S273-S275.
Vardakas, K. Z., A. A. Konstantelias, G. Loizidis, P. I. Rafailidis and M. E. Falagas (2012). "Risk factors for development of Clostridium difficile infection due to BI/NAP1/027 strain: a meta-analysis." Int J Infect Dis16(11): e768-773.
Walsh, C. (2000). "Molecular mechanisms that confer antibacterial drug resistance." Nature406(6797): 775-781.
Weisblum, B. (1995). "Erythromycin résistance by ribosome modification." Antimicrob Agents Chemother39(3): 577-585.
Wistrom, J., S. R. Norrby, E. B. Myhre, S. Eriksson, G. Granstrom, L. Lagergren, G. Englund, C. E. Nord and B. Svenungsson (2001). "Frequency of antibiotic-associated diarrhoea in 2462 antibiotic-treated hospitalized patients: a prospective study." J Antimicrob Chemother47(1): 43-50.
Zhou, X. Y., F. Bordon, D. Sirot, M. D. Kitzis and L. Gutmann (1994). "Emergence of clinical isolates of Escherichia coli producing TEM-1 derivatives or an OXA-1 beta-lactamase conferring résistance to beta-lactamase inhibitors." Antimicrob Agents Chemother38(5): 1085-1089.

## Revendications

1. Molécule protéique hybride comportant deux enzymes inhibant l'activité d'au moins un antibiotique, les deux enzymes ayant des caractéristiques biochimiques différentes et lesdites enzymes étant deux bêta-lactamases liées entre elles.

2. Molécule protéique hybride selon l'une quelconque des revendications précédentes **caractérisée en ce que** la séquence de l'une au moins des enzymes présente une homologie de séquence d'au moins 40% avec la SEQ ID1 à condition que l'enzyme conserve la même activité.

3. Molécule protéique hybride selon l'une quelconque des revendications précédentes **caractérisée en ce que** la séquence de l'une au moins des bêta-lactamases présente une homologie de séquence d'au moins 40% avec la SEQ ID2 à condition que l'enzyme conserve la même activité.

4. Molécule protéique hybride selon l'une quelconque des revendications précédentes **caractérisée en ce que** la séquence de l'une au moins des bêta-lactamases présente une homologie de séquence d'au moins 40% avec la SEQ ID3 à condition que l'enzyme conserve la même activité.

5. Molécule protéique hybride selon l'une quelconque des revendications précédentes **caractérisée en ce que** les enzymes sont fusionnées en une protéine unique monocaténaire.

6. Molécule protéique hybride selon l'une quelconque des revendications précédentes **caractérisée en ce que** les enzymes sont liées entre elles de manière covalente par cross-linking.

7. Molécule protéique hybride selon l'une quelconque des revendications 1, 5 ou 6 **caractérisée en ce que** l'une des bêta-lactamases est de type IR-TEM et l'autre bêta-lactamase est une cefotaximase de type CTX-M.

8. Molécule protéique hybride selon la revendication 7 **caractérisée en ce que** sa séquence correspond à SEQ ID NO.8, SEQ ID NO.9 ou SEQ ID NO.10.

9. Composition pharmaceutique à usage humain ou vétérinaire comportant la molécule protéique hybride selon l'une quelconque des revendications précédentes pour une utilisation dans la prévention des modifications de la flore intestinale.

10. Composition pharmaceutique selon la revendication 9 pour une utilisation dans la prévention des infections nosocomiales.

11. Composition pharmaceutique selon la revendication 9 ou 10 pour une utilisation dans la prévention des diarrhées associées à la prise d'antibiotiques.

12. Composition pharmaceutique selon l'une quelconque des revendications 9 à 11 **caractérisée en ce qu'**elle est sous une forme galénique pour une administration par voie orale.

13. Composition pharmaceutique selon l'une quelconque des revendications 9 à 12 **caractérisée en ce qu'**elle comporte en outre au moins un agent gastroprotecteur.

14. Procédé de production de la molécule protéique hybride conformément à l'une quelconque des revendications 1 à 8 dans un organisme recombinant vivant non humain.

## Patentansprüche

1. Hybrides Proteinmolekül mit zwei Enzymen, die die Aktivität von mindestens einem Antibiotikum hemmen, wobei die beiden Enzyme unterschiedliche biochemische Eigenschaften haben und besagte Enzyme zwei miteinander verknüpfte Betalaktamasen sind.

2. Hybrides Proteinmolekül nach einem der vorhergehenden Ansprüche, **dadurch gekennzeichnet, dass** die Sequenz von mindestens einem der Enzyme mindestens 40% Sequenzhomologie zur SEQ ID1 aufweist, unter der Bedingung, dass das Enzym seine Aktivität in gleichem Maße behält.

3. Hybride Proteinmolekül nach einem der vorhergehenden Ansprüche, **dadurch gekennzeichnet, dass** die Sequenz von mindestens einem der Betalaktamasen mindestens 40% Sequenzhomologie zur SEQ ID2 aufweist, unter der Bedingung, dass das Enzym seine Aktivität in gleichem Maße behält.

4. Hybrides Proteinmolekül nach einem der vorhergehenden Ansprüche, **dadurch gekennzeichnet, dass** die Sequenz von mindestens einem der Betalaktamasen mindestens 40% Sequenzhomologie zur SEQ ID3 aufweist, unter der Bedingung, dass das Enzym seine Aktivität in gleichem Maße behält.

5. Hybrides Proteinmolekül nach einem der vorhergehenden Ansprüche, **dadurch gekennzeichnet, dass** die Enzyme in einem einzigen einzelsträngigen Protein zusammengeführt sind.

6. Hybrides Proteinmolekül nach einem der vorhergehenden Ansprüche, **dadurch gekennzeichnet, dass** die Enzyme durch Crosslinking kovalent miteinander verknüpft sind.

7. Hybrides Proteinmolekül nach einem der Ansprüche 1, 5 oder 6, **dadurch gekennzeichnet, dass** eine der Betalaktamasen vom Typ IR-TEM und die andere Betalaktamase eine Cefotaximase vom Typ CTX-M ist.

8. Hybrides Proteinmolekül nach Anspruch 7, **dadurch gekennzeichnet, dass** seine Sequenz SEQ ID NO.8, SEQ ID NO.9 oder SEQ ID NO.10 ist.

9. Pharmazeutische Zusammensetzung für Menschen oder Tiere, welche das hybride Proteinmolekül nach einem der vorhergehenden Ansprüche zur Prävention einer Veränderung der Darmflora enthält.

10. Pharmazeutische Zusammensetzung nach Anspruch 9 zur Prävention von nosokomialen Infektionen.

11. Pharmazeutische Zusammensetzung nach Anspruch 9 oder 10 zur Prävention von Diarrhoe in Verbindung mit der Einnahme von Antibiotika.

12. Pharmazeutische Zusammensetzung nach einem der Ansprüche 9 bis 11, **dadurch gekennzeichnet, dass** sie in galenischer Form dargereicht wird, um oral verabreicht werden zu können.

13. Pharmazeutische Zusammensetzung nach einem der Ansprüche 9 bis 12, **dadurch gekennzeichnet, dass** sie des Weiteren mindestens einen gastroprotektiven Wirkstoff umfasst.

14. Verfahren zur Herstellung des hybriden Proteinmoleküls gemäß einem der Ansprüche 1 bis 8 in einem lebenden, nicht humanen rekombinanten Organismus.

## Claims

1. Hybrid protein molecule comprising two enzymes inhibiting the activity of at least one antibiotic, wherein the two enzymes have different biochemical characteristics and said enzymes are two interlinked beta-lactamases.

2. Hybrid protein molecule according to any one of the preceding claims, **characterised in that** the sequence of at least one of the enzymes has a sequence homology of at least 40% with SEQ ID1 provided that the enzyme retains the same activity.

3. Hybrid protein molecule according to any one of the preceding claims, **characterised in that** the sequence of at least one of the beta-lactamases has a sequence homology of at least 40% with SEQ ID2 provided that the enzyme retains the same activity.

4. Hybrid protein molecule according to any one of the preceding claims, **characterised in that** the sequence of at least one of the beta-lactamases has a sequence homology of at least 40% with SEQ ID3 provided that the enzyme retains the same activity.

5. Hybrid protein molecule according to any one of the preceding claims, **characterised in that** the enzymes are fused into one single-strand protein.

6. Hybrid protein molecule according to any one of the preceding claims, **characterised in that** the enzymes are covalently interlinked by cross-linking.

7. Hybrid protein molecule according to any one of claims 1, 5 or 6, **characterised in that** one of the beta-lactamases is of IR-TEM type and the other beta-lactamase is a CTX-M-type cefotaximase.

8. Hybrid protein molecule according to claim 7, **characterised in that** its sequence corresponds to SEQ ID NO. 8, SEQ ID NO. 9 or SEQ ID NO. 10.

9. Pharmaceutical composition for human or veterinary use comprising the hybrid protein molecule according to any one of the preceding claims for use in preventing changes in the intestinal flora.

10. Pharmaceutical composition according to claim 9 for use in preventing nosocomial infections.

11. Pharmaceutical composition according to claim 9 or 10 for use in preventing diarrhoea associated with administration of antibiotics.

12. Pharmaceutical composition according to any one of claims 9 to 11, **characterised in that** its pharmaceutical form is suitable for oral administration.

13. Pharmaceutical composition according to any one of claims 9 to 12, **characterised in that** it additionally comprises at least one gastroprotective agent.

14. Production process for the hybrid protein molecule in accordance with any one of claims 1 to 8 in a non-human recombinant organism.


### SEQUENCE LISTING

<110> AZURRX SAS
<120> MOLECULE PROTEIQUE HYBRIDE APTE A INHIBER AU MOINS UN ANTIBIOTIQUE ET COMPOSITION PHARMACEUTIQUE LA COMPORTANT
<130> AZR1 PCT
<150> FR1459935
   <151> 2014-10-16
<160> 44
<170> PatentIn version 3.5
<210> 1
   <211> 263
   <212> PRT
   <213> Escherichia coli
<400> 1
   His Pro Glu Thr Leu Val Lys Val Lys Asp Ala Glu Asp Gln Leu Gly
   1               5                   10                  15
   Ala Arg Val Gly Tyr Ile Glu Leu Asp Leu Asn Ser Gly Lys Ile Leu
               20                  25                  30
   Glu Ser Phe Arg Pro Glu Glu Arg Phe Pro Met Val Ser Thr Phe Lys
           35                  40                  45
   Val Leu Leu Cys Gly Ala Val Leu Ser Arg Val Asp Ala Gly Gln Glu
       50                  55                  60
   Gln Leu Gly Arg Arg Ile His Tyr Ser Gln Asn Asp Leu Val Glu Tyr
   65                  70                  75                  80
   Ser Pro Val Thr Glu Lys His Leu Thr Asp Gly Met Thr Val Arg Glu
                   85                  90                  95
   Leu Cys Ser Ala Ala Ile Thr Met Ser Asp Asn Thr Ala Ala Asn Leu
               100                 105                 110
   Leu Leu Thr Thr Ile Gly Gly Pro Lys Glu Leu Thr Ala Phe Leu His
           115                 120                 125
   Asn Met Gly Asp His Val Thr Arg Leu Asp Arg Trp Glu Pro Glu Leu
       130                 135                 140
   Asn Glu Ala Ile Pro Asn Asp Glu Arg Asp Thr Thr Met Pro Ala Ala
   145                 150                 155                 160
   Met Ala Thr Thr Leu Arg Lys Leu Leu Thr Gly Glu Leu Leu Thr Leu
                   165                 170                 175
   Ala Ser Arg Gln Gln Leu Ile Asp Trp Met Glu Ala Asp Lys Val Ala
               180                 185                 190
   Gly Pro Leu Leu Arg Ser Ala Leu Pro Ala Gly Trp Phe Ile Ala Asp
           195                 200                 205
   Lys Ser Gly Ala Gly Glu Arg Gly Ser Arg Gly Ile Ile Ala Ala Leu
       210                 215                 220
   Gly Pro Asp Gly Lys Pro Ser Arg Ile Val Val Ile Tyr Thr Thr Gly
   225                 230                 235                 240
   Ser Gln Ala Thr Met Asp Glu Arg Asp Arg Gln Ile Ala Glu Ile Gly
                   245                 250                 255
   Ala Ser Leu Ile Lys His Trp
   260
<210> 2
   <211> 263
   <212> PRT
   <213> Escherichia coli
<400> 2
   Gln Thr Ser Ala Val Gln Gln Lys Leu Ala Ala Leu Glu Lys Ser Ser
   1               5                   10                  15
   Gly Gly Arg Leu Gly Val Ala Leu Ile Asp Thr Ala Asp Asn Thr Gln
               20                  25                  30
   Val Leu Tyr Arg Gly Asp Glu Arg Phe Pro Met Cys Ser Thr Ser Lys
           35                  40                  45
   Val Met Ala Ala Ala Ala Val Leu Lys Gln Ser Glu Thr Gln Lys Gln
       50                  55                  60
   Leu Leu Asn Gln Pro Val Glu Ile Lys Pro Ala Asp Leu Val Asn Tyr
   65                  70                  75                  80
   Asn Pro Ile Ala Glu Lys His Val Asn Gly Thr Met Thr Leu Ala Glu
                   85                  90                  95
   Leu Ser Ala Ala Ala Leu Gln Tyr Ser Asp Asn Thr Ala Met Asn Lys
               100                 105                 110
   Leu Ile Ala Gln Leu Gly Gly Pro Gly Gly Val Thr Ala Phe Ala Arg
           115                 120                 125
   Ala Ile Gly Asp Glu Thr Phe Arg Leu Asp Arg Thr Glu Pro Thr Leu
       130                 135                 140
   Asn Thr Ala Ile Pro Gly Asp Pro Arg Asp Thr Thr Thr Pro Arg Ala
   145                 150                 155                 160
   Met Ala Gln Thr Leu Arg Gln Leu Thr Leu Gly His Ala Leu Gly Glu
                   165                 170                 175
   Thr Gln Arg Ala Gln Leu Val Thr Trp Leu Lys Gly Asn Thr Thr Gly
               180                 185                 190
   Ala Ala Ser Ile Arg Ala Gly Leu Pro Thr Ser Trp Thr Ala Gly Asp
           195                 200                 205
   Lys Thr Gly Ser Gly Gly Tyr Gly Thr Thr Asn Asp Ile Ala Val Ile
       210                 215                 220
   Trp Pro Gln Gly Arg Ala Pro Leu Val Leu Val Thr Tyr Phe Thr Gln
   225                 230                 235                 240
   Pro Gln Gln Asn Ala Glu Ser Arg Arg Asp Val Leu Ala Ser Ala Ala
                   245                 250                 255
   Arg Ile Ile Ala Glu Gly Leu
   260
<210> 3
   <211> 257
   <212> PRT
   <213> Staphylococcus aureus
<400> 3
   Lys Glu Leu Asn Asp Leu Glu Lys Lys Tyr Asn Ala His Ile Gly Val
   1               5                   10                  15
   Tyr Ala Leu Asp Thr Lys Ser Gly Lys Glu Val Lys Phe Asn Ser Asp
               20                  25                  30
   Lys Arg Phe Ala Tyr Ala Ser Thr Ser Lys Ala Ile Asn Ser Ala Ile
           35                  40                  45
   Leu Leu Glu Gln Val Pro Tyr Asn Lys Leu Asn Lys Lys Val His Ile
       50                  55                  60
   Asn Lys Asp Asp Ile Val Ala Tyr Ser Pro Ile Leu Glu Lys Tyr Val
   65                  70                  75                  80
   Gly Lys Asp Ile Thr Leu Lys Ala Leu Ile Glu Ala Ser Met Thr Tyr
                   85                  90                  95
   Ser Asp Asn Thr Ala Asn Asn Lys Ile Ile Lys Glu Ile Gly Gly Ile
               100                 105                 110
   Lys Lys Val Lys Gln Arg Leu Lys Glu Leu Gly Asp Lys Val Thr Asn
           115                 120                 125
   Pro Val Arg Tyr Glu Ile Glu Leu Asn Tyr Tyr Ser Pro Lys Ser Lys
       130                 135                 140
   Lys Asp Thr Ser Thr Pro Ala Ala Phe Gly Lys Thr Leu Asn Lys Leu
   145                 150                 155                 160
   Ile Ala Asn Gly Lys Leu Ser Lys Glu Asn Lys Lys Phe Leu Leu Asp
                   165                 170                 175
   Leu Met Leu Asn Asn Lys Ser Gly Asp Thr Leu Ile Lys Asp Gly Val
               180                 185                 190
   Pro Lys Asp Tyr Lys Val Ala Asp Lys Ser Gly Gln Ala Ile Thr Tyr
           195                 200                 205
   Ala Ser Arg Asn Asp Val Ala Phe Val Tyr Pro Lys Gly Gln Ser Glu
       210                 215                 220
   Pro Ile Val Leu Val Ile Phe Thr Asn Lys Asp Asn Lys Ser Asp Lys
   225                 230                 235                 240
   Pro Asn Asp Lys Leu Ile Ser Glu Thr Ala Lys Ser Val Met Lys Glu
                   245                 250                 255
   Phe
<210> 4
   <211> 199
   <212> PRT
   <213> Escherichia coli
<400> 4
   Met Ser Asn Ala Lys Thr Lys Leu Gly Ile Thr Lys Tyr Ser Ile Val
   1               5                   10                  15
   Thr Asn Ser Asn Asp Ser Val Thr Leu Arg Leu Met Thr Glu His Asp
               20                  25                  30
   Leu Ala Met Leu Tyr Glu Trp Leu Asn Arg Ser His Ile Val Glu Trp
           35                  40                  45
   Trp Gly Gly Glu Glu Ala Arg Pro Thr Leu Ala Asp Val Gln Glu Gln
       50                  55                  60
   Tyr Leu Pro Ser Val Leu Ala Gln Glu Ser Val Thr Pro Tyr Ile Ala
   65                  70                  75                  80
   Met Leu Asn Gly Glu Pro Ile Gly Tyr Ala Gln Ser Tyr Val Ala Leu
                   85                  90                  95
   Gly Ser Gly Asp Gly Arg Trp Glu Glu Glu Thr Asp Pro Gly Val Arg
               100                 105                 110
   Gly Ile Asp Gln Leu Leu Ala Asn Ala Ser Gln Leu Gly Lys Gly Leu
           115                 120                 125
   Gly Thr Lys Leu Val Arg Ala Leu Val Glu Leu Leu Phe Asn Asp Pro
       130                 135                 140
   Glu Val Thr Lys Ile Gln Thr Asp Pro Ser Pro Ser Asn Leu Arg Ala
   145                 150                 155                 160
   Ile Arg Cys Tyr Glu Lys Ala Gly Phe Glu Arg Gln Gly Thr Val Thr
                   165                 170                 175
   Thr Pro Tyr Gly Pro Ala Val Tyr Met Val Gln Thr Arg Gln Ala Phe
               180                 185                 190
   Glu Arg Thr Arg Ser Asp Ala
   195
<210> 5
   <211> 419
   <212> PRT
   <213> Escherichia coli
<400> 5
   Met Arg Phe Glu Glu Trp Val Lys Asp Lys His Ile Pro Phe Lys Leu
   1               5                   10                  15
   Asn His Pro Asp Asp Asn Tyr Asp Asp Phe Lys Pro Leu Arg Lys Ile
               20                  25                  30
   Ile Gly Asp Thr Arg Val Val Ala Leu Gly Glu Asn Ser His Phe Ile
   35 40 45
   Lys Glu Phe Phe Leu Leu Arg His Thr Leu Leu Arg Phe Phe Ile Glu
   50 55 60
   Asp Leu Gly Phe Thr Thr Phe Ala Phe Glu Phe Gly Phe Ala Glu Gly
   65 70 75 80
   Gln Ile Ile Asn Asn Trp Ile His Gly Gln Gly Thr Asp Asp Glu Ile
   85 90 95
   Gly Arg Phe Leu Lys His Phe Tyr Tyr Pro Glu Glu Leu Lys Thr Thr
   100 105 110
   Phe Leu Trp Leu Arg Glu Tyr Asn Lys Ala Ala Lys Glu Lys Ile Thr
   115 120 125
   Phe Leu Gly Ile Asp Ile Pro Arg Asn Gly Gly Ser Tyr Leu Pro Asn
   130 135 140
   Met Glu Ile Val His Asp Phe Phe Arg Thr Ala Asp Lys Glu Ala Leu
   145 150 155 160
   His Ile Ile Asp Asp Ala Phe Asn Ile Ala Lys Lys Ile Asp Tyr Phe
   165 170 175
   Ser Thr Ser Gln Ala Ala Leu Asn Leu His Glu Leu Thr Asp Ser Glu
   180 185 190
   Lys Cys Arg Leu Thr Ser Gln Leu Ala Arg Val Lys Val Arg Leu Glu
   195 200 205
   Ala Met Ala Pro Ile His Ile Glu Lys Tyr Gly Ile Asp Lys Tyr Glu
   210 215 220
   Thr Ile Leu His Tyr Ala Asn Gly Met Ile Tyr Leu Asp Tyr Asn Ile
   225 230 235 240
   Gln Ala Met Ser Gly Phe Ile Ser Gly Gly Gly Met Gln Gly Asp Met
   245 250 255
   Gly Ala Lys Asp Lys Tyr Met Ala Asp Ser Val Leu Trp His Leu Lys
   260 265 270
   Asn Pro Gln Ser Glu Gln Lys Val Ile Val Val Ala His Asn Ala His
   275 280 285
   Ile Gln Lys Thr Pro Ile Leu Tyr Asp Gly Phe Leu Ser Cys Leu Pro
       290                 295                 300
   Met Gly Gln Arg Leu Lys Asn Ala Ile Gly Asp Asp Tyr Met Ser Leu
   305                 310                 315                 320
   Gly Ile Thr Ser Tyr Ser Gly His Thr Ala Ala Leu Tyr Pro Glu Val
                   325                 330                 335
   Asp Thr Lys Tyr Gly Phe Arg Val Asp Asn Phe Gln Leu Gln Glu Pro
               340                 345                 350
   Asn Glu Gly Ser Val Glu Lys Ala Ile Ser Gly Cys Gly Val Thr Asn
           355                 360                 365
   Ser Phe Val Phe Phe Arg Asn Ile Pro Glu Asp Leu Gln Ser Ile Pro
       370                 375                 380
   Asn Met Ile Arg Phe Asp Ser Ile Tyr Met Lys Ala Glu Leu Glu Lys
   385                 390                 395                 400
   Ala Phe Asp Gly Ile Phe Gln Ile Glu Lys Ser Ser Val Ser Glu Val
                   405                 410                 415
   Val Tyr Glu
<210> 6
   <211> 388
   <212> PRT
   <213> Bacteroides fragilis
<400> 6
   Met Thr Met Arg Ile Asp Thr Asp Lys Gln Met Asn Leu Leu Ser Asp
   1               5                   10                  15
   Lys Asn Val Ala Ile Ile Gly Gly Gly Pro Val Gly Leu Thr Met Ala
               20                  25                  30
   Lys Leu Leu Gln Gln Asn Gly Ile Asp Val Ser Val Tyr Glu Arg Asp
           35                  40                  45
   Asn Asp Arg Glu Ala Arg Ile Phe Gly Gly Thr Leu Asp Leu His Lys
       50                  55                  60
   Gly Ser Gly Gln Glu Ala Met Lys Lys Ala Gly Leu Leu Gln Thr Tyr
   65                  70                  75                  80
   Tyr Asp Leu Ala Leu Pro Met Gly Val Asn Ile Ala Asp Lys Lys Gly
                   85                  90                  95
   Asn Ile Leu Ser Thr Lys Asn Val Lys Pro Glu Asn Arg Phe Asp Asn
               100                 105                 110
   Pro Glu Ile Asn Arg Asn Asp Leu Arg Ala Ile Leu Leu Asn Ser Leu
           115                 120                 125
   Glu Asn Asp Thr Val Ile Trp Asp Arg Lys Leu Val Met Leu Glu Pro
       130                 135                 140
   Gly Lys Lys Lys Trp Thr Leu Thr Phe Glu Asn Lys Pro Ser Glu Thr
   145                 150                 155                 160
   Ala Asp Leu Val Ile Leu Ala Asn Gly Gly Met Ser Lys Val Arg Lys
                   165                 170                 175
   Phe Val Thr Asp Thr Glu Val Glu Glu Thr Gly Thr Phe Asn Ile Gln
               180                 185                 190
   Ala Asp Ile His Gln Pro Glu Ile Asn Cys Pro Gly Phe Phe Gln Leu
           195                 200                 205
   Cys Asn Gly Asn Arg Leu Met Ala Ser His Gln Gly Asn Leu Leu Phe
       210                 215                 220
   Ala Asn Pro Asn Asn Asn Gly Ala Leu His Phe Gly Ile Ser Phe Lys
   225                 230                 235                 240
   Thr Pro Asp Glu Trp Lys Asn Gln Thr Gln Val Asp Phe Gln Asn Arg
                   245                 250                 255
   Asn Ser Val Val Asp Phe Leu Leu Lys Glu Phe Ser Asp Trp Asp Glu
               260                 265                 270
   Arg Tyr Lys Glu Leu Ile His Thr Thr Leu Ser Phe Val Gly Leu Ala
           275                 280                 285
   Thr Arg Ile Phe Pro Leu Glu Lys Pro Trp Lys Ser Lys Arg Pro Leu
       290                 295                 300
   Pro Ile Thr Met Ile Gly Asp Ala Ala His Leu Met Pro Pro Phe Ala
   305                 310                 315                 320
   Gly Gln Gly Val Asn Ser Gly Leu Val Asp Ala Leu Ile Leu Ser Asp
                   325                 330                 335
   Asn Leu Ala Asp Gly Lys Phe Asn Ser Ile Glu Glu Ala Val Lys Asn
               340                 345                 350
   Tyr Glu Gln Gln Met Phe Met Tyr Gly Lys Glu Ala Gln Glu Glu Ser
           355                 360                 365
   Thr Gln Asn Glu Ile Glu Met Phe Lys Pro Asp Phe Thr Phe Gln Gln
       370                 375                 380
   Leu Leu Asn Val
   385
<210> 7
   <211> 267
   <212> PRT
   <213> Enterococcus faecium
<400> 7
   Met Leu Lys Gln Lys Glu Leu Ile Ala Asn Val Lys Asn Leu Thr Glu
   1               5                   10                  15
   Ser Asp Glu Arg Ile Thr Ala Cys Met Met Tyr Gly Ser Phe Thr Lys
               20                  25                  30
   Gly Glu Gly Asp Gln Tyr Ser Asp Ile Glu Phe Tyr Ile Phe Leu Lys
           35                  40                  45
   His Ser Ile Thr Ser Asn Phe Asp Ser Ser Asn Trp Leu Phe Asp Val
       50                  55                  60
   Ala Pro Tyr Leu Met Leu Tyr Lys Asn Glu Tyr Gly Thr Glu Val Val
   65                  70                  75                  80
   Ile Phe Asp Asn Leu Ile Arg Gly Glu Phe His Phe Leu Ser Glu Lys
                   85                  90                  95
   Asp Met Asn Ile Ile Pro Ser Phe Lys Asp Ser Gly Tyr Ile Pro Asp
               100                 105                 110
   Thr Lys Ala Met Leu Ile Tyr Asp Glu Thr Gly Gln Leu Glu Asn Tyr
           115                 120                 125
   Leu Ser Glu Ile Ser Gly Ala Arg Pro Asn Arg Leu Thr Glu Glu Asn
       130                 135                 140
   Ala Asn Phe Leu Leu Cys Asn Phe Ser Asn Leu Trp Leu Met Gly Ile
   145                 150                 155                 160
   Asn Val Leu Lys Arg Gly Glu Tyr Ala Arg Ser Leu Glu Leu Leu Ser
   165 170 175
   Gln Leu Gln Lys Asn Thr Leu Gln Leu Ile Arg Met Ala Glu Lys Asn
   180 185 190
   Ala Asp Asn Trp Leu Asn Met Ser Lys Asn Leu Glu Lys Glu Ile Ser
   195 200 205
   Leu Glu Asn Tyr Lys Lys Phe Ala Lys Thr Thr Ala Arg Leu Asp Lys
   210 215 220
   Val Glu Leu Phe Glu Ala Tyr Lys Asn Ser Leu Leu Leu Val Met Asp
   225 230 235 240
   Leu Gln Ser His Leu Ile Glu Gln Tyr Asn Leu Lys Val Thr His Asp
   245 250 255
   Ile Leu Glu Arg Leu Leu Asn Tyr Ile Ser Glu
   260 265
<210> 8
   <211> 532
   <212> PRT
   <213> Artificial Sequence
<220>
   <223> fusion 1 TEM36-6G-CTXM16
<400> 8
   His Pro Glu Thr Leu Val Lys Val Lys Asp Ala Glu Asp Gln Leu Gly
   1 5 10 15
   Ala Arg Val Gly Tyr Ile Glu Leu Asp Leu Asn Ser Gly Lys Ile Leu
   20 25 30
   Glu Ser Phe Arg Pro Glu Glu Arg Phe Pro Met Val Ser Thr Phe Lys
   35 40 45
   Val Leu Leu Cys Gly Ala Val Leu Ser Arg Val Asp Ala Gly Gln Glu
   50 55 60
   Gln Leu Gly Arg Arg Ile His Tyr Ser Gln Asn Asp Leu Val Glu Tyr
   65 70 75 80
   Ser Pro Val Thr Glu Lys His Leu Thr Asp Gly Met Thr Val Arg Glu
   85 90 95
   Leu Cys Ser Ala Ala Ile Thr Met Ser Asp Asn Thr Ala Ala Asn Leu
               100                 105                 110
   Leu Leu Thr Thr Ile Gly Gly Pro Lys Glu Leu Thr Ala Phe Leu His
           115                 120                 125
   Asn Met Gly Asp His Val Thr Arg Leu Asp Arg Trp Glu Pro Glu Leu
       130                 135                 140
   Asn Glu Ala Ile Pro Asn Asp Glu Arg Asp Thr Thr Met Pro Ala Ala
   145                 150                 155                 160
   Met Ala Thr Thr Leu Arg Lys Leu Leu Thr Gly Glu Leu Leu Thr Leu
                   165                 170                 175
   Ala Ser Arg Gln Gln Leu Ile Asp Trp Met Glu Ala Asp Lys Val Ala
               180                 185                 190
   Gly Pro Leu Leu Arg Ser Ala Leu Pro Ala Gly Trp Phe Ile Ala Asp
           195                 200                 205
   Lys Ser Gly Ala Gly Glu Arg Gly Ser Arg Gly Ile Ile Ala Ala Leu
       210                 215                 220
   Gly Pro Asp Gly Lys Pro Ser Arg Ile Val Val Ile Tyr Thr Thr Gly
   225                 230                 235                 240
   Ser Gln Ala Thr Met Asp Glu Arg Asp Arg Gln Ile Ala Glu Ile Gly
                   245                 250                 255
   Ala Ser Leu Ile Lys His Trp Gly Gly Gly Gly Gly Gly Gln Thr Ser
               260                 265                 270
   Ala Val Gln Gln Lys Leu Ala Ala Leu Glu Lys Ser Ser Gly Gly Arg
           275                 280                 285
   Leu Gly Val Ala Leu Ile Asp Thr Ala Asp Asn Thr Gln Val Leu Tyr
       290                 295                 300
   Arg Gly Asp Glu Arg Phe Pro Met Cys Ser Thr Ser Lys Val Met Ala
   305                 310                 315                 320
   Ala Ala Ala Val Leu Lys Gln Ser Glu Thr Gln Lys Gln Leu Leu Asn
                   325                 330                 335
   Gln Pro Val Glu Ile Lys Pro Ala Asp Leu Val Asn Tyr Asn Pro Ile
               340                 345                 350
   Ala Glu Lys His Val Asn Gly Thr Met Thr Leu Ala Glu Leu Ser Ala
           355                 360                 365
   Ala Ala Leu Gln Tyr Ser Asp Asn Thr Ala Met Asn Lys Leu Ile Ala
       370                 375                 380
   Gln Leu Gly Gly Pro Gly Gly Val Thr Ala Phe Ala Arg Ala Ile Gly
   385                 390                 395                 400
   Asp Glu Thr Phe Arg Leu Asp Arg Thr Glu Pro Thr Leu Asn Thr Ala
                   405                 410                 415
   Ile Pro Gly Asp Pro Arg Asp Thr Thr Thr Pro Arg Ala Met Ala Gln
               420                 425                 430
   Thr Leu Arg Gln Leu Thr Leu Gly His Ala Leu Gly Glu Thr Gln Arg
           435                 440                 445
   Ala Gln Leu Val Thr Trp Leu Lys Gly Asn Thr Thr Gly Ala Ala Ser
       450                 455                 460
   Ile Arg Ala Gly Leu Pro Thr Ser Trp Thr Ala Gly Asp Lys Thr Gly
   465                 470                 475                 480
   Ser Gly Gly Tyr Gly Thr Thr Asn Asp Ile Ala Val Ile Trp Pro Gln
                   485                 490                 495
   Gly Arg Ala Pro Leu Val Leu Val Thr Tyr Phe Thr Gln Pro Gln Gln
               500                 505                 510
   Asn Ala Glu Ser Arg Arg Asp Val Leu Ala Ser Ala Ala Arg Ile Ile
           515                 520                 525
   Ala Glu Gly Leu
   530
<210> 9
   <211> 532
   <212> PRT
   <213> Artificial Sequence
<220>
   <223> fusion 2 CTXM16-6G-TEM36
<400> 9
   Gln Thr Ser Ala Val Gln Gln Lys Leu Ala Ala Leu Glu Lys Ser Ser
   1               5                   10                  15
   Gly Gly Arg Leu Gly Val Ala Leu Ile Asp Thr Ala Asp Asn Thr Gln
               20                  25                  30
   Val Leu Tyr Arg Gly Asp Glu Arg Phe Pro Met Cys Ser Thr Ser Lys
           35                  40                  45
   Val Met Ala Ala Ala Ala Val Leu Lys Gln Ser Glu Thr Gln Lys Gln
       50                  55                  60
   Leu Leu Asn Gln Pro Val Glu Ile Lys Pro Ala Asp Leu Val Asn Tyr
   65                  70                  75                  80
   Asn Pro Ile Ala Glu Lys His Val Asn Gly Thr Met Thr Leu Ala Glu
                   85                  90                  95
   Leu Ser Ala Ala Ala Leu Gln Tyr Ser Asp Asn Thr Ala Met Asn Lys
               100                 105                 110
   Leu Ile Ala Gln Leu Gly Gly Pro Gly Gly Val Thr Ala Phe Ala Arg
           115                 120                 125
   Ala Ile Gly Asp Glu Thr Phe Arg Leu Asp Arg Thr Glu Pro Thr Leu
       130                 135                 140
   Asn Thr Ala Ile Pro Gly Asp Pro Arg Asp Thr Thr Thr Pro Arg Ala
   145                 150                 155                 160
   Met Ala Gln Thr Leu Arg Gln Leu Thr Leu Gly His Ala Leu Gly Glu
                   165                 170                 175
   Thr Gln Arg Ala Gln Leu Val Thr Trp Leu Lys Gly Asn Thr Thr Gly
               180                 185                 190
   Ala Ala Ser Ile Arg Ala Gly Leu Pro Thr Ser Trp Thr Ala Gly Asp
           195                 200                 205
   Lys Thr Gly Ser Gly Gly Tyr Gly Thr Thr Asn Asp Ile Ala Val Ile
       210                 215                 220
   Trp Pro Gln Gly Arg Ala Pro Leu Val Leu Val Thr Tyr Phe Thr Gln
   225                 230                 235                 240
   Pro Gln Gln Asn Ala Glu Ser Arg Arg Asp Val Leu Ala Ser Ala Ala
                   245                 250                 255
   Arg Ile Ile Ala Glu Gly Leu Gly Gly Gly Gly Gly Gly His Pro Glu
               260                 265                 270
   Thr Leu Val Lys Val Lys Asp Ala Glu Asp Gln Leu Gly Ala Arg Val
           275                 280                 285
   Gly Tyr Ile Glu Leu Asp Leu Asn Ser Gly Lys Ile Leu Glu Ser Phe
       290                 295                 300
   Arg Pro Glu Glu Arg Phe Pro Met Val Ser Thr Phe Lys Val Leu Leu
   305                 310                 315                 320
   Cys Gly Ala Val Leu Ser Arg Val Asp Ala Gly Gln Glu Gln Leu Gly
                   325                 330                 335
   Arg Arg Ile His Tyr Ser Gln Asn Asp Leu Val Glu Tyr Ser Pro Val
               340                 345                 350
   Thr Glu Lys His Leu Thr Asp Gly Met Thr Val Arg Glu Leu Cys Ser
           355                 360                 365
   Ala Ala Ile Thr Met Ser Asp Asn Thr Ala Ala Asn Leu Leu Leu Thr
       370                 375                 380
   Thr Ile Gly Gly Pro Lys Glu Leu Thr Ala Phe Leu His Asn Met Gly
   385                 390                 395                 400
   Asp His Val Thr Arg Leu Asp Arg Trp Glu Pro Glu Leu Asn Glu Ala
                   405                 410                 415
   Ile Pro Asn Asp Glu Arg Asp Thr Thr Met Pro Ala Ala Met Ala Thr
               420                 425                 430
   Thr Leu Arg Lys Leu Leu Thr Gly Glu Leu Leu Thr Leu Ala Ser Arg
           435                 440                 445
   Gln Gln Leu Ile Asp Trp Met Glu Ala Asp Lys Val Ala Gly Pro Leu
       450                 455                 460
   Leu Arg Ser Ala Leu Pro Ala Gly Trp Phe Ile Ala Asp Lys Ser Gly
   465                 470                 475                 480
   Ala Gly Glu Arg Gly Ser Arg Gly Ile Ile Ala Ala Leu Gly Pro Asp
                   485                 490                 495
   Gly Lys Pro Ser Arg Ile Val Val Ile Tyr Thr Thr Gly Ser Gln Ala
               500                 505                 510
   Thr Met Asp Glu Arg Asp Arg Gln Ile Ala Glu Ile Gly Ala Ser Leu
   515 520 525
   Ile Lys His Trp
   530
<210> 10
   <211> 537
   <212> PRT
   <213> Artificial Sequence
<220>
   <223> fusion 3 TEM36-G(EAAAK)2-CTXM16
<400> 10
   His Pro Glu Thr Leu Val Lys Val Lys Asp Ala Glu Asp Gln Leu Gly
   1               5                   10                  15
   Ala Arg Val Gly Tyr Ile Glu Leu Asp Leu Asn Ser Gly Lys Ile Leu
               20                  25                  30
   Glu Ser Phe Arg Pro Glu Glu Arg Phe Pro Met Val Ser Thr Phe Lys
           35                  40                  45
   Val Leu Leu Cys Gly Ala Val Leu Ser Arg Val Asp Ala Gly Gln Glu
       50                  55                  60
   Gln Leu Gly Arg Arg Ile His Tyr Ser Gln Asn Asp Leu Val Glu Tyr
   65                  70                  75                  80
   Ser Pro Val Thr Glu Lys His Leu Thr Asp Gly Met Thr Val Arg Glu
                   85                  90                  95
   Leu Cys Ser Ala Ala Ile Thr Met Ser Asp Asn Thr Ala Ala Asn Leu
               100                 105                 110
   Leu Leu Thr Thr Ile Gly Gly Pro Lys Glu Leu Thr Ala Phe Leu His
           115                 120                 125
   Asn Met Gly Asp His Val Thr Arg Leu Asp Arg Trp Glu Pro Glu Leu
       130                 135                 140
   Asn Glu Ala Ile Pro Asn Asp Glu Arg Asp Thr Thr Met Pro Ala Ala
   145                 150                 155                 160
   Met Ala Thr Thr Leu Arg Lys Leu Leu Thr Gly Glu Leu Leu Thr Leu
                   165                 170                 175
   Ala Ser Arg Gln Gln Leu Ile Asp Trp Met Glu Ala Asp Lys Val Ala
               180                 185                 190
   Gly Pro Leu Leu Arg Ser Ala Leu Pro Ala Gly Trp Phe Ile Ala Asp
           195                 200                 205
   Lys Ser Gly Ala Gly Glu Arg Gly Ser Arg Gly Ile Ile Ala Ala Leu
       210                 215                 220
   Gly Pro Asp Gly Lys Pro Ser Arg Ile Val Val Ile Tyr Thr Thr Gly
   225                 230                 235                 240
   Ser Gln Ala Thr Met Asp Glu Arg Asp Arg Gln Ile Ala Glu Ile Gly
                   245                 250                 255
   Ala Ser Leu Ile Lys His Trp Gly Glu Ala Ala Ala Lys Glu Ala Ala
               260                 265                 270
   Ala Lys Gln Thr Ser Ala Val Gln Gln Lys Leu Ala Ala Leu Glu Lys
           275                 280                 285
   Ser Ser Gly Gly Arg Leu Gly Val Ala Leu Ile Asp Thr Ala Asp Asn
       290                 295                 300
   Thr Gln Val Leu Tyr Arg Gly Asp Glu Arg Phe Pro Met Cys Ser Thr
   305                 310                 315                 320
   Ser Lys Val Met Ala Ala Ala Ala Val Leu Lys Gln Ser Glu Thr Gln
                   325                 330                 335
   Lys Gln Leu Leu Asn Gln Pro Val Glu Ile Lys Pro Ala Asp Leu Val
               340                 345                 350
   Asn Tyr Asn Pro Ile Ala Glu Lys His Val Asn Gly Thr Met Thr Leu
           355                 360                 365
   Ala Glu Leu Ser Ala Ala Ala Leu Gln Tyr Ser Asp Asn Thr Ala Met
       370                 375                 380
   Asn Lys Leu Ile Ala Gln Leu Gly Gly Pro Gly Gly Val Thr Ala Phe
   385                 390                 395                 400
   Ala Arg Ala Ile Gly Asp Glu Thr Phe Arg Leu Asp Arg Thr Glu Pro
                   405                 410                 415
   Thr Leu Asn Thr Ala Ile Pro Gly Asp Pro Arg Asp Thr Thr Thr Pro
               420                 425                 430
   Arg Ala Met Ala Gln Thr Leu Arg Gln Leu Thr Leu Gly His Ala Leu
           435                 440                 445
   Gly Glu Thr Gln Arg Ala Gln Leu Val Thr Trp Leu Lys Gly Asn Thr
       450                 455                 460
   Thr Gly Ala Ala Ser Ile Arg Ala Gly Leu Pro Thr Ser Trp Thr Ala
   465                 470                 475                 480
   Gly Asp Lys Thr Gly Ser Gly Gly Tyr Gly Thr Thr Asn Asp Ile Ala
                   485                 490                 495
   Val Ile Trp Pro Gln Gly Arg Ala Pro Leu Val Leu Val Thr Tyr Phe
               500                 505                 510
   Thr Gln Pro Gln Gln Asn Ala Glu Ser Arg Arg Asp Val Leu Ala Ser
           515                 520                 525
   Ala Ala Arg Ile Ile Ala Glu Gly Leu
       530                 535
<210> 11
   <211> 4158
   <212> DNA
   <213> Artificial Sequence
<220>
   <223> vecteur et sequence clonnee TEM36
<400> 11
   acattgcctg aattggtttg ccccggaaga ttggggaaac ttggatctga ttaccttagc      60
   tgcaggtacc actgagcgtc agaccccgta gaaaagatca aaggatcttc ttgagatcct     120
   ttttttctgc gcgtaatctg ctgcttgcaa acaaaaaaac caccgctacc agcggtggtt     180
   tgtttgtcgg atcaagagct accaactctt tttccgaagg taactggctt cagcagagcg     240
   cagataccaa atactgttct tctagtgtag ccgtagttag gccaccactt caagaactct     300
   gtagcaccgc ctacatacct cgctctgcta atcctgttac cagtggctgc tgccagtggc     360
   gataagtcgt gtcttaccgg gttggactca agacgatagt taccggataa ggcgcagcgg     420
   tcgggctgaa cggggggttc gtgcacacag cccagcttgg agcgaacgac ctacaccgaa     480
   ctgagatacc tacagcgtga gctatgagaa agcgccacgc ttcccgaagg gagaaaggcg     540
   gacaggtatc cggtaagcgg cagggtcgga acaggagagc gcacgaggga gcttccaggg     600
   ggaaacgcct ggtatcttta tagtcctgtc gggtttcgcc acctctgact tgagcgtcga     660
   tttttgtgat gctcgtcagg ggggcggagc ctatggaaaa acgccagcaa cgcggccttt     720
   ttacggttcc tggccttttg ctggcctttt gctcacatgt tctttcctgc ggtacccaga     780
   tccaattccc gctttgactg cctgaaatct ccatcgccta caatgatgac atttggattt     840
   ggttgactca tgttggtatt gtgaaataga cgcagatcgg gaacactgaa aaatacacag 900
   ttattattca tttaaatttt ttgtagaaat gtcttggtgt cctcgtccaa tcaggtagcc 960
   atctctgaaa tatctggctc cgttgcaact ccgaacgacc tgctggcaac gtaaaattct 1020
   ccggggtaaa acttaaatgt ggagtaatgg aaccagaaac gtctcttccc ttctctctcc 1080
   ttccaccgcc cgttaccgtc cctaggaaat tttactctgc tggagagctt cttctacggc 1140
   ccccttgcag caatgctctt cccagcatta cgttgcgggt aaaacggagg tcgtgtaccc 1200
   gacctagcag cccagggatg gaaaagtccc ggccgtcgct ggcaataata gcgggcggac 1260
   gcatgtcatg agattattgg aaaccaccag aatcgaatat aaaaggcgaa cacctttccc 1320
   aattttggtt tctcctgacc caaagacttt aaatttaatt tatttgtccc tatttcaatc 1380
   aattgaacaa ctatcaaaac acagaattcc gaaacgatga gattcccatc tattttcacc 1440
   gctgtcttgt tcgctgcctc ctctgcattg gctgcccctg ttaacactac cactgaagac 1500
   gagactgctc aaattccagc tgaagcagtt atcggttact ctgaccttga gggtgatttc 1560
   gacgtcgctg ttttgccttt ctctaactcc actaacaacg gtttgttgtt cattaacacc 1620
   actatcgctt ccattgctgc taaggaagag ggtgtctctc tcgagaaaag agaggccgaa 1680
   gctcaccctg agacacttgt caaggttaag gatgccgaag atcaactagg agcaagggtt 1740
   ggttacattg aactggactt gaatagtggt aagattttag agagcttcag acctgaggag 1800
   cgattcccaa tggttagcac ctttaaggta ctcctatgcg gagcagtgtt gtcaagagtc 1860
   gatgctggtc aagagcagtt aggaaggaga atccattatt cacagaacga cttggttgaa 1920
   tattccccag tgactgagaa acacttgaca gatggaatga cagttcgtga gttgtgttct 1980
   gctgctatca ctatgtctga taatacagct gcaaatctgc tgctgacgac aatcggtggt 2040
   cccaaagagt tgaccgcttt tctgcataac atgggcgatc acgtaacaag acttgaccgt 2100
   tgggagcctg aacttaacga agccatcccc aatgacgaaa gagatactac tatgccagct 2160
   gcaatggcta ccactctgcg taagttgctt actggtgaat tgttgacgtt agcttctagg 2220
   caacaattaa tcgactggat ggaagctgat aaagttgctg gcccattgtt gagaagtgct 2280
   ttgccggcag gatggttcat tgcagacaaa tcgggagccg gtgaaagagg ttcaagaggt 2340
   attatagctg cattgggtcc tgacggtaaa ccatccagaa ttgtggtcat ctacactact 2400
   ggttcccagg ctacaatgga cgagcgtgat agacaaattg ccgaaatagg agcatcccta 2460
   atcaaacatt ggtgaggggc ggccgctcaa gaggatgtca gaatgccatt tgcctgagag 2520
   atgcaggctt catttttgat acttttttat ttgtaaccta tatagtatag gatttttttt 2580
   gtcattttgt ttcttctcgt acgagcttgc tcctgatcag cctatctcgc agcagatgaa 2640
   tatcttgtgg taggggtttg ggaaaatcat tcgagtttga tgtttttctt ggtatttccc 2700
   actcctcttc agagtacaga agattaagtg aaaccttcgt ttgtgcggat ccttcagtaa      2760
   tgtcttgttt cttttgttgc agtggtgagc cattttgact tcgtgaaagt ttctttagaa      2820
   tagttgtttc cagaggccaa acattccacc cgtagtaaag tgcaagcgta ggaagaccaa      2880
   gactggcata aatcaggtat aagtgtcgag cactggcagg tgatcttctg aaagtttcta      2940
   ctagcagata agatccagta gtcatgcata tggcaacaat gtaccgtgtg gatctaagaa      3000
   cgcgtcctac taaccttcgc attcgttggt ccagtttgtt gttatcgatc aacgtgacaa      3060
   ggttgtcgat tccgcgtaag catgcatacc caaggacgcc tgttgcaatt ccaagtgagc      3120
   cagttccaac aatctttgta atattagagc acttcattgt gttgcgcttg aaagtaaaat      3180
   gcgaacaaat taagagataa tctcgaaacc gcgacttcaa acgccaatat gatgtgcggc      3240
   acacaataag cgttcatatc cgctgggtga ctttctcgct ttaaaaaatt atccgaaaaa      3300
   attttctaga gtgttgttac tttatacttc cggctcgtat aatacgacaa ggtgtaagga      3360
   ggactaaacc atggctaaac tcacctctgc tgttccagtc ctgactgctc gtgatgttgc      3420
   tggtgctgtt gagttctgga ctgatagact cggtttctcc cgtgacttcg tagaggacga      3480
   ctttgccggt gttgtacgtg acgacgttac cctgttcatc tccgcagttc aggaccaggt      3540
   tgtgccagac aacactctgg catgggtatg ggttcgtggt ctggacgaac tgtacgctga      3600
   gtggtctgag gtcgtgtcta ccaacttccg tgatgcatct ggtccagcta tgaccgagat      3660
   cggtgaacag ccctggggtc gtgagtttgc actgcgtgat ccagctggta actgcgtgca      3720
   tttcgtcgca gaagagcagg actaacaatt gacaccttac gattatttag agagtattta      3780
   ttagttttat tgtatgtata cggatgtttt attatctatt tatgccctta tattctgtaa      3840
   ctatccaaaa gtcctatctt atcaagccag caatctatgt ccgcgaacgt caactaaaaa      3900
   taagcttttt atgctcttct ctcttttttt cccttcggta taattatacc ttgcatccac      3960
   agattctcct gccaaatttt gcataatcct ttacaacatg gctatatggg agcacttagc      4020
   gccctccaaa acccatattg cctacgcatg tataggtgtt ttttccacaa tattttctct      4080
   gtgctctctt tttattaaag agaagctcta tatcggagaa gcttctgtgg ccgttatatt      4140
   cggccttatc gtgggacc                                                    4158
<210> 12
   <211> 900
   <212> DNA
   <213> Escherichia coli
<400> 12
   actatcgctt ccattgctgc taaggaagag ggtgtctctc tcgagaaaag agaggccgaa 60
   gctcagacgt cagccgtgca gcaaaagcta gctgcgctag aaaagtcgag cggcggtcgg 120
   cttggtgttg cattgattga caccgccgac aacacccagg tattgtatcg tggggatgaa 180
   cgcttcccga tgtgctctac atctaaagtt atggcggccg ctgcagtgct gaaacaatct 240
   gaaacgcaga agcaattgct gaatcagccc gttgagatta agccggctga tctggtgaat 300
   tacaatccga ttgccgaaaa acacgtgaac ggtaccatga cgctggctga actgagtgct 360
   gcagcgcttc aatatagcga taacacggcc atgaataagc tgatcgccca gttaggcggt 420
   ccgggaggag tgaccgcctt cgcgcgtgcc ataggtgatg aaactttccg tctggatcgc 480
   acggaaccga cactgaacac agcgatcccg ggtgatccac gtgataccac cacgccgcgc 540
   gcaatggcac agaccctgcg ccaactgacg ctcggccatg cgctggggga gacacaacgt 600
   gcgcagctgg ttacttggct gaaagggaat acaactgggg ctgcgtcgat tcgtgctgga 660
   ctgccgacaa gttggactgc cggcgataaa actgggtcag gtggatatgg cactaccaat 720
   gatatcgcag tgatatggcc gcagggtaga gcgcctcttg ttttggttac ctacttcacg 780
   caaccccagc agaacgcgga gtcacgtcgt gacgtgcttg catcagccgc tcggatcata 840
   gctgaaggtt tgtaaaagcg gccgccgctc aagaggatgt cagaatgcca tttgcctgag 900
<210> 13
   <211> 1680
   <212> DNA
   <213> Artificial Sequence
<220>
   <223> fusion 1 TEM36-G6-CTXM16
<400> 13
   actatcgctt ccattgctgc taaggaagag ggtgtctctc tcgagaaaag agaggccgaa      60
   gctcaccctg agacacttgt caaggttaag gatgccgaag atcaactagg agcaagggtt     120
   ggttacattg aactggactt gaatagtggt aagattttag agagcttcag acctgaggag     180
   cgattcccaa tggttagcac ctttaaggta ctcctatgcg gagcagtgtt gtcaagagtc     240
   gatgctggtc aagagcagtt aggaaggaga atccattatt cacagaacga cttggttgaa     300
   tattccccag tgactgagaa acacttgaca gatggaatga cagttcgtga gttgtgttct     360
   gctgctatca ctatgtctga taatacagct gcaaatctgc tgctgacgac aatcggtggt     420
   cccaaagagt tgaccgcttt tctgcataac atgggcgatc acgtaacaag acttgaccgt     480
   tgggagcctg aacttaacga agccatcccc aatgacgaaa gagatactac tatgccagct     540
   gcaatggcta ccactctgcg taagttgctt actggtgaat tgttgacgtt agcttctagg     600
   caacaattaa tcgactggat ggaagctgat aaagttgctg gcccattgtt gagaagtgct     660
   ttgccggcag gatggttcat tgcagacaaa tcgggagccg gtgaaagagg ttcaagaggt     720
   attatagctg cattgggtcc tgacggtaaa ccatccagaa ttgtggtcat ctacactact     780
   ggttcccagg ctacaatgga cgagcgtgat agacaaattg ccgaaatagg agcatcccta     840
   atcaaacatt ggggaggagg tggaggtgga cagacgtcag ccgtgcagca aaagctagct     900
   gcgctagaaa agtcgagcgg cggtcggctt ggtgttgcat tgattgacac cgccgacaac     960
   acccaggtat tgtatcgtgg ggatgaacgc ttcccgatgt gctctacatc taaagttatg    1020
   gcggccgctg cagtgctgaa acaatctgaa acgcagaagc aattgctgaa tcagcccgtt    1080
   gagattaagc cggctgatct ggtgaattac aatccgattg ccgaaaaaca cgtgaacggt    1140
   accatgacgc tggctgaact gagtgctgca gcgcttcaat atagcgataa cacggccatg    1200
   aataagctga tcgcccagtt aggcggtccg ggaggagtga ccgccttcgc gcgtgccata    1260
   ggtgatgaaa ctttccgtct ggatcgcacg gaaccgacac tgaacacagc gatcccgggt    1320
   gatccacgtg ataccaccac gccgcgcgca atggcacaga ccctgcgcca actgacgctc    1380
   ggccatgcgc tgggggagac acaacgtgcg cagctggtta cttggctgaa agggaataca    1440
   actggggctg cgtcgattcg tgctggactg ccgacaagtt ggactgccgg cgataaaact    1500
   gggtcaggtg gatatggcac taccaatgat atcgcagtga tatggccgca gggtagagcg    1560
   cctcttgttt tggttaccta cttcacgcaa ccccagcaga acgcggagtc acgtcgtgac    1620
   gtgcttgcat cagccgctcg gatcatagct gaaggtttgt aaaagcggcc gctcaagaga    1680
<210> 14
   <211> 1634
   <212> DNA
   <213> Artificial Sequence
<220>
   <223> fusion 2 CTXM16-G6-TEM36
<400> 14
   ctcgagaaaa gagaggccga agctcagacg tcagccgtgc agcaaaagct agctgcgcta      60
   gaaaagtcga gcggcggtcg gcttggtgtt gcattgattg acaccgccga caacacccag     120
   gtattgtatc gtggggatga acgcttcccg atgtgctcta catctaaagt tatggcggcc     180
   gctgcagtgc tgaaacaatc tgaaacgcag aagcaattgc tgaatcagcc cgttgagatt     240
   aagccggctg atctggtgaa ttacaatccg attgccgaaa aacacgtgaa cggtaccatg     300
   acgctggctg aactgagtgc tgcagcgctt caatatagcg ataacacggc catgaataag     360
   ctgatcgccc agttaggcgg tccgggagga gtgaccgcct tcgcgcgtgc cataggtgat     420
   gaaactttcc gtctggatcg cacggaaccg acactgaaca cagcgatccc gggtgatcca     480
   cgtgatacca ccacgccgcg cgcaatggca cagaccctgc gccaactgac gctcggccat     540
   gcgctggggg agacacaacg tgcgcagctg gttacttggc tgaaagggaa tacaactggg     600
   gctgcgtcga ttcgtgctgg actgccgaca agttggactg ccggcgataa aactgggtca     660
   ggtggatatg gcactaccaa tgatatcgca gtgatatggc cgcagggtag agcgcctctt     720
   gttttggtta cctacttcac gcaaccccag cagaacgcgg agtcacgtcg tgacgtgctt     780
   gcatcagccg ctcggatcat agctgaaggt ttgggaggag gaggaggagg acaccctgag     840
   acacttgtca aggttaagga tgccgaagat caactaggag caagggttgg ttacattgaa     900
   ctggacttga atagtggtaa gattttagag agcttcagac ctgaggagcg attcccaatg     960
   gttagcacct ttaaggtact cctatgcgga gcagtgttgt caagagtcga tgctggtcaa    1020
   gagcagttag gaaggagaat ccattattca cagaacgact tggttgaata ttccccagtg    1080
   actgagaaac acttgacaga tggaatgaca gttcgtgagt tgtgttctgc tgctatcact    1140
   atgtctgata atacagctgc aaatctgctg ctgacgacaa tcggtggtcc caaagagttg    1200
   accgcttttc tgcataacat gggcgatcac gtaacaagac ttgaccgttg ggagcctgaa    1260
   cttaacgaag ccatccccaa tgacgaaaga gatactacta tgccagctgc aatggctacc    1320
   actctgcgta agttgcttac tggtgaattg ttgacgttag cttctaggca acaattaatc    1380
   gactggatgg aagctgataa agttgctggc ccattgttga gaagtgcttt gccggcagga    1440
   tggttcattg cagacaaatc gggagccggt gaaagaggtt caagaggtat tatagctgca    1500
   ttgggtcctg acggtaaacc atccagaatt gtggtcatct acactactgg ttcccaggct    1560
   acaatggacg agcgtgatag acaaattgcc gaaataggag catccctaat caaacattgg    1620
   tgaggggcgg ccgc                                                      1634
<210> 15
   <211> 1648
   <212> DNA
   <213> Artificial Sequence
<220>
   <223> fusion 3 TEM36-G(EAAAK)2-CTXM16
<400> 15
   ctcgagaaaa gagaggccga agctcaccct gagacacttg tcaaggttaa ggatgccgaa 60
   gatcaactag gagcaagggt tggttacatt gaactggact tgaatagtgg taagatttta 120
   gagagcttca gacctgagga gcgattccca atggttagca cctttaaggt actcctatgc 180
   ggagcagtgt tgtcaagagt cgatgctggt caagagcagt taggaaggag aatccattat 240
   tcacagaacg acttggttga atattcccca gtgactgaga aacacttgac agatggaatg 300
   acagttcgtg agttgtgttc tgctgctatc actatgtctg ataatacagc tgcaaatctg 360
   ctgctgacga caatcggtgg tcccaaagag ttgaccgctt ttctgcataa catgggcgat 420
   cacgtaacaa gacttgaccg ttgggagcct gaacttaacg aagccatccc caatgacgaa 480
   agagatacta ctatgccagc tgcaatggct accactctgc gtaagttgct tactggtgaa 540
   ttgttgacgt tagcttctag gcaacaatta atcgactgga tggaagctga taaagttgct 600
   ggcccattgt tgagaagtgc tttgccggca ggatggttca ttgcagacaa atcgggagcc 660
   ggtgaaagag gttcaagagg tattatagct gcattgggtc ctgacggtaa accatccaga 720
   attgtggtca tctacactac tggttcccag gctacaatgg acgagcgtga tagacaaatt      780
   gccgaaatag gagcatccct aatcaaacat tggggagagg cggccgctaa agaggcagca      840
   gcaaaacaga cgtcagccgt gcagcaaaag ctagctgcgc tagaaaagtc gagcggcggt      900
   cggcttggtg ttgcattgat tgacaccgcc gacaacaccc aggtattgta tcgtggggat      960
   gaacgcttcc cgatgtgctc tacatctaaa gttatggcgg ccgctgcagt gctgaaacaa     1020
   tctgaaacgc agaagcaatt gctgaatcag cccgttgaga ttaagccggc tgatctggtg     1080
   aattacaatc cgattgccga aaaacacgtg aacggtacca tgacgctggc tgaactgagt     1140
   gctgcagcgc ttcaatatag cgataacacg gccatgaata agctgatcgc ccagttaggc     1200
   ggtccgggag gagtgaccgc cttcgcgcgt gccataggtg atgaaacttt ccgtctggat     1260
   cgcacggaac cgacactgaa cacagcgatc ccgggtgatc cacgtgatac caccacgccg     1320
   cgcgcaatgg cacagaccct gcgccaactg acgctcggcc atgcgctggg ggagacacaa     1380
   cgtgcgcagc tggttacttg gctgaaaggg aatacaactg gggctgcgtc gattcgtgct     1440
   ggactgccga caagttggac tgccggcgat aaaactgggt caggtggata tggcactacc     1500
   aatgatatcg cagtgatatg gccgcagggt agagcgcctc ttgttttggt tacctacttc     1560
   acgcaacccc agcagaacgc ggagtcacgt cgtgacgtgc ttgcatcagc cgctcggatc     1620
   atagctgaag gtttgtaaaa gcggccgc                                        1648
<210> 16
   <211> 870
   <212> DNA
   <213> Escherichia coli
<400> 16
   catatgagca ttcagcattt tcgcgtggcc ctgatcccgt ttttcgcggc cttttgcctg 60
   ccggttttcg cacagacgtc agccgtgcag caaaagctag ctgcgctaga aaagtcgagc 120
   ggcggtcggc ttggtgttgc attgattgac accgccgaca acacccaggt attgtatcgt 180
   ggggatgaac gcttcccgat gtgctctaca tctaaagtta tggcggccgc tgcagtgctg 240
   aaacaatctg aaacgcagaa gcaattgctg aatcagcccg ttgagattaa gccggctgat 300
   ctggtgaatt acaatccgat tgccgaaaaa cacgtgaacg gtaccatgac gctggctgaa 360
   ctgagtgctg cagcgcttca atatagcgat aacacggcca tgaataagct gatcgcccag 420
   ttaggcggtc cgggaggagt gaccgccttc gcgcgtgcca taggtgatga aactttccgt 480
   ctggatcgca cggaaccgac actgaacaca gcgatcccgg gtgatccacg tgataccacc 540
   acgccgcgcg caatggcaca gaccctgcgc caactgacgc tcggccatgc gctgggggag 600
   acacaacgtg cgcagctggt tacttggctg aaagggaata caactggggc tgcgtcgatt 660
   cgtgctggac tgccgacaag ttggactgcc ggcgataaaa ctgggtcagg tggatatggc 720
   actaccaatg atatcgcagt gatatggccg cagggtagag cgcctcttgt tttggttacc 780
   tacttcacgc aaccccagca gaacgcggag tcacgtcgtg acgtgcttgc atcagccgct 840
   cggatcatag ctgaaggttt gtaaaagctt 870
<210> 17
   <211> 627
   <212> DNA
   <213> Escherichia coli
<400> 17
   catatgagca acgctaagac gaaactgggt attaccaagt acagcatcgt gacgaatagc 60
   aacgattccg tgacgctgcg tctgatgacc gaacacgact tggcaatgct gtacgagtgg 120
   ctgaatcgca gccatatcgt tgagtggtgg ggtggtgaag aagcccgtcc gaccctggcc 180
   gacgttcaag agcagtatct gccttctgtg ttggcgcaag aaagcgtgac cccgtatatt 240
   gcaatgctga atggcgagcc gatcggttac gcgcaatcgt acgtggcact gggcagcggt 300
   gatggtcgtt gggaagaaga gactgatccg ggcgttcgtg gtatcgacca gctgctggcc 360
   aacgcgtccc agctcggtaa aggcttaggt accaaactgg tccgtgcatt ggttgagctg 420
   ctgttcaacg atccggaagt caccaagatt caaaccgacc cgagcccgag caatctgcgt 480
   gcgattcgct gctatgagaa agcgggcttc gagcgccagg gcactgtcac cacgccgtat 540
   ggtccagctg tttacatggt ccagacgcgc caggcgtttg agcgtacccg tagcgacgcg 600
   caccaccatc atcaccacta aaagctt 627
<210> 18
   <211> 1416
   <212> DNA
   <213> Artificial Sequence
<220>
   <223> fusion 5 AAC-H6-CTXM16
<400> 18
   catatgagca acgctaagac gaaactgggt attaccaagt acagcatcgt gacgaatagc      60
   aacgattccg tgacgctgcg tctgatgacc gaacacgact tggcaatgct gtacgagtgg     120
   ctgaatcgca gccatatcgt tgagtggtgg ggtggtgaag aagcccgtcc gaccctggcc     180
   gacgttcaag agcagtatct gccttctgtg ttggcgcaag aaagcgtgac cccgtatatt     240
   gcaatgctga atggcgagcc gatcggttac gcgcaatcgt acgtggcact gggcagcggt     300
   gatggtcgtt gggaagaaga gactgatccg ggcgttcgtg gtatcgacca gctgctggcc     360
   aacgcgtccc agctcggtaa aggcttaggt accaaactgg tccgtgcatt ggttgagctg     420
   ctgttcaacg atccggaagt caccaagatt caaaccgacc cgagcccgag caatctgcgt     480
   gcgattcgct gctatgagaa agcgggcttc gagcgccagg gcactgtcac cacgccgtat     540
   ggtccagctg tttacatggt ccagacgcgc caggcgtttg agcgtacccg tagcgacgcg     600
   caccaccatc atcaccacca gacgtcagcc gtgcagcaaa agctagctgc gctagaaaag     660
   tcgagcggcg gtcggcttgg tgttgcattg attgacaccg ccgacaacac ccaggtattg     720
   tatcgtgggg atgaacgctt cccgatgtgc tctacatcta aagttatggc ggccgctgca     780
   gtgctgaaac aatctgaaac gcagaagcaa ttgctgaatc agcccgttga gattaagccg     840
   gctgatctgg tgaattacaa tccgattgcc gaaaaacacg tgaacggtac catgacgctg     900
   gctgaactga gtgctgcagc gcttcaatat agcgataaca cggccatgaa taagctgatc     960
   gcccagttag gcggtccggg aggagtgacc gccttcgcgc gtgccatagg tgatgaaact    1020
   ttccgtctgg atcgcacgga accgacactg aacacagcga tcccgggtga tccacgtgat    1080
   accaccacgc cgcgcgcaat ggcacagacc ctgcgccaac tgacgctcgg ccatgcgctg    1140
   ggggagacac aacgtgcgca gctggttact tggctgaaag ggaatacaac tggggctgcg    1200
   tcgattcgtg ctggactgcc gacaagttgg actgccggcg ataaaactgg gtcaggtgga    1260
   tatggcacta ccaatgatat cgcagtgata tggccgcagg gtagagcgcc tcttgttttg    1320
   gttacctact tcacgcaacc ccagcagaac gcggagtcac gtcgtgacgt gcttgcatca    1380
   gccgctcgga tcatagctga aggtttgtaa aagctt                              1416
<210> 19
   <211> 1287
   <212> DNA
   <213> Escherichia coli
<400> 19
   catatgcgtt ttgaagagtg ggtcaaggac aagcacattc cgtttaagtt aaaccacccg      60
   gatgacaatt acgacgactt caaaccgctg cgtaagatta ttggtgacac gcgcgtcgtg     120
   gcgctgggtg agaatagcca ctttatcaaa gaattcttct tgctgcgcca cacgctgctg     180
   cgtttcttta ttgaggacct gggcttcacc acgttcgcct ttgagttcgg cttcgcagag     240
   ggtcagatca tcaacaattg gatccatggc caaggtaccg atgatgagat tggtcgtttc     300
   ctgaaacact tctattaccc ggaagaactg aaaaccacct ttttgtggct gcgtgagtac     360
   aataaagccg cgaaggaaaa gatcacgttt ctgggtattg acatcccgcg taacggtggc     420
   agctatctgc cgaacatgga aatcgttcat gactttttcc gtaccgctga caaagaagcg     480
   ctgcacatta ttgacgacgc gtttaacatc gcgaaaaaga tcgattactt tagcaccagc     540
   caagcagcgc tgaatttgca tgagctgacg gacagcgaga aatgccgcct cacttcccag     600
   ctggcacgtg tcaaggttcg cctggaagca atggcgccga tccacatcga gaagtatggc     660
   attgacaagt acgaaacgat cctgcactat gcaaacggca tgatttatct ggattacaat     720
   attcaggcca tgagcggttt catttccggt ggcggcatgc agggtgacat gggtgcgaaa     780
   gataagtaca tggccgattc tgtcctgtgg cacctgaaga accctcagag cgagcagaaa     840
   gttattgtgg tggctcataa tgcacatatt cagaaaaccc cgattttgta cgacggtttc     900
   ctgagctgcc tgccgatggg tcaacgtctg aagaacgcga tcggtgatga ctacatgtct     960
   ttgggcatca cctcgtatag cggtcatact gcggcactgt atccggaagt tgataccaaa    1020
   tacggtttcc gcgtggataa ctttcaactg caagagccga acgagggtag cgtggagaaa    1080
   gctattagcg gttgtggcgt taccaattcc ttcgtgttct ttcgtaatat cccggaagat    1140
   ctgcagagca ttccaaacat gatccgcttc gatagcattt acatgaaagc ggagcttgag    1200
   aaagcgtttg atggcatctt ccaaatcgaa aagagcagcg tcagcgaagt ggtttatgag    1260
   caccaccatc accatcacta aaagctt                                        1287
<210> 20
   <211> 2076
   <212> DNA
   <213> Artificial Sequence
<220>
   <223> fusion 6 EreB-H6-TEM36
<400> 20
   catatgcgtt ttgaagagtg ggtcaaggac aagcacattc cgtttaagtt aaaccacccg      60
   gatgacaatt acgacgactt caaaccgctg cgtaagatta ttggtgacac gcgcgtcgtg     120
   gcgctgggtg agaatagcca ctttatcaaa gaattcttct tgctgcgcca cacgctgctg     180
   cgtttcttta ttgaggacct gggcttcacc acgttcgcct ttgagttcgg cttcgcagag     240
   ggtcagatca tcaacaattg gatccatggc caaggtaccg atgatgagat tggtcgtttc     300
   ctgaaacact tctattaccc ggaagaactg aaaaccacct ttttgtggct gcgtgagtac      360
   aataaagccg cgaaggaaaa gatcacgttt ctgggtattg acatcccgcg taacggtggc     420
   agctatctgc cgaacatgga aatcgttcat gactttttcc gtaccgctga caaagaagcg     480
   ctgcacatta ttgacgacgc gtttaacatc gcgaaaaaga tcgattactt tagcaccagc     540
   caagcagcgc tgaatttgca tgagctgacg gacagcgaga aatgccgcct cacttcccag     600
   ctggcacgtg tcaaggttcg cctggaagca atggcgccga tccacatcga gaagtatggc     660
   attgacaagt acgaaacgat cctgcactat gcaaacggca tgatttatct ggattacaat     720
   attcaggcca tgagcggttt catttccggt ggcggcatgc agggtgacat gggtgcgaaa     780
   gataagtaca tggccgattc tgtcctgtgg cacctgaaga accctcagag cgagcagaaa     840
   gttattgtgg tggctcataa tgcacatatt cagaaaaccc cgattttgta cgacggtttc     900
   ctgagctgcc tgccgatggg tcaacgtctg aagaacgcga tcggtgatga ctacatgtct     960
   ttgggcatca cctcgtatag cggtcatact gcggcactgt atccggaagt tgataccaaa    1020
   tacggtttcc gcgtggataa ctttcaactg caagaaccaa acgagggtag cgtggagaaa 1080
   gctattagcg gttgtggcgt taccaattcc ttcgtgttct ttcgtaatat cccggaagat 1140
   ctgcagagca ttccaaacat gatccgcttc gatagcattt acatgaaagc ggagcttgag 1200
   aaagcgtttg atggcatctt ccaaatcgaa aagagcagcg tcagcgaagt ggtttatgag 1260
   caccaccatc accatcacca cccggaaacc ctggtgaaag ttaaagatgc ggaagatcag 1320
   ctgggcgccc gtgtgggtta tattgaactg gatctgaaca gcggcaaaat cctggaatct 1380
   tttcgtccgg aagaacgctt cccgatggtg tctaccttta aagttctgct gtgcggtgca 1440
   gtgctgagtc gcgttgatgc gggccaggaa cagctgggtc gtcgcattca ttatagccag 1500
   aatgatctgg tggaatactc tccggttacc gaaaaacacc tgaccgatgg catgacggtg 1560
   cgtgaactgt gtagtgcagc gattaccatg agcgataaca cggccgcaaa tctgctgctg 1620
   accacgatcg gcggtccgaa agaactgacc gccttcctgc ataacatggg tgatcacgtt 1680
   acgcgtctgg atcgctggga accggaactg aacgaagcga tcccgaatga tgaacgtgat 1740
   accaccatgc cggccgcaat ggcaaccacc ctgcgcaaac tgctgaccgg cgaactgctg 1800
   acgctggcaa gccgtcagca gctgattgat tggatggaag cggataaagt tgcgggtccg 1860
   ctgctgcgtt ctgcactgcc ggcaggttgg tttatcgccg ataaatctgg cgcaggtgaa 1920
   cgtggcagtc gcggtattat cgcagcgctg ggcccggatg gtaaaccgag tcgtattgtg 1980
   gttatctaca ccacgggcag ccaggcgacc atggatgaac gtgatcgcca gattgcagaa 2040
   atcggtgcga gcctgattaa acattggtaa aagctt 2076
<210> 21
   <211> 468
   <212> PRT
   <213> Artificial Sequence
<220>
   <223> fusion 5 AAC-H6-CTXM16
<400> 21
   Met Ser Asn Ala Lys Thr Lys Leu Gly Ile Thr Lys Tyr Ser Ile Val
   1 5 10 15
   Thr Asn Ser Asn Asp Ser Val Thr Leu Arg Leu Met Thr Glu His Asp
   20 25 30
   Leu Ala Met Leu Tyr Glu Trp Leu Asn Arg Ser His Ile Val Glu Trp
   35 40 45
   Trp Gly Gly Glu Glu Ala Arg Pro Thr Leu Ala Asp Val Gln Glu Gln
   50 55 60
   Tyr Leu Pro Ser Val Leu Ala Gln Glu Ser Val Thr Pro Tyr Ile Ala
   65                  70                  75                  80
   Met Leu Asn Gly Glu Pro Ile Gly Tyr Ala Gln Ser Tyr Val Ala Leu
                   85                  90                  95
   Gly Ser Gly Asp Gly Arg Trp Glu Glu Glu Thr Asp Pro Gly Val Arg
               100                 105                 110
   Gly Ile Asp Gln Leu Leu Ala Asn Ala Ser Gln Leu Gly Lys Gly Leu
           115                 120                 125
   Gly Thr Lys Leu Val Arg Ala Leu Val Glu Leu Leu Phe Asn Asp Pro
       130                 135                 140
   Glu Val Thr Lys Ile Gln Thr Asp Pro Ser Pro Ser Asn Leu Arg Ala
   145                 150                 155                 160
   Ile Arg Cys Tyr Glu Lys Ala Gly Phe Glu Arg Gln Gly Thr Val Thr
                   165                 170                 175
   Thr Pro Tyr Gly Pro Ala Val Tyr Met Val Gln Thr Arg Gln Ala Phe
               180                 185                 190
   Glu Arg Thr Arg Ser Asp Ala His His His His His His Gln Thr Ser
           195                 200                 205
   Ala Val Gln Gln Lys Leu Ala Ala Leu Glu Lys Ser Ser Gly Gly Arg
       210                 215                 220
   Leu Gly Val Ala Leu Ile Asp Thr Ala Asp Asn Thr Gln Val Leu Tyr
   225                 230                 235                 240
   Arg Gly Asp Glu Arg Phe Pro Met Cys Ser Thr Ser Lys Val Met Ala
                   245                 250                 255
   Ala Ala Ala Val Leu Lys Gln Ser Glu Thr Gln Lys Gln Leu Leu Asn
               260                 265                 270
   Gln Pro Val Glu Ile Lys Pro Ala Asp Leu Val Asn Tyr Asn Pro Ile
           275                 280                 285
   Ala Glu Lys His Val Asn Gly Thr Met Thr Leu Ala Glu Leu Ser Ala
       290                 295                 300
   Ala Ala Leu Gln Tyr Ser Asp Asn Thr Ala Met Asn Lys Leu Ile Ala
   305                 310                 315                 320
   Gln Leu Gly Gly Pro Gly Gly Val Thr Ala Phe Ala Arg Ala Ile Gly
                   325                 330                 335
   Asp Glu Thr Phe Arg Leu Asp Arg Thr Glu Pro Thr Leu Asn Thr Ala
               340                 345                 350
   Ile Pro Gly Asp Pro Arg Asp Thr Thr Thr Pro Arg Ala Met Ala Gln
           355                 360                 365
   Thr Leu Arg Gln Leu Thr Leu Gly His Ala Leu Gly Glu Thr Gln Arg
       370                 375                 380
   Ala Gln Leu Val Thr Trp Leu Lys Gly Asn Thr Thr Gly Ala Ala Ser
   385                 390                 395                 400
   Ile Arg Ala Gly Leu Pro Thr Ser Trp Thr Ala Gly Asp Lys Thr Gly
                   405                 410                 415
   Ser Gly Gly Tyr Gly Thr Thr Asn Asp Ile Ala Val Ile Trp Pro Gln
               420                 425                 430
   Gly Arg Ala Pro Leu Val Leu Val Thr Tyr Phe Thr Gln Pro Gln Gln
           435                 440                 445
   Asn Ala Glu Ser Arg Arg Asp Val Leu Ala Ser Ala Ala Arg Ile Ile
       450                 455                 460
   Ala Glu Gly Leu
   465
<210> 22
   <211> 688
   <212> PRT
   <213> Artificial Sequence
<220>
   <223> fusion 6 EreB-H6-TEM36
<400> 22
   Met Arg Phe Glu Glu Trp Val Lys Asp Lys His Ile Pro Phe Lys Leu
   1               5                   10                  15
   Asn His Pro Asp Asp Asn Tyr Asp Asp Phe Lys Pro Leu Arg Lys Ile
               20                  25                  30
   Ile Gly Asp Thr Arg Val Val Ala Leu Gly Glu Asn Ser His Phe Ile
           35                  40                  45
   Lys Glu Phe Phe Leu Leu Arg His Thr Leu Leu Arg Phe Phe Ile Glu
       50                  55                  60
   Asp Leu Gly Phe Thr Thr Phe Ala Phe Glu Phe Gly Phe Ala Glu Gly
   65                  70                  75                  80
   Gln Ile Ile Asn Asn Trp Ile His Gly Gln Gly Thr Asp Asp Glu Ile
                   85                  90                  95
   Gly Arg Phe Leu Lys His Phe Tyr Tyr Pro Glu Glu Leu Lys Thr Thr
               100                 105                 110
   Phe Leu Trp Leu Arg Glu Tyr Asn Lys Ala Ala Lys Glu Lys Ile Thr
           115                 120                 125
   Phe Leu Gly Ile Asp Ile Pro Arg Asn Gly Gly Ser Tyr Leu Pro Asn
       130                 135                 140
   Met Glu Ile Val His Asp Phe Phe Arg Thr Ala Asp Lys Glu Ala Leu
   145                 150                 155                 160
   His Ile Ile Asp Asp Ala Phe Asn Ile Ala Lys Lys Ile Asp Tyr Phe
                   165                 170                 175
   Ser Thr Ser Gln Ala Ala Leu Asn Leu His Glu Leu Thr Asp Ser Glu
               180                 185                 190
   Lys Cys Arg Leu Thr Ser Gln Leu Ala Arg Val Lys Val Arg Leu Glu
           195                 200                 205
   Ala Met Ala Pro Ile His Ile Glu Lys Tyr Gly Ile Asp Lys Tyr Glu
       210                 215                 220
   Thr Ile Leu His Tyr Ala Asn Gly Met Ile Tyr Leu Asp Tyr Asn Ile
   225                 230                 235                 240
   Gln Ala Met Ser Gly Phe Ile Ser Gly Gly Gly Met Gln Gly Asp Met
                   245                 250                 255
   Gly Ala Lys Asp Lys Tyr Met Ala Asp Ser Val Leu Trp His Leu Lys
               260                 265                 270
   Asn Pro Gln Ser Glu Gln Lys Val Ile Val Val Ala His Asn Ala His
           275                 280                 285
   Ile Gln Lys Thr Pro Ile Leu Tyr Asp Gly Phe Leu Ser Cys Leu Pro
       290                 295                 300
   Met Gly Gln Arg Leu Lys Asn Ala Ile Gly Asp Asp Tyr Met Ser Leu
   305 310 315 320
   Gly Ile Thr Ser Tyr Ser Gly His Thr Ala Ala Leu Tyr Pro Glu Val
   325 330 335
   Asp Thr Lys Tyr Gly Phe Arg Val Asp Asn Phe Gln Leu Gln Glu Pro
   340 345 350
   Asn Glu Gly Ser Val Glu Lys Ala Ile Ser Gly Cys Gly Val Thr Asn
   355 360 365
   Ser Phe Val Phe Phe Arg Asn Ile Pro Glu Asp Leu Gln Ser Ile Pro
   370 375 380
   Asn Met Ile Arg Phe Asp Ser Ile Tyr Met Lys Ala Glu Leu Glu Lys
   385 390 395 400
   Ala Phe Asp Gly Ile Phe Gln Ile Glu Lys Ser Ser Val Ser Glu Val
   405 410 415
   Val Tyr Glu His His His His His His His Pro Glu Thr Leu Val Lys
   420 425 430
   Val Lys Asp Ala Glu Asp Gln Leu Gly Ala Arg Val Gly Tyr Ile Glu
   435 440 445
   Leu Asp Leu Asn Ser Gly Lys Ile Leu Glu Ser Phe Arg Pro Glu Glu
   450 455 460
   Arg Phe Pro Met Val Ser Thr Phe Lys Val Leu Leu Cys Gly Ala Val
   465 470 475 480
   Leu Ser Arg Val Asp Ala Gly Gln Glu Gln Leu Gly Arg Arg Ile His
   485 490 495
   Tyr Ser Gln Asn Asp Leu Val Glu Tyr Ser Pro Val Thr Glu Lys His
   500 505 510
   Leu Thr Asp Gly Met Thr Val Arg Glu Leu Cys Ser Ala Ala Ile Thr
   515 520 525
   Met Ser Asp Asn Thr Ala Ala Asn Leu Leu Leu Thr Thr Ile Gly Gly
   530 535 540
   Pro Lys Glu Leu Thr Ala Phe Leu His Asn Met Gly Asp His Val Thr
   545 550 555 560
   Arg Leu Asp Arg Trp Glu Pro Glu Leu Asn Glu Ala Ile Pro Asn Asp
   565 570 575
   Glu Arg Asp Thr Thr Met Pro Ala Ala Met Ala Thr Thr Leu Arg Lys
   580 585 590
   Leu Leu Thr Gly Glu Leu Leu Thr Leu Ala Ser Arg Gln Gln Leu Ile
   595 600 605
   Asp Trp Met Glu Ala Asp Lys Val Ala Gly Pro Leu Leu Arg Ser Ala
   610 615 620
   Leu Pro Ala Gly Trp Phe Ile Ala Asp Lys Ser Gly Ala Gly Glu Arg
   625 630 635 640
   Gly Ser Arg Gly Ile Ile Ala Ala Leu Gly Pro Asp Gly Lys Pro Ser
   645 650 655
   Arg Ile Val Val Ile Tyr Thr Thr Gly Ser Gln Ala Thr Met Asp Glu
   660 665 670
   Arg Asp Arg Gln Ile Ala Glu Ile Gly Ala Ser Leu Ile Lys His Trp
   675 680 685
<210> 23
   <211> 18
   <212> DNA
   <213> Pichia pastoris
<400> 23
   gagggtgtct ctctcgag 18
<210> 24
   <211> 36
   <212> DNA
   <213> Pichia pastoris
<400> 24
   tccacctcca cctcctcccc aatgtttgat taggga 36
<210> 25
   <211> 39
   <212> DNA
   <213> Pichia pastoris
<400> 25
   ggaggtggag gtggacagac gtcagccgtg cagcaaaag 39
<210> 26
   <211> 30
   <212> DNA
   <213> Pichia pastoris
<400> 26
   gctaggcggc cgcttttaca aaccttcagc 30
<210> 27
   <211> 18
   <212> DNA
   <213> Pichia pastoris
<400> 27
   gagggtgtct ctctcgag 18
<210> 28
   <211> 39
   <212> DNA
   <213> Pichia pastoris
<400> 28
   tcctcctcct cctcctccca aaccttcagc tatgatccg 39
<210> 29
   <211> 39
   <212> DNA
   <213> Pichia pastoris
<400> 29
   ggaggaggag gaggaggaca ccctgagaca cttgtcaag 39
<210> 30
   <211> 18
   <212> DNA
   <213> Pichia pastoris
<400> 30
   ttgagcggcc gcccctca 18
<210> 31
   <211> 45
   <212> DNA
   <213> Pichia pastoris
<400> 31
   tgctgcctct ttagcggccg cctctcccca atgtttgatt aggga 45
<210> 32
   <211> 39
   <212> DNA
   <213> Pichia pastoris
<400> 32
   gccgctaaag aggcagcagc aaaacagacg tcagccgtg 39
<210> 33
   <211> 24
   <212> DNA
   <213> Escherichia coli
<400> 33
   taatacgact cactataggg gaat 24
<210> 34
   <211> 36
   <212> DNA
   <213> Escherichia coli
<400> 34
   tcctcctcct cctcctcccc aatgtttaat caggct 36
<210> 35
   <211> 39
   <212> DNA
   <213> Escherichia coli
<400> 35
   ggaggaggag gaggacagac gtcagccgtg cagcaaaag 39
<210> 36
   <211> 39
   <212> DNA
   <213> Escherichia coli
<400> 36
   tcctcctcct cctcctccca aaccttcagc tatgatccg 39
<210> 37
   <211> 39
   <212> DNA
   <213> Escherichia coli
<400> 37
   ggaggaggag gaggaggaca ccctgagaca cttgtcaag 39
<210> 38
   <211> 21
   <212> DNA
   <213> Escherichia coli
<400> 38
   ctagttattg ctcagcggtg g 21
<210> 39
   <211> 27
   <212> DNA
   <213> Escherichia coli
<400> 39
   gaaggagata tacatatgag caacgct 27
<210> 40
   <211> 21
   <212> DNA
   <213> Escherichia coli
<400> 40
   gtggtgatga tggtggtgcg c 21
<210> 41
   <211> 39
   <212> DNA
   <213> Escherichia coli
<400> 41
   caccatcatc accaccagac gtcagccgtg cagcaaaag 39
<210> 42
   <211> 27
   <212> DNA
   <213> Escherichia coli
<400> 42
   gatatacata tgcgttttga agagtgg 27
<210> 43
   <211> 27
   <212> DNA
   <213> Escherichia coli
<400> 43
   gtgatggtga tggtggtgct cataaac 27
<210> 44
   <211> 42
   <212> DNA
   <213> Escherichia coli
<400> 44
   caccaccatc accatcacca cccggaaacc ctggtgaaag tt 42
37